# EUROPEAN PATENT SPECIFICATION

(11) **EP 2 661 275 B1**
(45) Date of publication and mention of the grant of the patent: **02.01.2019**
(21) Application number: 12732165.1
(22) Date of filing: 09.01.2012
(51) Int. Cl.: A61K 9/127, A61K 9/19, A61K 38/17, A61K 31/7088, A61N 5/10, A61K 38/42, A61K 31/715, B65D 25/00, A61K 41/00, A61K 38/16, A61K 31/713

(54) **COMPOSITIONS AND METHODS FOR DELIVERY OF HIGH-AFFINITY OXYGEN BINDING AGENTS TO TUMORS**
ZUSAMMENSETZUNGEN UND VERFAHREN ZUR ABGABE HOCHAFFINER SAUERSTOFFBINDEMITTEL IN TUMORE
COMPOSITIONS ET PROCÉDÉS POUR DÉLIVRER AUX TUMEURS DES AGENTS SE LIANT À L'OXYGÈNE AVEC UNE AFFINITÉ ÉLEVÉE

(30) Priority: 07.01.2011 US 201161430628 P; 25.01.2011 US 201161435886 P
(43) Date of publication of application: 13.11.2013
(73) Proprietor: Poseida Therapeutics, Inc., San Diego, CA 92121 (US)
(72) Inventor: GHOROGHCHIAN, P. Peter, Lexington, KY 40511 (US)
(74) Representative: Cooley (UK) LLP
(86) International application number: PCT/US2012/020680
(87) International publication number: WO 2012/094679

(56) References cited:
- WO-A1-03/059363
- WO-A2-2011/133635
- US-A- 5 833 974
- US-A1- 2005 129 747
- CONOVER, C. D. ET AL.: 'The ability of polyethylene glycol conjugated bovine hemoglobin (PEG-Hb) to adequately deliver oxygen in both exchange transfusion and top-loaded rat models' ARTIF. CELLS BLOOD SUBSTIT. IMMOBIL. BIOTENHNOL. vol. 27, no. 2, March 1999, pages 93 - 107, XP008169461
- ZHAO J. ET AL.: 'Preparation of hemoglobin-loaded nano-sized particles with porous structure as oxygen carriers' BIOMATERIALS vol. 28, no. 7, March 2007, pages 1414 - 1422, XP005812898

## Description

### FIELD OF INVENTION

The present application is related to compositions and methods for synthesis of high-affinity oxygen binding agents suitable for being delivered to tumors to increase intratumoral partial pressures of oxygen, mitigate the natural selection of tumor cells that demonstrate aggressive molecular behavior and metastatic potential, and potentiate the effects of radiation and chemotherapies.

### BACKGROUND OF THE INVENTION

Each year, approximately 1.2 million Americans are diagnosed with solid tumor malignancies, resulting in aggregate health care costs of greater than $55 billion for treatment. ^{1, 2} More than 50% of these patients undergo radiotherapy (XRT) as part of their treatment plan.³⁻⁵ Local tumor recurrence in the radiated field is often implicated as a primary cause of treatment failure in patients undergoing definitive therapy.⁴⁻⁷ The ability of XRT to eradicate malignant cells depends critically upon the intratumoral content of O₂, a potent radiosensitizer involved in mediating DNA damage.⁸⁻¹⁰ The intratumoral O₂ level is one of the most important determinants of response among tumors of the same type treated with a single fraction of ionizing radiation therapy.^{4, 5, 11} Experimental studies suggest that hypoxic cells are 2-3 times more resistant to a single fraction of ionizing radiation than those with normal levels of O₂.^{5, 10, 12, 13} While XRT generates high levels of localized reactive oxygen species (ROS) that are cytotoxic, tumor hypoxia promotes baseline endogenous ROS¹⁴ that result in the stabilization of hypoxia-inducible factor 1 (HIF-1) and lead to a more aggressive tumorigenic phenotype.^{3, 5, 8, 15-17} Investigations on the prognostic significance of the pretreatment O₂ levels of tumors in patients with head, neck, and cervical cancers have further demonstrated that worsening hypoxia, typically designated in these studies as oxygen tension (pO₂) levels below 2.5-10 mmHg, is associated with both radiation and chemotherapy resistance, decreased local tumor control after surgery, as well as lower rates of survival.^{4, 5,} 18-28

Although hypoxia has been recognized as a cause of treatment failure in solid tumors for more than 50 years, efforts to overcome it have generally been unsuccessful.^{4, 5, 8, 29-35} A number of strategies have been designed to enhance the radiosensitivity and radiocurability of solid tumors. The most well-studied, hypoxia-altering methods have involved the use of electron-affinity radiosensitizers that mimic the actions of O₂ but are more slowly metabolized. During the past three decades, the nitroimidazole compounds have been extensively evaluated as adjuncts to XRT in carcinomas of the head, neck, cervix, and lung.³⁶⁻⁴³ Most of these studies have reported disappointing local control and survival outcomes,^{36-38, 40, 41, 43} but efforts to maximize their efficacy and safety, as well as to develop newer classes of agents, are ongoing.⁴⁴⁻⁴⁷ The majority of alternative strategies have relied on the use of bulk alkylating compounds that confer direct cytotoxic effects that are independent of XRT administration. Clinical trials evaluating mitomycin C, tirapazamine, porfiromycin and others have shown statistically and clinically significant improvements in loco-regional control and cause-specific survival of various cancers, but often at the cost of significant toxicities with repeated dosing.^{30-32, 48-73}

The most direct and least toxic path to overcoming tumor hypoxia is to increase the intratumoral pO₂. The administration of hyperbaric oxygen was initially attempted but is not used clinically as it exhibits inconsistent response, prohibitive cost, inconvenience, and administration-related safety issues.^{4, 5, 8, 10, 74} More recent strategies have included administration of carbogen,^{45, 75-82} transfusions of blood, synthetic hemoglobin-based oxygen carriers, or perfluorocarbon emulsions,^{5, 83, 84} and injections of recombinant human erythropoietin,^{5, 85-89} allosteric effectors (RSR13), or angiogenesis inhibitors.⁹⁰⁻⁹² All of these strategies have met with minimal clinical success due to their reliance on hyperbaric oxygen loading, formulation instabilities, release of hemoglobin-bound oxygen that occurs at pO₂ values (20-40 mmHg) that are much higher than those found in hypoxic tumor regions (< 3 mmHg), and/or intravascular regulatory mechanisms that alter blood flow to maintain relatively constant tissue oxygenation levels. ^{5, 8, 83, 91}

Relevant prior art documents are WO 2011/133635 A2, WO 03/059363 A1 and US 5833974 A. A list of publications referenced in this disclosure follows.
1. American Cancer Society, Cancer Facts and Figures 2010, http://www.cancer.org/research/cancerfactsfigures/cancerfactsfigures/cancer-facts-and-figures-2010 (last visited Oct. 10, 2011).
2. Care Core National, Radiation Therapy Management 2010, http://www.carecorenational.com/radiation-therapy-management.asp (last visited 2010).
3. Feldmann H.J., Molls M., Vaupel P., Blood Flow And Oxygenation Status Of Human Tumors - Clinical Investigations, STRAHLENTHERAPIE UND ONKOLOGIE 1999;175:1-9.
4. Harrison L., Blackwell K., Hypoxia and Anemia: Factors In Decreased Sensitivity To Radiation Therapy And Chemotherapy, ONCOLOGIST 2004; 9:31-40.
5. Harrison L.B., Chadha M., Hill R.J., Hu K., Shasha D., Impact Of Tumor Hypoxia And Anemia On Radiation Therapy Outcomes, ONCOLOGIST 2002; 7:492-508.
6. Mundt A.J., Connell P.P., Campbell T., Hwang J.H., Rotmensch J., Waggoner S., Race And Clinical Outcome In Patients With Carcinoma Of The Uterine Cervix Treated With Radiation Therapy, GYNECOLOGIC ONCOLOGY 1998; 71:151-8.
7. Takeshi K., Katsuyuki K., Yoshiaki T., et al., Definitive Radiotherapy Combined With High-Dose-Rate Brachytherapy For Stage III Carcinoma Of The Uterine Cervix: Retrospective Analysis Of Prognostic Factors Concerning Patient Characteristics And Treatment Parameters, INTERNATIONAL JOURNAL OF RADIATION ONCOLOGY BIOLOGY PHYSICS 1998; 41:319-27.
8. Rockwell S., Dobrucki I.T., Kim E.Y., Marrison S.T., Vu V.T., Hypoxia and Radiation Therapy: Past History, Ongoing Research, and Future Promise, CURRENT MOLECULAR MEDICINE 2009; 9:442-58.
9. Carlson D.J., Keall P.J., Chen Z.J., Stewart R.D., Nath R., Brown J.M., Towards Temporal Optimization of Radiation Fractionation: The Kinetic Effects of Tumor Hypoxia, DNA Damage Repair, and Tumor Cell Repopulation, INTERNATIONAL JOURNAL OF RADIATION ONCOLOGY BIOLOGY PHYSICS 2009; 75:S615-S6.
10. Brown J.M., The Hypoxic Cell: A Target For Selective Cancer Therapy - Eighteenth Bruce F. Cain Memorial Award Lecture, CANCER RESEARCH 1999; 59:5863-70.
11. Dietz A., Rudat V., Vanselow B., et al. Rise Of Oxygenation In Cervical Lymph Node Metastasis During The Initial Course Of Radiochemotherapy, OTOLARYNGOLOGY-HEAD AND NECK SURGERY 1999; 121:789-96.
12. Evans S.M., Jenkins W.T., Shapiro M., Koch C.J., Evaluation Of The Concept Of "Hypoxic Fraction" As A Descriptor Of Tumor Oxygenation Status, ADV. EXP. MED. BIOL 1997; 411:215-25, OXYGEN TRANSPORT TO TISSUE XVIII (Nemoto E.M., LaManna J.C. eds.)(1997).
13. Teicher B.A., Physiological-Mechanisms of Therapeutic Resistance - Blood-Flow and Hypoxia, HEMATOLOGY-ONCOLOGY CLINICS OF NORTH AMERICA 1995; 9:475-506.
14. Kaelin W.G., ROS: Really Involved In Oxygen Sensing. Cell Metabolism 2005; 1:357-8.
15. Alarcon R., Koumenis C., Geyer R.K., Maki C.G., Giaccia A.J., Hypoxia Induces Accumulation Through MDM2 Down-Regulation And Inhibition Of E6-Mediated Degradation, Cancer Research 1999; 59:6046-51.
16. Graeber T.G., Osmanian C., Jacks T., et al. Hypoxia-Mediated Selection Of Cells With Diminished Apoptotic Potential In Solid Tumors, Nature 1996; 379:88-91.
17. Rockwell S., Oxygen Delivery: Implications For The Biology And Therapy Of Solid Tumors, Oncology Research 1997; 9:383-90.
18. Brizel D.M., Dodge R.K., Clough R.W., Dewhirst M.W., Oxygenation Of Head And Neck Cancer: Changes During Radiotherapy And Impact On Treatment Outcome, Radiotherapy and Oncology 1999; 53:113-7.
19. Brizel D.M., Sibley G.S., Prosnitz L.R., Scher R.L., Dewhirst M.W., Tumor Hypoxia Adversely Affects The Prognosis Of Carcinoma Of The Head And Neck, International Journal of Radiation Oncology Biology Physics 1997; 38:285-9.
20. Fyles A.W., Milosevic M., Wong R., et al., Oxygenation Predicts Radiation Response And Survival In Patients With Cervix Cancer. Radiotherapy And Oncology 1998; 48:149-56.
21. Hockel M., Knoop C., Schlenger K., et al., Intratumoral Po2 Predicts Survival In Advanced Cancer Of The Uterine Cervix, Radiotherapy and Oncology 1993; 26:45-50.
22. Hockel M., Schlenger K., Aral B., Mitze M., Schaffer U., Vaupel P., Association Between Tumor Hypoxia And Malignant Progression In Advanced Cancer Of The Uterine Cervix, Cancer Research 1996; 56:4509-15.
23. Hockel M., Vorndran B., Schlenger K., Baussmann E., Knapstein P.G., Tumor Oxygenation - A New Predictive Parameter In Locally Advanced Cancer Of The Uterine Cervix, Gynecologic Oncology 1993; 51:141-9.
24. Knocke T.H., Weitmann H.D., Feldmann H.J., Selzer E., Potter R., Intratumoral Po(2)-Measurements As Predictive Assay In The Treatment Of Carcinoma Of The Uterine Cervix, Radiotherapy and Oncology 1999; 53:99-104.
25. Rofstad E.K., Sundfor K., Lyng H., Trope C.G., Hypoxia-Induced Treatment Failure In Advanced Squamous Cell Carcinoma Of The Uterine Cervix Is Primarily Due To Hypoxia Induced Radiation Resistance Rather Than Hypoxia-Induced Metastasis, British Journal of Cancer 2000; 83:354-9.
26. Rudat V., Vanselow B., Wollensack P., et al. Repeatability And Prognostic Impact Of The Pretreatment Po(2) Histography In Patients With Advanced Head And Neck Cancer, RADIOTHERAPY AND ONCOLOGY 2000; 57:31-7.
27. Stadler P., Becker A., Feldmann H.J., et al., Influence Of The Hypoxic Subvolume On The Survival Of Patients With Head And Neck Cancer, INTERNATIONAL JOURNAL OF RADIATION ONCOLOGY BIOLOGY PHYSICS 1999; 44:749-54.
28. Stadler P., Feldmann H.J., Creighton C., Kau R., Molls M., Changes In Tumor Oxygenation During Combined Treatment With Split-Course Radiotherapy And Chemotherapy In Patients With Head And Neck Cancer, RADIOTHERAPY AND ONCOLOGY 1998; 48:157-64.
29. Karar J., Maity A., Modulating the Tumor Microenvironment to Increase Radiation Responsiveness, CANCER BIOLOGY & THERAPY 2009; 8:1994-2001.
30. Bache M., Kappler M., Said H.M., Staab A., Vordermark D., Detection And Specific Targeting Of Hypoxic Regions Within Solid Tumors: Current Preclinical And Clinical Strategies, CURRENT MEDICINAL CHEMISTRY 2008; 15:322-38.
31. Ahn G.O., Brown M., Targeting Tumors With Hypoxia-Activated Cytotoxins, FRONTIERS IN BIOSCIENCE 2007; 12:3483-501.
32. Nagasawa H., Uto Y., Kirk K.L., Hori H., Design Of Hypoxia-Targeting Drugs As New Cancer Chemotherapeutics. BIOLOGICAL & PHARMACEUTICAL BULLETIN 2006; 29:2335-42.
33. Janssen H.L., Haustermans K.M., Balm A.J., Begg A.C., Hypoxia In Head And Neck Cancer: How Much, How Important? HEAD AND NECK-JOURNAL FOR THE SCIENCES AND SPECIALTIES OF THE HEAD AND NECK 2005; 27:622-38.
34. Wouters B.G., Weppler S.A., Koritzinsky M., et al., Hypoxia As A Target For Combined Modality Treatments, EUROPEAN JOURNAL OF CANCER 2002; 38:240-57.
35. Kondo A., Safaei R., Mishima M., Niedner H., Lin X.J., Howell S.B., Hypoxia-Induced Enrichment And Mutagenesis Of Cells That Have Lost DNA Mismatch Repair, CANCER RESEARCH 2001; 61:7603-7.
36. Grigsby P.W., Winter K., Wasserman T.H., et al., Irradiation With Or Without Misonidazole For Patients With Stages IIIB And IVA Carcinoma Of The Cervix: Final Results of RTOG 80-05. INTERNATIONAL JOURNAL OF RADIATION ONCOLOGY BIOLOGY PHYSICS 1999; 44:513-7.
37. Lee D.J., Cosmatos D., Marcial V.A., et al., Results Of An RTOG Phase-III Trial (RTOG-85-27) Comparing Radiotherapy Plus Etanidazole With Radiotherapy Alone For Locally Advanced Head And Neck Carcinomas, INTERNATIONAL JOURNAL OF RADIATION ONCOLOGY BIOLOGY PHYSICS 1995; 32:567-76.
38. Lee D.J., Pajak T.F., Stetz J., Order S.E., Weissberg J.B., Fischer J.J., A Phase-I Phase-II Study Of The Hypoxic Cell Sensitizer Misonidazole As An Adjunct To High Fractional Dose Radiotherapy In Patients With Unresectable Squamous-Cell Carcinoma Of The Head And Neck - A RTOG Randomized Study (79-04), INTERNATIONAL JOURNAL OF RADIATION ONCOLOGY BIOLOGY PHYSICS 1989; 16:465-70.
39. Overgaard J., Sensitization Of Hypoxic Tumor-Cells - Clinical-Experience, INTERNATIONAL JOURNAL OF RADIATION BIOLOGY 1989; 56:801-11.
40. Overgaard J., Bentzen S.M., Kolstad P., et al., Misonidazole Combined With Radiotherapy In The Treatment Of Carcinoma Of The Uterine Cervix, INTERNATIONAL JOURNAL OF RADIATION ONCOLOGY BIOLOGY PHYSICS 1989; 16:1069-72.
41. Overgaard J., Hansen H.S., Andersen A.P., et al. Misonidazole Combined With Split-Course Radiotherapy In The Treatment Of Invasive-Carcinoma Of Larynx And Pharynx - Report From The Dahanca-2 Study, INTERNATIONAL JOURNAL OF RADIATION ONCOLOGY BIOLOGY PHYSICS 1989; 16:1065-8.
42. Overgaard J., Hansen H.S., Overgaard M., et al., A Randomized Double-Blind Phase III Study Of Nimorazole As A Hypoxic Radiosensitizer Of Primary Radiotherapy In Supraglottic Larynx And Pharynx Carcinoma, Results Of The Danish Head And Neck Cancer Study (DAHANCA) Protocol 5-85, RADIOTHERAPY AND ONCOLOGY 1998; 46:135-46.
43. Wasserman T.H., Lee D.J., Cosmatos D., et al., Clinical-Trials With Etanidazole (Sr-2508) By The Radiation-Therapy Oncology Group (RTOG), RADIOTHERAPY AND ONCOLOGY 1991; 20:129-35.
44. Lim SH, Lee JY, Park SH, et al. Effect of Combination of Anticancer Agents and Nitroimidazoles on the Survival of Human Hepatocellular Carcinoma Cells under Hypoxic Conditions, JOURNAL OF THE KOREAN SURGICAL SOCIETY 2009; 76:337-47.
45. Fenton B.M., Lord E.M., Paoni S.E., Enhancement Of Tumor Perfusion And Oxygenation By Carbogen And Nicotinamide During Single- And Multifraction Irradiation, RADIATION RESEARCH 2000; 153:75-83.
46. Rosenthal D.I., Nurenberg P., Becerra C.R., et al. A Phase I Single-Dose Trial Of Gadolinium Texaphyrin (Gd-Tex), A Tumor Selective Radiation Sensitizer Detectable By Magnetic Resonance Imaging, CLINICAL CANCER RESEARCH 1999; 5:739-45.
47. Eisbruch A., Robertson J.M., Johnston C.M., et al., Bromodeoxyuridine Alternating With Radiation For Advanced Uterine Cervix Cancer: A Phase I And Drug Incorporation Study, JOURNAL OF CLINICAL ONCOLOGY 1999; 17:31-40.
48. Rischin D., Peters L.J., O'Sullivan B., et al., Tirapazamine, Cisplatin, and Radiation Versus Cisplatin and Radiation for Advanced Squamous Cell Carcinoma of the Head and Neck (TROG 02.02, HeadSTART): A Phase III Trial of the Trans-Tasman Radiation Oncology Group, JOURNAL OF CLINICAL ONCOLOGY 2010; 28:2989-95.
49. Fogh S., Machtay M., Werner-Wasik M., et al., Phase I Trial Using Patupilone (Epothilone B) And Concurrent Radiotherapy For Central Nervous System Malignancies, INTERNATIONAL JOURNAL OF RADIATION ONCOLOGY BIOLOGY PHYSICS 2010; 77:1009-16.
50. de la Fouchardiere C., Negrier S., Labrosse H., et al., Phase I Study Of Daily Irinotecan As A Radiation Sensitizer For Locally Advanced Pancreatic Cancer, INTERNATIONAL JOURNAL OF RADIATION ONCOLOGY BIOLOGY PHYSICS 2010; 77:409-13.
51. Williams K.J., Albertella M.R., Fitzpatrick B., et al., In Vivo Activation Of The Hypoxia-Targeted Cytotoxin AQ4N In Human Tumor Xenografts, MOLECULAR CANCER THERAPEUTICS 2009; 8:3266-75.
52. Hay M.P., Pruijn F.B., Gamage S.A., et al., DNA-Targeted 1,2,4-Benzotriazine 1,4-Dioxides: Potent Analogues Of The Hypoxia-Selective Cytotoxin Tirapazamine, JOURNAL OF MEDICINAL CHEMISTRY 2004; 47:475-88.
53. Shibamoto Y., Zhou L., Hatta H., Mori M., Nishimoto S., In Vivo Evaluation Of A Novel Antitumor Prodrug, 1-(2'-Oxopropyl)-5-Fluorouracil (OFU001), Which Releases 5-Fluorouracil Upon Hypoxic Irradiation, INTERNATIONAL JOURNAL OF RADIATION ONCOLOGY BIOLOGY PHYSICS 2001; 49:407-13.
54. Koch C.J., Hahn S.M., Rockwell K., Covey J.M., McKenna W.G., Evans S.M., Pharmacokinetics Of EF5 2-(2-Nitro-1-H-Imidazol-1-Yl)-N-(2,2,3,3,3-Pentafluoropropyl) Acetamide In Human Patients: Implications For Hypoxia Measurements In Vivo By 2-Nitrolmidazoles, CANCER CHEMOTHER PHARMACOL 2001; 48:177-87.
55. von Pawel J., von Roemeling R., Gatzemeier U., et al., Tirapazamine Plus Cisplatin Versus Cisplatin In Advanced Non-Small-Cell Lung Cancer: A Report Of The International CATAPULT I Study Group, JOURNAL OF CLINICAL ONCOLOGY 2000; 18:1351-9.
56. Roberts K.B., Urdaneta N., Vera R., et al., Interim Results Of A Randomized Trial Of Mitomycin C As An Adjunct To Radical Radiotherapy In The Treatment Of Locally Advanced Squamous-Cell Carcinoma Of The Cervix, INTERNATIONAL JOURNAL OF CANCER 2000; 90:206-23.
57. Papadopoulou M.V., Ji M., Rao M.K., Bloomer W.D., 4- 3-(2-Nitro-1-Imidazolyl)Propylamino -7-Chloroquinoline Hydrochloride (NLCQ-1), A Novel Bioreductive Compound As A Hypoxia-Selective Cytotoxin, ONCOLOGY RESEARCH 2000; 12:185-92.
58. Papadopoulou M.V., Ji M., Rao M.K., Bloomer W.D., 4- 3-(2-Nitro-1-Imidazolyl)Propylamino -7-Chloroquinoline Hydrochloride (NLCQ-1), A Novel Bioreductive Agent As Radiosensitizer In Vitro And In Vivo: Comparison With Tirapazamine, ONCOLOGY RESEARCH 2000; 12:325-33.
59. Craighead P.S., Pearcey R., Stuart G., A Phase I/II Evaluation Of Tirapazamine Administered Intravenously Concurrent With Cisplatin And Radiotherapy In Women With Locally Advanced Cervical Cancer, INTERNATIONAL JOURNAL OF RADIATION ONCOLOGY BIOLOGY PHYSICS 2000; 48:791-5.
60. Weitman S., Mangold G., Marty J., et al., Evidence Of Enhanced In Vivo Activity Using Tirapazamine With Paclitaxel And Paraplatin Regimens Against The MV-522 Human Lung Cancer Xenograft, CANCER CHEMOTHER PHARMACOL 1999; 43:402-8.
61. Treat J., Johnson E., Langer C., et al., Tirapazamine With Cisplatin In Patients With Advanced Non-Small-Cell Lung Cancer: A Phase II Study, JOURNAL OF CLINICAL ONCOLOGY 1998; 16:3524-7.
62. Siemann D.W., Hinchman C.A., Potentiation Of Cisplatin Activity By The Bioreductive Agent Tirapazamine, RADIOTHERAPY AND ONCOLOGY 1998; 47:215-20.
63. Lee D.J., Trotti A., Spencer S., et al., Concurrent Tirapazamine And Radiotherapy For Advanced Head And Neck Carcinomas: A Phase II Study, INTERNATIONAL JOURNAL OF RADIATION ONCOLOGY BIOLOGY PHYSICS 1998; 42:811-5.
64. Harrison L.B., Raben A., Pfister D.G., et al. A Prospective Phase II Trial Of Concomitant Chemotherapy And Radiotherapy With Delayed Accelerated Fractionation In Unresectable Tumors Of The Head And Neck, HEAD AND NECK-JOURNAL FOR THE SCIENCES AND SPECIALTIES OF THE HEAD AND NECK 1998; 20:497-503.
65. Gatzemeier U., Rodriguez G., Treat J., et al., Tirapazamine-Cisplatin: The Synergy, BRITISH JOURNAL OF CANCER 1998; 77:15-7.
66. Brown J.M., Wang L.H., Tirapazamine: Laboratory Data Relevant To Clinical Activity, ANTI-CANCER DRUG DESIGN 1998; 13:529-39.
67. Haffty B.G., Son Y.H., Papac R., et al., Chemotherapy As An Adjunct To Radiation In The Treatment Of Squamous Cell Carcinoma Of The Head And Neck: Results Of The Yale Mitomycin Randomized Trials, JOURNAL OF CLINICAL ONCOLOGY 1997; 15:268-76.
68. Adelstein D.J., Saxton J.P., Lavertu P., et al., A Phase III Randomized Trial Comparing Concurrent Chemotherapy And Radiotherapy With Radiotherapy Alone In Resectable Stage III And IV Squamous Cell Head And Neck Cancer: Preliminary Results, HEAD AND NECK-JOURNAL FOR THE SCIENCES AND SPECIALTIES OF THE HEAD AND NECK 1997; 19:567-75.
69. Sartorelli A.C., Hodnick W.F., Belcourt M.F., et al., Mitomycin-C - A Prototype Bioreductive Agent, ONCOLOGY RESEARCH 1994; 6:501-8.
70. Dobrowsky W., Mitomycin-C, 5-Fluorouracil And Radiation In Advanced, Locally Recurrent Rectal-Cancer, BRITISH JOURNAL OF RADIOLOGY 1992; 65:143-7.
71. Sivanesaratnam V., Jayalakshmi P., Mitomycin-C Adjuvant Chemotherapy After Wertheim Hysterectomy For Stage-Ib Cervical-Cancer, CANCER 1989; 64:798-800.
72. Keyes S.R., Rockwell S., Sartorelli A.C., Enhancement Of Mitomycin-C CytoToxicity To Hypoxic Tumor-Cells By Dicoumarol Invivo And Invitro. CANCER RESEARCH 1985; 45:213-6.
73. Keyes S.R, Rockwell S., Sartorelli A.C., Porfiromycin As A Bioreductive Alkylating Agent With Selective Toxicity To Hypoxic Emt6 Tumor-Cells Invivo And Invitro, CANCER RESEARCH 1985; 45:3642-5.
74. Overgaard J., Horsman M.R., Modification Of Hypoxia-Induced Radioresistance In Tumors By The Use Of Oxygen And Sensitizers, SEMINARS IN RADIATION ONCOLOGY 1996; 6:10-21.
75. Aquino-Parsons C., Lim P., Green A., Minchinton A.I., Carbogen Inhalation In Cervical Cancer: Assessment Of Oxygenation Change, GYNECOLOGIC ONCOLOGY 1999; 74:259-64.
76. Bernier J., Denekamp J., Rojas A., et al., ARCON: Accelerated Radiotherapy With Carbogen And Nicotinamide In Head And Neck Squamous Cell Carcinomas: The Experience Of The Cooperative Group Of Radiotherapy Of The European Organization For Research And Treatment Of Cancer (EORTC), RADIOTHERAPY AND ONCOLOGY 2000; 55:111-9.
77. Bussink J., Kaanders J., Van der Kogel A.J., Clinical Outcome And Tumour Microenvironmental Effects Of Accelerated Radiotherapy With Carbogen And Nicotinamide, ACTA ONCOLOGICA 1999; 38:875-82.
78. Hoskin P.J., Saunders M.I., Dische S., Hypoxic Radiosensitizers In Radical Radiotherapy For Patients With Bladder Carcinoma - Hyperbaric Oxygen, Misonidazole, And Accelerated Radiotherapy, Carbogen, And Nicotinamide, CANCER 1999; 86:1322-8.
79. Miralbell R., Mornex F., Greiner R., et al., Accelerated Radiotherapy, Carbogen, And Nicotinamide In Glioblastoma Multiforme: Report Of European Organization For Research And Treatment Of Cancer Trial 22933, JOURNAL OF CLINICAL ONCOLOGY 1999; 17:3143-9.
80. Saunders M., Dische S., Clinical Results Of Hypoxic Cell Radiosensitisation From Hyperbaric Oxygen To Accelerated Radiotherapy, Carbogen And Nicotinamide, BRITISH JOURNAL OF CANCER 1996; 74:S271-S8.
81. Stuben G., Stuschke M., Knuhmann K., Horsman M.R., Sack H., The Effect Of Combined Nicotinamide And Carbogen Treatments In Human Tumour Xenografts: Oxygenation And Tumour Control Studies, RADIOTHERAPY AND ONCOLOGY 1998; 48:143-8.
82. Zhan H.W., Liu H.B., Bao C.K., Ye X.J., Zhang H., He G.Q., Effect Of Carbogen On Tumour Oxygenation And 32P-Colloid Interstitial Irradiation Response, MEDICAL SCIENCE MONITOR 2010; 16:BR11-BR6.
83. Yu M.H., Dai M., Liu Q., Xiu R.J., Oxygen Carriers And Cancer Chemo- And Radiotherapy Sensitization: Bench To Bedside And Back, CANCER TREATMENT REVIEWS 2007; 33:757-61.
84. Hoogsteen I.J., Marrest A.M., van der Kogel A.J., Kaanders J., The Hypoxic Tumour Microenvironment, Patient Selection And Hypoxia-Modifying Treatments, CLINICAL ONCOLOGY 2007; 19:385-96.
85. Shasha D., The Negative Impact Of Anemia On Radiotherapy And Chemoradiation Outcomes, SEMINARS IN HEMATOLOGY 2001; 38:8-15.
86. Shasha D., George M.J., Harrison L.B., Once-Weekly Dosing Of Epoetin Alfa Increases Hemoglobin And Improves Quality Of Life In Anemic Cancer Patients Receiving Radiation Therapy Either Concomitantly Or Sequentially With Chemotherapy, BLOOD 2000; 96:1866.
87. Henke M., Guttenberger R., Barke A., Pajonk F., Potter R., Frommhold H., Erythropoietin For Patients Undergoing Radiotherapy: A Pilot Study, RADIOTHERAPY AND ONCOLOGY 1999; 50:185-90.
88. Dusenbery K.E., McGuire W.A., Holt P.J., et al., Erythropoietin Increases Hemoglobin During Radiation-Therapy For Cervical-Cancer, INTERNATIONAL JOURNAL OF RADIATION ONCOLOGY BIOLOGY PHYSICS 1994; 29:1079-84.
89. Lavey R.S., Dempsey W.H., Erythropoietin Increases Hemoglobin In Cancer-Patients During Radiation-Therapy, INTERNATIONAL JOURNAL OF RADIATION ONCOLOGY BIOLOGY PHYSICS 1993; 27:1147-52.
90. McGee M.C., Hamner J.B., Williams R.F., et al., Improved Intratumoral Oxygenation Through Vascular Normalization Increases Glioma Sensitivity To Ionizing Radiation, INTERNATIONAL JOURNAL OF RADIATION ONCOLOGY BIOLOGY PHYSICS 2010; 76:1537-45.
91. Katz D., Ito E., Liu F.F., On The Path To Seeking Novel Radiosensitizers, INTERNATIONAL JOURNAL OF RADIATION ONCOLOGY BIOLOGY PHYSICS 2009; 73:988-96.
92. Gali-Muhtasib H., Sidani M., Geara F., et al., Quinoxaline 1,4-Dioxides Are Novel Angiogenesis Inhibitors That Potentiate Antitumor Effects Of Ionizing Radiation, INTERNATIONAL JOURNAL OF ONCOLOGY 2004; 24:1121-31.
93. Ordway G.A., Garry D.J., Myoglobin: An Essential Hemoprotein In Striated Muscle, JOURNAL OF EXPERIMENTAL BIOLOGY 2004; 207:3441-6.
94. Wittenberg J.B., Wittenberg B.A., Myoglobin Function Reassessed, JOURNAL OF EXPERIMENTAL BIOLOGY 2003; 206:2011-20.
95. Kooyman G.L., Ponganis P.J., The Physiological Basis Of Diving To Depth: Birds And Mammals, ANNUAL REVIEW OF PHYSIOLOGY 1998; 60:19-32.
96. Hochachka P.W., The Metabolic Implications Of Intracellular Circulation, PROCEEDINGS OF THE NATIONAL ACADEMY OF SCIENCES OF THE UNITED STATES OF AMERICA 1999; 96:12233-9.
97. Conley K.E., Jones C., Myoglobin Content And Oxygen Diffusion: Model Analysis Of Horse And Steer Muscle, AMERICAN JOURNAL OF PHYSIOLOGY-CELL PHYSIOLOGY 1996; 271:C2027-C36.
98. Galluzzo M., Pennacchietti S., Rosano S., Comoglio P.M., Michieli P., Prevention Of Hypoxia By Myoglobin Expression In Human Tumor Cells Promotes Differentiation And Inhibits Metastasis, JOURNAL OF CLINICAL INVESTIGATION 2009; 119:865-75.
99. Floegel U., Dang C.V., Myoglobin Tames Tumor Growth And Spread, JOURNAL OF CLINICAL INVESTIGATION 2009; 119:766-8.
100. Bunn H.F., The Role Of Hemoglobin Based Substitutes In Transfusion Medicine, TRANSFUS CLIN. BIOL. 1995; 2:433-9.
101. Gottschalk A., Raabe A., Hommel M., Rempf C., Freitag M., Stand1 T., Influence Of The Hemoglobin Solution HBOC-201 On Tissue Oxygenation In The Rat R1H-Tumor, ARTIF. CELLS BLOOD SUBSTIT. BIOTECHNOL. 2005; 33:379-89.
102. Gundersen S.I., Palmer A.F., Hemoglobin-Based Oxygen Carrier Enhanced Tumor Oxygenation: A Novel Strategy for Cancer Therapy, BIOTECHNOL. PROG. 2008; 24:1353-64.
103. Palaparthy R., Wang H.S., Gulati A., Current Aspects In Pharmacology Of Modified Hemoglobins, ADV DRUG DELIV. REV 2000; 40:185-98.
104. Robinson M.F., Dupuis N.P., Kusumoto T., Liu F., Menon K., Teicher B.A., Increased Tumor Oxygenation And Radiation Sensitivity In 2 Rat-Tumors By A Hemoglobin-Based, Oxygen-Carrying Preparation, ARTIF. CELLS BLOOD SUBSTIT. IMMOBIL. BIOTECHNOL. 1995; 23:431-8.
105. Rowinsky E.K., Novel Radiation Sensitizers Targeting Tissue Hypoxia, ONCOLOGY-NY 1999; 13:61-70.
106. Teicher B.A., Herman T.S., Hopkins R.E., Menon K., Effect Of A Bovine Hemoglobin Preparation On The Response Of The Fsaiic Fibrosarcoma To Chemotherapeutic Alkylating-Agents, J. CANCER RES. CLIN. ONCOL. 1992; 118:123-8.
107. Teicher B.A., Holden S.A., Dupuis N.P., et al., Oxygenation Of The Rat-9l Gliosarcoma And The Rat-13672 Mammary-Carcinoma With Various Doses Of A Hemoglobin Solution, ARTIF. CELLS BLOOD SUBSTIT. IMMOBIL. BIOTECHNOL. 1994; 22:827-33.
108. Teicher B.A., Holden S.A., Menon K., Hopkins R.E., Gawryl M.S., Effect Of Hemoglobin Solution On The Response Of Intracranial And Subcutaneous Tumors To Antitumor Alkylating-Agents, CANCER CHEMOTHER. PHARMACOL. 1993; 33:57-62.
109. Teicher B.A., Schwartz G.N., Sotomayor E.A., Robinson M.F., Dupuis N.P., Menon K., Oxygenation Of Tumors By A Hemoglobin Solution, J. CANCER RES. CLIN. ONCOL. 1993; 120:85-90.
110. Christian D.A., Cai S., Bowen D.M., Kim Y., Pajerowski J.D., Discher D.E., Polymersome Carriers: From Self-Assembly To siRNA And Protein Therapeutics, EUR. J. PHARM. BIOPHARM. 2009; 71:463-74.
111. Levine D.H., Ghoroghchian P.P., Freudenberg J., et al., Polymersomes: A New Multi-Functional Tool For Cancer Diagnosis And Therapy, METHODS 2008; 46:25-32.
112. Letchford K., Burt H., A Review Of The Formation And Classification Of Amphiphilic Block Copolymer Nanoparticulate Structures: Micelles, Nanospheres, Nanocapsules And Polymersomes, EUR. J. PHARM. BIOPHARM. 2007; 65:259-69.
113. Discher B.M., Hammer D.A., Bates F.S., Discher D.E., Polymer Vesicles In Various Media, CURR. OPIN. COLLOID INTERFACE SCI. 2000; 5:125-31.
114. Discher D.E., Ortiz V., Srinivas G., et al., Emerging Applications Of Polymersomes In Delivery: From Molecular Dynamics To Shrinkage Of Tumors, PROG. POLYM. SCI. 2007; 32:838-57.
115. Discher B.M., Won Y.Y., Ege D.S., et al., Polymersomes: Tough Vesicles Made From Diblock Copolymers, SCIENCE 1999; 284:1143-6.
116. O'Neil C.P., Suzuki T., Demurtas D., Finka A., Hubbell J.A., A Novel Method For The Encapsulation Of Biomolecules Into Polymersomes Via Direct Hydration, LANGMUIR 2009; 25:9025-9.
117. Lee J.C.M., Bermudez H., Discher B.M., et al., Preparation, Stability, And In Vitro Performance Of Vesicles Made With Diblock Copolymers, BIOTECHNOL. BIOENG. 2001; 73:135-45.
118. Ghoroghchian P.P., Li G.Z., Levine D.H., et al., Bioresorbable Vesicles Formed Through Spontaneous Self-Assembly Of Amphiphilic Poly(Ethylene Oxide)-Block-Polycaprolactone, MACROMOLECULES 2006; 39:1673-5.
119. Kim Y.H., Tewari M., Pajerowski J.D., et al., Polymersome Delivery Of siRNA And Antisense Oligonucleotides, J. CONTROL RELEASE 2009; 134:132-40.
120. Hearnden V., Lomas H., MacNeil S., et al., Diffusion Studies of Nanometer Polymersomes Across Tissue Engineered Human Oral Mucosa, PHARMACEUTICAL RESEARCH 2009; 26:1718-28.
121. Meng F.H., Engbers G.H.M., Feijen J., Biodegradable Polymersomes As A Basis For Artificial Cells: Encapsulation, Release And Targeting, J. CONTROL RELEASE 2005; 101:187-98.
122. Photos P.J., Bacakova L., Discher B., Bates F.S., Discher D.E., Polymer Vesicles In Vivo: Correlations With PEG Molecular Weight, J. CONTROL RELEASE 2003; 90:323-34.
123. Ghoroghchian P.P., Lin J.J., Brannan A.K., et al., Quantitative Membrane Loading Of Polymer Vesicles, SOFT MATTER 2006; 2:973-80.
124. Ghoroghchian P.P., Frail P.R., Susumu K., et al., Near-Infrared-Emissive Polymersomes: Self-Assembled Soft Matter For In Vivo Optical Imaging, PROC NATL. ACAD. SCI. U.S.A. 2005; 102:2922-7.
125. Bermudez H., Hammer D.A., Discher D.E., Effect Of Bilayer Thickness On Membrane Bending Rigidity, LANGMUIR 2004; 20:540-3.
126. Bermudez H., Brannan A.K., Hammer D.A., Bates F.S., Discher D.E., Molecular Weight Dependence Of Polymersome Membrane Structure, Elasticity, And Stability, MACROMOLECULES 2002; 35:8203-8.
127. Massignani M., LoPresti C., Blanazs A., et al., Controlling Cellular Uptake by Surface Chemistry, Size, and Surface Topology at the Nanoscale, SMALL 2009; 5:2424-32.
128. Blanazs A., Massignani M., Battaglia G., Armes S.P., Ryan A.J., Tailoring Macromolecular Expression at Polymersome Surfaces, ADVANCED FUNCTIONAL MATERIALS 2009; 19:2906-14.
129. van Dongen S.F.M., Nallani M., Schoffelen S., Cornelissen J., Nolte R.J.M., van Hest J.C.M., A Block Copolymer For Functionalisation Of Polymersome Surfaces, MACROMOLECULAR RAPID COMMUNICATIONS 2008; 29:321-5.
130. Christian N.A., Milone M.C., Ranka S.S., et al., Tat-Functionalized Near-Infrared Emissive Polymersomes For Dendritic Cell Labeling, BIOCONJUGATE. CHEM. 2007; 18:31-40.
131. Binder W.H., Sachsenhofer R, Farnik D, Blaas D. Guiding The Location Of Nanoparticles Into Vesicular Structures: A Morphological Study, PHYSICAL CHEMISTRY CHEMICAL PHYSICS 2007; 9:6435-41.
132. Lin J.J., Ghoroghchian P., Zhang Y., Hammer D.A., Adhesion Of Antibody-Functionalized Polymersomes, LANGMUIR 2006; 22:3975-9.
133. Lin J.J., Silas J.A., Bermudez H., Milam V.T., Bates F.S., Hammer D.A., The Effect Of Polymer Chain Length And Surface Density On The Adhesiveness Of Functionalized Polymersomes, LANGMUIR 2004; 20:5493-500.
134. Pangu G.D., Davis K.P., Bates F.S., Hammer D.A., Ultrasonically Induced Release from Nanosized Polymer Vesicles, MACROMOL. BIOSCI. 2010; 10:546-54.
135. Kim M.S., Lee D.S., Biodegradable And Ph-Sensitive Polymersome With Tuning Permeable Membrane For Drug Delivery Carrier, CHEMICAL COMMUNICATIONS 2010; 46:4481-3.
136. Chen W., Meng F.H., Cheng R., Zhong Z.Y., pH-Sensitive Degradable Polymersomes For Triggered Release Of Anticancer Drugs: A Comparative Study With Micelles, J CONTROL RELEASE 2010; 142:40-6.
137. Robbins G.P., Jimbo M., Swift J., Therien M.J., Hammer D.A., Dmochowski I.J., Photoinitiated Destruction of Composite Porphyrin-Protein Polymersomes, J AM CHEM SOC 2009; 131:3872-+.
138. Kim K.T., Cornelissen J., Nolte R.J.M., van Hest J.C.M., A Polymersome Nanoreactor with Controllable Permeability Induced by Stimuli-Responsive Block Copolymers, ADVANCED MATERIALS 2009; 21:2787.
139. Sanson C., Le Meins J.F., Schatz C., Soum A., Lecommandoux S., Temperature Responsive Poly(Trimethylene Carbonate)-Block-Poly(L-Glutamic Acid) Copolymer: Polymersomes Fusion And Fission, SOFT MATTER 2010; 6:1722-30.
140. Castillo R.V., Muller A.J., Raquez J.M., Dubois P., Crystallization Kinetics and Morphology of Biodegradable Double Crystalline PLLA-b-PCL Diblock Copolymers, MACROMOLECULES 2010; 43:4149-60.
141. Wang F., Wang Y.C., Yan L.F., Wang J., Biodegradable Vesicular Nanocarriers Based On Poly(Epsilon-Caprolactone)-Block-Poly(Ethyl Ethylene Phosphate) For Drug Delivery, POLYMER 2009; 50:5048-54.
142. Schatz C., Louguet S., Le Meins J.F., Lecommandoux S., Polysaccharide-Block-Polypeptide Copolymer Vesicles: Towards Synthetic Viral Capsids, ANGEW. CHEM. INT. EDIT 2009; 48:2572-5.
143. Rabotyagova O.S., Cebe P., Kaplan D.L., Self-Assembly of Genetically Engineered Spider Silk Block Copolymers, BIOMACROMOLECULES 2009; 10:229-36.
144. Katz J.S., Levine D.H., Davis K.P., Bates F.S., Hammer D.A., Burdick J.A., Membrane Stabilization of Biodegradable Polymersomes, LANGMUIR 2009; 25:4429-34.
145. Bromley E.H.C, Channon K., Moutevelis E., Woolfson D.N., Peptide And Protein Building Blocks For Synthetic Biology: From Programming Biomolecules To Self-Organized Biomolecular Systems, Acs CHEMICAL BIOLOGY 2008; 3:38-50.
146. Najafi F., Sarbolouki M.N., Biodegradable Micelles/Polymersomes From Fumaric/Sebacic Acids And Poly(Ethylene Glycol), BIOMATERIALS 2003;24:1175-82.
147. Rameez S., Bamba I., Palmer A.F., Large Scale Production of Vesicles by Hollow Fiber Extrusion: A Novel Method for Generating Polymersome Encapsulated Hemoglobin Dispersions, LANGMUIR 2010; 26:5279-85.
148. Shum H.C., Kim J.W., Weitz D.A., Microfluidic Fabrication Of Monodisperse Biocompatible And Biodegradable Polymersomes With Controlled Permeability, J AM CHEM SOC 2008; 130:9543-9.
149. Yildiz M.E., Prud'homme R.K., Robb I., Adamson D.H., Formation And Characterization Of Polymersomes Made By A Solvent Injection Method, POLYMERS FOR ADVANCED TECHNOLOGIES 2007; 18:427-32.
150. Dewhirst M.W., Relationships Between Cycling Hypoxia, HIF-1, Angiogenesis And Oxidative Stress, RADIATION RESEARCH 2009; 172:653-65.
151. Palmer G.M., Boruta R.J., Viglianti B.L., Lan L., Spasojevic I., Dewhirst M.W., Non-Invasive Monitoring Of Intra-Tumor Drug Concentration And Therapeutic Response Using Optical Spectroscopy, J CONTROL RELEASE 2010; 142:457-64.
152. Dewhirst M.W., Thrall D.E., Palmer G., et al., Utility Of Functional Imaging In Prediction Or Assessment Of Treatment Response And Prognosis Following Thermotherapy, INTERNATIONAL JOURNAL OF HYPERTHERMIA 2010; 26:283-93.
153. Zhang G.Q., Palmer G.M., Dewhirst M., Fraser C.L., A Dual-Emissive-Materials Design Concept Enables Tumour Hypoxia Imaging, NATURE MATERIALS 2009; 8:747-51.
154. Palmer G.M., Viola R.J., Schroeder T., Yarmolenko P.S., Dewhirst M.W., Ramanujam N., Quantitative Diffuse Reflectance And Fluorescence Spectroscopy: Tool To Monitor Tumor Physiology In Vivo, JOURNAL OF BIOMEDICAL OPTICS 2009; 14.
155. Hardee M.E., Dewhirst M.W., Agarwal N., Sorg B.S., Novel Imaging Provides New Insights into Mechanisms of Oxygen Transport in Tumors, CURRENT MOLECULAR MEDICINE 2009; 9:435-41.
156. Sorg B.S., Hardee M.E., Agarwal N., Moeller B.J., Dewhirst M.W., Spectral Imaging Facilitates Visualization And Measurements Of Unstable And Abnormal Microvascular Oxygen Transport In Tumors, JOURNAL OF BIOMEDICAL OPTICS 2008; 13.
157. Dewhirst M.W., Cao Y., Moeller B., Cycling Hypoxia And Free Radicals Regulate Angiogenesis And Radiotherapy Response, NATURE REVIEWS CANCER 2008; 8:425-37.
158. Sorg B.S., Moeller B.J., Donovan O., Cao Y.T., Dewhirst M.W., Hyperspectral Imaging Of Hemoglobin Saturation In Tumor Microvasculature And Tumor Hypoxia Development, JOURNAL OF BIOMEDICAL OPTICS 2005; 10.
159. Dewhirst M.W., Cao Y.T., Li C.Y., Moeller B., Exploring The Role Of HIF-1 In Early Angiogenesis And Response To Radiotherapy, RADIOTHERAPY AND ONCOLOGY 2007; 83:249-55.
160. Moeller B., Dewhirst M.W., HIF-I and tumour radiosensitivity, BRITISH JOURNAL OF CANCER 2006; 95:1-5.
161. Moeller B.J., Dreher M.R., Rabbani Z.N., et al., Pleiotropic Effects Of HIF-1 Blockade On Tumor Radiosensitivity, CANCER CELL 2005; 8:99-110.
162. Arifin D.R., Palmer A.F., Polymersome Encapsulated Hemoglobin: A Novel Type Of Oxygen Carrier, BIOMACROMOLECULES 2005; 6:2172-81.
163. Rameez S., Alosta H., Palmer A.F., Biocompatible And Biodegradable Polymersome Encapsulated Hemoglobin: A Potential Oxygen Carrier, BIOCONJUGATE CHEM 2008; 19:1025-32.
164. Ghoroghchian P.P., Therien M.J., Hammer D.A., In Vivo Fluorescence Imaging: A Personal Perspective, WILEY INTERDISCIP. REV-NANOMED NANOBIOTECHNOL. 2009; 1:156-67.
165. Duncan T.V., Ghoroghchian P.P., Rubtsov I.V., Hammer D.A., Therien M.J., Ultrafast Excited-State Dynamics Of Nanoscale Near-Infrared Emissive Polymersomes, J AM. CHEM. SOC 2008; 130:9773-84.
166. Ghoroghchian P.P., Frail P.R., Li G.Z., et al., Controlling Bulk Optical Properties Of Emissive Polymersomes Through Intramembranous Polymer-Fluorophore Interactions, CHEM. MAT 2007; 19:1309-18.
167. Ghoroghchian P.P., Frail P.R., Susumu K., et al., Broad Spectral Domain Fluorescence Wavelength Modulation Of Visible And Near-Infrared Emissive Polymersomes, J. AM. CHEM. SOC. 2005; 127:15388-90.
168. Christian N.A., Benencia F., Milone M.C., et al., In Vivo Dendritic Cell Tracking Using Fluorescence Lifetime Imaging and Near-Infrared-Emissive Polymersomes, MOL. IMAGING BIOL. 2009; 11:167-77.
169. Hearnden V., Battaglia G., MacNeil S., Murdoch C., Thornhill M., Penetration Of Polymersome Drug And Gene Delivery Nanoparticles Into In Vitro Models Of Head And Neck Cancer And Tissue Engineered Oral Mucosa, ORAL ONCOLOGY 2009:66.
170. Li S.L., Byrne B., Welsh J., Palmer A.F., Self-Assembled Poly(Butadiene)-B-Poly(Ethylene Oxide) Polymersomes As Paclitaxel Carriers, BIOTECHNOL. PROG. 2007; 23:278-85.
171. Ahmed F., Pakunlu R.I., Brannan A., Bates F., Minko T., Discher D.E., Biodegradable Polymersomes Loaded With Both Paclitaxel And Doxorubicin Permeate And Shrink Tumors, Inducing Apoptosis In Proportion To Accumulated Drug, J. CONTROL RELEASE 2006; 116:150-8.
172. Zhang X.L., Liu C.S., Yuan Y., et al., Key Parameters Affecting The Initial Leaky Effect Of Hemoglobin-Loaded Nanoparticles As Blood Substitutes, JOURNAL OF MATERIALS SCIENCE-MATERIALS IN MEDICINE 2008; 19:2463-70.
173. Dewhirst M.W., Kimura H., Rehmus S.W.E., et al., Microvascular Studies On The Origins Of Perfusion-Limited Hypoxia, BRITISH JOURNAL OF CANCER 1996; 74:S247-S51.
174. Zupancich J.A., Bates F.S., Hillmyer M.A., Aqueous Dispersions Of Poly(Ethylene Oxide)-B-Poly(Gamma-Methyl-Epsilon-Caprolactone) Block Copolymers, MACROMOLECULES 2006; 39:4286-8.
175. Hahn J.S., Braun R.D., Dewhirst M.W., et al., Stroma-Free Human Hemoglobin A Decreases R3230Ac Rat Mammary Adenocarcinoma Blood Flow And Oxygen Partial Pressure, RADIATION RESEARCH 1997; 147:185-94.
176. Arifin D.R., Palmer A.F., Determination Of Size Distribution And Encapsulation Efficiency Of Liposome-Encapsulated Hemoglobin Blood Substitutes Using Asymmetric Flow Field-Flow Fractionation Coupled With Multi-Angle Static Light Scattering, BIOTECHNOL PROG 2003; 19:1798-811.
177. Sakai H., Sato A., Sobolewski P., et al., NO And CO Binding Profiles Of Hemoglobin Vesicles As Artificial Oxygen Carriers, BIOCHIMICA ET BIOPHYSICA ACTA-PROTEINS AND PROTEOMICS 2008; 1784:1441-7.
178. Sakai H., Hamada K., Takeoka S., Nishide H., Tsuchida E., Functional Evaluation Of Hemoglobin- And Lipidheme-Vesicles As Red Cell Substitutes, POLYMERS FOR ADVANCED TECHNOLOGIES 1996; 7:639-44.
179. Frauenfelder H., McMahon B.H., Fenimore P.W., Myoglobin: The Hydrogen Atom Of Biology And A Paradigm Of Complexity, PROCEEDINGS OF THE NATIONAL ACADEMY OF SCIENCES OF THE UNITED STATES OF AMERICA 2003; 100:8615-7.
180. Herold S., Exner M., Nauser T., Kinetic And Mechanistic Studies Of The NO Center Dot-Mediated Oxidation Of Oxymyoglobin And Oxyhemoglobin, BIOCHEMISTRY 2001; 40:3385-95.
181. Nienhaus G.U., Mourant J.R., Chu K., Frauenfelder H., Ligand-Binding To Heme-Proteins - The Effect Of Light On Ligand-Binding In Myoglobin, BIOCHEMISTRY 1994; 33:13413-30.
182. Ansari A., Jones C.M., Henry E.R., Hofrichter J., Eaton W.A., Conformational Relaxation And Ligand-Binding In Myoglobin, BIOCHEMISTRY 1994; 33:5128-45.
183. Mourant J.R., Braunstein D.P., Chu K., et al., Ligand-Binding To Heme-Proteins .2. Transitions In The Heme Pocket Of Myoglobin, BIOPHYSICAL JOURNAL 1993; 65:1496-507.
184. Kanner J., Harel S., Granit R., Nitric-Oxide As An Antioxidant, ARCHIVES OF BIOCHEMISTRY AND BIOPHYSICS 1991; 289:130-6.
185. Chance B., Optical Method. ANNUAL REVIEW OF BIOPHYSICS AND BIOPHYSICAL CHEMISTRY 1991; 20:1-28.
186. Chance B., Nioka S., Kent J., et al., Time-Resolved Spectroscopy Of Hemoglobin And Myoglobin In Resting And Ischemic Muscle, ANALYTICAL BIOCHEMISTRY 1988; 174:698-707.
187. Privalov P.L., Griko Y.V., Venyaminov S.Y., Kutyshenko V.P., Cold Denaturation Of Myoglobin, JOURNAL OF MOLECULAR BIOLOGY 1986; 190:487-98.
188. Helcke G.A., Ingram D.J.E., Slade E.F., Electron Resonance Studies Of Haemoglobin Derivatives .3. Line-Width And G-Value Measurements Of Acid-Met Myoglobin And Of Met Myoglobin Azide Derivatives, PROCEEDINGS OF THE ROYAL SOCIETY OF LONDON SERIES B-BIOLOGICAL SCIENCES 1968; 169:275.
189. Moeller B.J., Richardson R.A., Dewhirst M.W., Hypoxia And Radiotherapy: Opportunities For Improved Outcomes In Cancer Treatment, CANCER AND METASTASIS REVIEWS 2007; 26:241-8.
190. Kong G., Braun R.D., Dewhirst M.W., Characterization Of The Effect Of Hyperthermia On Nanoparticle Extravasation From Tumor Vasculature, CANCER RESEARCH 2001; 61:3027-32.
191. Kong G., Braun R.D., Dewhirst M.W., Hyperthermia Enables Tumor-Specific Nanoparticle Delivery: Effect Of Particle Size, CANCER RESEARCH 2000; 60:4440-5.
192. Moeller B.J., Dewhirst M.W., Raising The Bar - How HIF-1 Helps Determine Tumor Radiosensitivity, CELL CYCLE 2004; 3:1107-10.

### SUMMARY OF THE INVENTION

The invention relates to a high-oxygen affinity agent for use in a method of increasing efficacy of radiation or chemotherapy applied to a tumor in a subject, said method comprising:delivering said high-oxygen affinity agent to the tumor by administering to the subject an oxygen carrier that incorporates the high-oxygen affinity agent, wherein:the high-oxygen affinity agent comprises a myoglobin composition; and oxygen partial pressure gradient characteristics of the high-oxygen affinity agent are such that oxygen is released from the high-oxygen affinity agent at oxygen tensions of less than 10 mmHg, wherein the released oxygen diffuses out of the oxygen carrier while the high-oxygen affinity agent remains within the oxygen carrier. Said high-affinity oxygen binding agent is suitable for being delivered to tumors in order to increase intratumoral partial pressures of oxygen, mitigate the natural selection of tumor cells that demonstrate aggressive molecular behavior and metastatic potential, and potentiate the effects of radiation and chemotherapies.

The various embodiments include compositions of matter and methods to develop an advanced slowly biodegradable, myoglobin-based oxygen carrier (MBOC) that utilizes a nanoparticle-based delivery vehicle. In some embodiments, the nanoparticle-based delivery vehicle is a self-assembled biodegradable polymeric vesicle called the polymersome. Polymersome technology allows for effective segregation of myoglobin (Mb) from surrounding tissues and blood components, thereby avoiding the toxic effects of myoglobin. Further, *in situ* manipulation of the polymersomes' physicochemical properties influences its *in vivo* pharmacokinetic and pharmacodynamic profiles to enable optimized oxygen delivery to tumor tissues. Polymersome-derived MBOC constructs exhibit the requisite Mb encapsulation efficiency, oxygen binding characteristics, colloidal and rheological properties, as well as the chemical and mechanical stability necessary for effective *in vivo* oxygen delivery to tumors.

The various embodiments provide polymersome-encapsulated Mb (PEM) formulations that are comprised of diblock copolymers of polyethylene oxide (PEO) and either poly (ε-caprolactone) (PCL), poly (γ-methyl ε-caprolactone) (PMCL), or poly (trimethylcarbonate) (PTMC). In some preferred embodiments, the polymersome-forming diblock copolymer composition is PEO(2k)-b-PCL(12k). In other preferred embodiments, the PEO block size in the polymersome-forming copolymers is approximately 1-4kDa and the PEO block fractions are approximately 10% to approximately 20% of the total copolymer by weight. In some preferred embodiments, utilizing a diblock copolymer consisting essentially of PEO(2k)-b-PCL(12k) circumvents formulation challenges observed with previous attempts at developing an appropriate cellular based oxygen carriers. Additionally, the various embodiments provide for the utilization of PEO-b-PMCL and PEO-b-PTMC diblock copolymers to successfully generate PEM dispersions that are not only biodegradable but also deformable.

Biodegradable PEM dispersions comprised of PEO-b-PCL, PEO-b-PMCL, and PEO-b-PTMC copolymers are ideal cellular-based oxygen carriers and could help in cancer treatment and enhancement of cancer radiation therapy. The various embodiments provide for the combination of copolymers with different oxygen binding substances (including Mb and other oxygen-binding agents with similar properties derived from various sources), concentrations, and co-encapsulated reductant molecules to create an array of biodegradable and deformable cellular oxygen carriers for animal and human applications. In various preferred embodiments, derivatives of PEO-b-PCL, PEO-b-PMCL, PEO-b-PTMC diblock copolymers are blended and/or chemically modified in order to generate biodegradable PEM dispersions that enable a novel method for MBOC preparation and delivery; this method includes the following steps: 1) self-assembly of the MBOC in aqueous solution, 2) stabilization of the MBOC via chemical modification, 3) lypholization of the resultant construct, 4) dry-phase storage, 5) point-of-care solution rehydration, and 6) *in vivo* delivery of biodegradable MBOCs that retain their original Mb. In some preferred embodiments, *in vivo* delivery is achieved by intravenous, inhalational, transmucosal (e.g. buccal) or transcutaneous routes of administration.

Various embodiments provide MBOCs where the oxygen carriers is comprised of either polymeric micelles, polymersomes, or other nanoparticle based vehicles that incorporate oxygen binding proteins that unload oxygen at low tissue pO2. The various embodiments encapsulate Mb.

In various embodiments, the high-affinity oxygen-binding agents is unmodified human myoglobin. In various embodiments, the high-affinity oxygen-binding agents may be unmodified myoglobin from another biological species. In various embodiments, the high-affinity oxygen-binding agents may be chemically or genetically modified myoglobin from humans or from another biological species.

In an embodiment, the high-oxygen affinity agent includes a PEGylated or polymerized version of the high-affinity oxygen-binding agent. The affinity agent comprises myoglobin and releases oxygen at oxygen tensions less than 10 mmHg. In an embodiment, the high-oxygen affinity agent is natural myoglobin. In an embodiment, the high-oxygen affinity agent is selected from one of unmodified human myoglobin, unmodified myoglobin from another biological species, chemically or genetically modified myoglobin from humans or from another biological species. In an embodiment, the high-oxygen affinity agent includes a carrier vehicle that encapsulates the high-affinity oxygen agent and protects the high-affinity oxygen agent from being released into the blood stream. In an embodiment, the carrier vehicle is a nanoparticle-based vehicle (e.g., drug delivery vehicle). In an embodiment, the carrier vehicle is a vesicle. In an embodiment, the vesicle is a lipid vesicle and the high-oxygen affinity agent is within the lipid vesicle. In an embodiment, the vesicle is a polymer vesicle and the high-oxygen affinity agent is within the polymer vesicle. In an embodiment, the carrier vehicle is a Polymersome. In an embodiment, the carrier vehicle includes a plurality of biodegradable polymers. In an embodiment, the plurality of biodegradable polymers form a nanoparticle. In an embodiment, the nanoparticle is less than 200 nanometers in diameter. In an embodiment, the nanoparticle is less than 100 nanometers in diameter. In an embodiment, the carrier vehicle includes a plurality of biodegradable polymers that form a solid nanoparticle, a micelle or a shell nanoparticle.

The high-oxygen affinity agent is suitable for binding oxygen tightly at physiological oxygen binding tensions found in lungs and releasing the oxygen at oxygen tensions less than 10 mmHg. In an embodiment, the high-oxygen affinity agent is selected from one or more of unmodified human myoglobin, unmodified myoglobin from another biological species, chemically or genetically modified myoglobin from humans or from another biological species. The high-oxygen affinity agent is able to bind oxygen tightly while circulating in a bloodstream and to release oxygen in a linear or absolute fashion at oxygen tensions less than 10 mmHg. In an embodiment, the high-oxygen affinity agent is natural human myoglobin. In an embodiment, the high-oxygen affinity agent is myoglobin derived from another animal species. In an embodiment, the high-oxygen affinity agents is a chemically, biologically, or genetically modified myoglobin from another animal species. In an embodiment, high-oxygen affinity agent comprises a PEGylated or polymerized version of the high-oxygen affinity agent. In an embodiment, a carrier vehicle encapsulates the high-oxygen affinity agent and protects the high-oxygen affinity agent from being released into a bloodstream. In an embodiment, the carrier vehicle is a nanoparticle-based vehicle. In an embodiment, the carrier vehicle is a vesicle. In an embodiment, the vesicle is a lipid vesicle and the high-oxygen affinity agent is within an aqueous core of the lipid vesicle. In an embodiment, the vesicle is a lipid vesicle and the high-oxygen affinity agent is within a membranous portion of the lipid vesicle. In an embodiment, the vesicle is a lipid vesicle and the high-oxygen affinity agent is attached to an outside surface of the lipid vesicle. In an embodiment, the vesicle comprises synthetic polymers and the high-oxygen affinity agent is within an aqueous core of the polymer vesicle. In an embodiment, the vesicle comprises synthetic polymers and the high-oxygen affinity agent is within a membranous portion of the polymer vesicle. In an embodiment, the vesicle comprises synthetic polymers and the high-oxygen affinity agent is attached to the outside surface of the polymer vesicle. In an embodiment, the carrier vehicle is a uni- or multi-lamellar polymersome. In an embodiment, the carrier vehicle comprises a plurality of biodegradable polymers. In an embodiment, the plurality of biodegradable polymers form a nanoparticle. In an embodiment, the nanoparticle is less than 200 nanometers in diameter. In an embodiment, the nanoparticle is less than 100 nanometers in diameter. In an embodiment, the carrier vehicle comprises a plurality of biodegradable polymers that form a solid nanoparticle, a micelle, a vesicle, or a shell nanoparticle. In an embodiment, the high-oxygen affinity agent comprises a carrier vehicle that co-encapsulates the high-oxygen affinity agent with at least one other radiation-sensitizing or chemotherapeutic agent.

Further embodiments include a composition having a high-oxygen affinity agent PEGylated or polymerized to reduce toxicity. The high-oxygen affinity agent binds oxygen tightly at physiological oxygen binding tensions found in lungs and releases its bound oxygen at tissue oxygen tensions that are less than 10 mmHg. The affinity agent binds oxygen tightly while circulating in the bloodstream and only releases oxygen in a linear or absolute fashion at tissue oxygen tensions that are less than 10 mmHg. The high-oxygen affinity agent is selected from one of unmodified human myoglobin, unmodified myoglobin from another biological species, chemically or genetically modified myoglobin from humans or from another biological species.

Further embodiments include a composition consisting of a carrier vehicle and one or more high-oxygen affinity agent(s) that binds oxygen tightly and releases the oxygen at tissue oxygen tensions less than 10 mmHg. In an embodiment, the high-oxygen affinity agent is coupled to the carrier vehicle such that the carrier vehicle reduces toxicity of the high-affinity oxygen agent when the composition is within an animal or human subject. In an embodiment, the high-oxygen affinity agent is selected from one of unmodified human myoglobin, unmodified myoglobin from another biological species, chemically or genetically modified myoglobin from humans or from another biological species. In an embodiment, the high-oxygen affinity agent releases oxygen at oxygen tensions below 5 mmHg. In an embodiment, the high-oxygen affinity agent is natural myoglobin. In an embodiment, the high-oxygen affinity agent is selected from one of unmodified human myoglobin; unmodified myoglobin from another biological species; chemically or genetically modified myoglobin from humans or from another biological species. In an embodiment, the carrier vehicle is a Polymersome. In an embodiment, the carrier vehicle is a vesicle consisting of a bilayer or multi-layer membrane. In an embodiment, the vesicle is a lipid vesicle and the high-oxygen affinity agent is within the aqueous core of the lipid vesicle. In an embodiment, the vesicle is a polymer vesicle and the high-oxygen affinity agent is within the polymer vesicle. In an embodiment, the carrier vehicle is a vesicle composed of a bi- or multi-layer membrane comprised of a single homopolymer, one or more blocks of copolymers, or random arrangement of one or more copolymers, and the high-oxygen affinity agent is within the aqueous core of the nanoparticle-based vesicle. In an embodiment, the carrier vehicle is a vesicle composed of a bi- or multi-layer membrane comprised of a single homopolymer, one or more blocks of copolymers, or random copolymers, and the high-oxygen affinity agent is within the membraneous region of the nanoparticle-based vesicle. In an embodiment, the carrier vehicle includes a plurality of biodegradable polymers. In an embodiment, the plurality of biodegradable polymers form a nanoparticle. In an embodiment, the nanoparticle is less than 200 nanometers in diameter. In an embodiment, the nanoparticle is less than 100 nanometers in diameter. In an embodiment, the carrier vehicle includes a plurality of biodegradable polymers that form a solid nanoparticle or form a shell nanoparticle. In an embodiment, the composition includes PEGylated myoglobin. The high-oxygen affinity agent binds oxygen tightly at physiological oxygen binding tensions found in lungs and releases the oxygen at oxygen tensions less than 10 mmHg. The high-oxygen affinity agent is selected from one or more of unmodified human myoglobin, unmodified myoglobin from another biological species, chemically or genetically modified myoglobin from humans or from another biological species. The high-oxygen affinity agent binds oxygen tightly while circulating in a bloodstream and only releases oxygen in a linear or absolute fashion at oxygen tensions less than 10 mmHg. In an embodiment, the high-oxygen affinity agent is natural myoglobin. In an embodiment, the high-oxygen affinity agent releases oxygen at oxygen tensions below 5 mmHg. In an embodiment, the high-oxygen affinity agent is selected from one or more of unmodified human myoglobin, unmodified myoglobin from another biological species. In an embodiment, the carrier vehicle is a nanoparticle-based vehicle. In an embodiment, the carrier vehicle is a vesicle. In an embodiment, the vesicle is a lipid vesicle and the high-oxygen affinity agent is within an aqueous core of the lipid vesicle. In an embodiment, the vesicle is a lipid vesicle and the high-oxygen affinity agent is within a membranous portion of the lipid vesicle. In an embodiment, the vesicle is a lipid vesicle and the high-oxygen affinity agent is attached to the surface of the lipid vesicle. In an embodiment, the vesicle comprises synthetic polymers and the high-oxygen affinity agent is within an aqueous core of the polymer vesicle. In an embodiment, the vesicle comprises synthetic polymers and the high-oxygen affinity agent is within a membranous portion of the polymer vesicle. In an embodiment, the vesicle comprises synthetic polymers and the high-oxygen affinity agent is attached to the outside surface of the polymer vesicle. In an embodiment, the carrier vehicle is a uni- or multi-lamellar polymersome. In an embodiment, the carrier vehicle comprises a plurality of biodegradable polymers. In an embodiment, the plurality of biodegradable polymers form a nanoparticle. In an embodiment, the nanoparticle is less than 200 nanometers in diameter. In an embodiment, the nanoparticle is less than 100 nanometers in diameter. In an embodiment, the carrier vehicle comprises a plurality of biodegradable polymers that form a solid nanoparticle or form a shell nanoparticle. In an embodiment, the carrier vehicle co-encapsulates the high-oxygen affinity agent with at least one other radiation-sensitizing or chemotherapeutic agent. In an embodiment, comprising PEGylated myoglobin.

Further embodiments include a composition having an oxygen carrier that includes a plurality of nanoparticle-based vehicles and a high-oxygen affinity agent encapsulated within the plurality of nanoparticle-based vehicles. In an embodiment, the plurality of nanoparticle-based vehicles may consist of one or more vesicles, micelles, or solid nanoparticles, wherein the vesicles, micelles, or solid nanoparticles include at least one of a lipid, a biodegradable polymer, a polysaccharide or a protein. In an embodiment, the plurality of nanoparticle-based vehicles are multimeric vesicles. In an embodiment, the vesicles are polymersomes. In an embodiment, the plurality of nanoparticle-based vehicles include compositions that allow for accumulation at a target site of interest. In an embodiment, the nanoparticle vehicles include compositions that allow for their accumulation at sites of interest via passive diffusion or via a targeting modality comprised of a conjugation of a targeting molecule of separate chemical composition from that of the nanoparticles. In an embodiment, the targeting molecule consists of a compound selected from one or more of a naturally occurring protein, a recombinant protein, a recombinant polypeptide, a synthetic polypeptide, a chemical synthesized by an animal, a synthetic small molecule, a metal-chelator complex, a carbohydrate, a nucleic acid, a lipid and/or a polymer. In an embodiment, the nanoparticle vehicles include a targeting molecule composition in which use of an external energy source such as heat, X-ray, and magnetic resonance can be used to localize the nanoparticle-based vehicles to sites of interest within the subject. The high-oxygen affinity agent binds oxygen tightly at physiological oxygen binding tensions found in lungs and releases the oxygen at oxygen tensions less than 10 mmHg. In an embodiment, the high-oxygen affinity agent is natural myoglobin. In an embodiment, at least some of the plurality of polymer vesicles are biodegradable polymer vesicles and at least some of the plurality of polymer vesicles are biocompatible polymer vesicles. In an embodiment, the biocompatible polymer vesicles are comprised of copolymers that include poly(ethylene oxide) or poly(ethylene glycol). In an embodiment, the biodegradable polymer vesicles are comprised of copolymers that include poly(ε-caprolactone). In an embodiment, the biodegradable polymer vesicles are comprised of copolymers that include poly(y-methyl ε-caprolactone). In an embodiment, the biodegradable polymer vesicles are comprised of copolymers that include poly(trimethyl carbonate). In an embodiment, the oxygen carrier is further comprised of copolymers that include one of a poly(peptide), a poly(saccharide) or a poly(nucleic acid). In an embodiment, the biodegradable polymer vesicles are comprised of block copolymers of poly(ethylene oxide) and poly(ε-caprolactone). In an embodiment, the biodegradable polymer vesicles are comprised of block copolymers of poly(ethylene oxide) and poly(y-methyl ε-caprolactone). In an embodiment, the biodegradable polymer vesicles are comprised of block copolymesr of poly(ethylene oxide) and poly(trimethyl carbonate). In an embodiment, the biodegradable polymer vesicles are either pure or blends of multiblock copolymer, wherein the copolymer includes at least one of poly(ethylene oxide) (PEO), poly(lactide) (PLA), poly(glycolide) (PLGA), poly(lactic-co-glycolic acid) (PLGA), poly(ε-caprolactone) (PCL), and poly (trimethylene carbonate) (PTMC), poly(lactic acid) (PLA), and/or poly(methyl ε-caprolactone) (PMCL).

Further embodiments include methods of manufacturing a composition by preparing an organic solution comprising a plurality of polymers and exposing the organic solution to a plastic, polytetrafluoroethylene, or glass surface, dehydrating the organic solution on the plastic, polytetrafluoroethylene, or glass surface to create a single or multilayer film of polymers, rehydrating the film of polymers in an aqueous solution comprising myoglobin as oxygen-binding molecule, and cross-linking the polymers in the aqueous solution via chemical modification. Further embodiments include methods of manufacturing a composition by preparing an organic solution comprising a plurality of polymers and myoglobin as oxygen-binding molecule, then exposing the organic solution to a plastic, polytetrafluoroethylene, or glass surface, dehydrating the organic solution on the plastic, polytetrafluoroethylene, or glass surface to create a single or multilayer film of polymers, rehydrating the film of polymers in an aqueous solution, and cross-linking the polymers in the aqueous solution via chemical modification.

Further embodiments include a kit that includes a pharmaceutical composition having an oxygen carrier, wherein the oxygen carrier includes a plurality of polymers and myoglobin as high-oxygen affinity agent, and an implement suitable for administering the oxygen carrier intravenously, via inhalation, topically, per rectum, per the vagina, transdermally, subcutaneously, intraperitoneally, intrathecally, intramuscularly, or orally. The pharmaceutical composition may include pharmaceutically active agent in an effective amount to treat or prevent a disease or disorder in a subject, or to improve the efficacy of radiation therapy. The pharmaceutical composition may include any of the above-mentioned compositions, agents, and/or vehicles for increasing oxygen levels in an effective amount for a subject in need thereof. The pharmaceutical composition may include any of the above-mentioned compositions, agents, and/or vehicles in effective amounts for treatment or prevention of malignant cancer or tumor growth in an effective amount for a subject in need thereof. The pharmaceutical composition may include any of the above-mentioned compositions, agents, and/or vehicles in effective amounts for treating to preventing benign or malignant tumor growth in a subject in need thereof.

Further embodiments include a kit that includes a first container and a second container, the first container having a high-oxygen affinity agent comprising myoglobin, and the second container including a rehydration mixture.

Further embodiments include a high-oxygen affinity agent comprising myoglobin, for use in treating a tumor within a patient, the high-oxygen affinity agent being configured to have low toxicity and to accumulate within the tumor, and administering ionizing radiation to the tumor. The high-oxygen affinity agent may administered intravenously, via inhalation, topically, per rectum, per the vagina, transdermally, subcutaneously, intraperitoneally, intrathecally, intramuscularly, or orally.

In various embodiments, the inert carrier may be any one or more of a liposome, polymersome, micelle, modified lipoprotein, solid nanoparticle, solid micron-sized particle, lipid or perflurocarbon emulsion, dendrimer, virus, or virus-like particle. In various embodiments, the inert carrier may be a PEGylated or polymerized version of myoglobin.

In various embodiments, human myoglobin may be encapsulated within nanoparticles, polymer vesicles and/or polymersomes. In various embodiments, the nanoparticles, polymer vesicles and/or polymersomes may be constructed from one of a number of different materials.

In some embodiments, the invention relates to kits comprising a composition, pharmaceutical composition or polymersome disclosed herein. Various embodiments provide a kit that includes a pharmaceutical composition comprising myoglobin. The composition may include a delivery vehicle and myoglobin. The kit may include an implement suitable for administering the high-oxygen affinity agent comprising myoglobin, intravenously, via inhalation, topically, per rectum, per the vagina, transdermally, subcutaneously, intraperitoneally, intrathecally, intramuscularly, or orally.

### BRIEF DESCRIPTION OF THE DRAWINGS

The accompanying drawings relating to oxygen affinity agents other than myoglobin do not belong to the claimed invention.
FIG. 1 is a graph illustrating the oxygen dissociation curve of hemoglobin.
FIG. 2 is a graph illustrating the oxygen dissociation curves of hemoglobin and example agents that may be used to manipulate oxygen levels in tissues in accordance with various embodiments.
FIG. 3 is a graph illustrating the oxygen dissociation curves of hemoglobin and myoglobin.
FIG. 4A is an illustration of biodegradable polymers that may be a component of a biodegradable cellular oxygen carrier in various embodiments.
FIG. 4B is an illustration of water-soluble near-infrared fluorophores "◇" and water-soluble oxygen-binding proteins "○" that may be used as components of a biodegradable cellular oxygen carrier in various embodiments.
FIGs. 4C-D are illustrations of the synthesis of nanoscale polymer encapsulated myoglobins and processing procedures (heat, sonication, and extrusion to yield) of nanoscale polymer encapsulated myoglobins in accordance with various embodiments.
FIG. 4E is an illustration of an encapsulation schematic of an embodiment polymersome.
FIG. 4F is a cryogenic transmission electron micrograph and a confocal micrograph of polymer encapsulated myoglobins.
FIG. 5 are photographs illustrating the (A) bright field, (B) oxygen tension in % oxygen, and (C) functional blood vasculature for a window chamber tumor.
Figure 6A is a cryogenic transmission electron micrograph of PEO(2K)-*b*-PCL(12K)-based polymersomes in de-ionized water (5 mg/ml) that illustrates the membrane core thickness of the vesicles as being 22.5 ± 2.3 nanometer.
Figure 6B a graph illustrating the cumulative *in situ* release of doxorubicin, loaded within 200 nm diameter PEO(2K)-b-PCL(12K) based polymersomes, under various physiological conditions (pH 5.5 and 7.4; T= 37 °C) as measured fluorometrically over 14 days.
FIG. 7A is an *in vivo* optical image of encapsulated oligo(porphyrin)-based near-infrared (NIR) fluorophores (NIRFs) that illustrates the accumulation of an embodiment carrier in tumors.
FIG. 7B is a graph of *in vivo* tumor growth as inhibited by phosphate buffered saline (PBS), doxorubicin (Dox), liposome, and Polymersome.
FIG. 8A is a bar chart illustrating the agent encapsulation efficiencies of four polymersome-encapsulated agent formulations extruded through 200 nm diameter polycarbonate membranes.
FIG. 8B is a bar chart illustrating the P₅₀ (mmHg) of red blood cells, hemoglobin and four polymersome-encapsulated hemoglobin formulations extruded through 200 nm polycarbonate membranes.
Figure 9 is a process flow diagram illustrating an embodiment method for the preparation and delivery of a hemoglobin-based oxygen carrier.
FIG. 10 is a process flow diagram illustrating an embodiment method for preparing a polymersome comprising at least one biocompatible polymer and at least one biodegradable polymer.

### DETAILED DESCRIPTION OF THE INVENTION

The present invention may be understood more readily by reference to the following detailed description taken in connection with the accompanying figures and examples, which form a part of this disclosure.

The terms "subject" and "patient" are used interchangeably herein to refer to human patients, whereas the term "subject" may also refer to any animal. It should be understood that in various embodiments, the subject may be a mammal, a non-human animal, a canine and/or a vertebrate.

The term "monomeric units" is used herein to mean a unit of polymer molecule containing the same or similar number of atoms as one of the monomers. Monomeric units, as used in this specification, may be of a single type (homogeneous) or a variety of types (heterogeneous).

The term "polymers" is used according to its ordinary meaning of macromolecules comprising connected monomeric molecules.

The term "amphiphilic substance" is used herein to mean a substance containing both polar (water-soluble) and hydrophobic (water-insoluble) groups.

The term "*in vivo* delivery" is used herein to refer to delivery of a biologic by routes of administration such as topical, transdermal, suppository (rectal, vaginal), pessary (vaginal), intravenous, oral, subcutaneous, intraperitoneal, intrathecal, intramuscular, intracranial, inhalational, oral, and the like.

The term "an effective amount" is used herein to refer to an amount of a compound, material, or composition effective to achieve a particular biological result such as, but not limited to, biological results disclosed, described, or exemplified herein. Such results may include, but are not limited to, the effective reduction of symptoms associated with any of the disease states mentioned herein, as determined by any means suitable in the art.

The term "membrane" is used herein to mean a spatially distinct collection of molecules that defines a two-dimensional surface in three-dimensional space, and thus separates one space from another in at least a local sense.

The term "pharmaceutically active agent" is used herein to refer to any a protein, peptide, sugar, saccharide, nucleoside, inorganic compound, lipid, nucleic acid, small synthetic chemical compound, or organic compound that appreciably alters or affects the biological system to which it is introduced.

The term "drug delivery" is used herein to refer to a method or process of administering a pharmaceutical compound to achieve a therapeutic effect in humans or animals.

The term, "vehicle" is used herein to refer to agents with no inherent therapeutic benefit but when combined with an pharmaceutically active agent for the purposes of drug delivery result in modification of the pharmaceutical active agent's properties, including but not limited to its mechanism or mode of in vivo delivery, its concentration, bioavailability, absorption, distribution and elimination for the benefit of improving product efficacy and safety, as well as patient convenience and compliance.

The term "carrier" is used herein to describe a delivery vehicle that is used to incorporate a pharmaceutically active agent for the purposes of drug delivery.

The term "oxygen-binding agent" or "oxygen-binding compound" is used herein to refer to myoglobin.

The term "allosteric effector" is used herein to refer to a molecule that modulates the rate or amount of oxygen binding to or releasing from of an oxygen carrier.

The term "high-oxygen affinity" agent or "high oxygen affinity compound" is used herein to refer to myoglobin.

The term "oxygen-binding carrier" or "oxygen carrier" is used herein to refer to a carrier comprised of a synthetic or partially synthetic vehicle that incorporates a single or plurality of oxygen-binding agents.

The term "homopolymer" is used herein to refer to a polymer derived from one monomeric species of polymer.

The term "copolymer" is used herein to refer to a polymer derived from two (or more) monomeric species of polymer, as opposed to a homopolymer where only one monomer is used. Since a copolymer consists of at least two types of constituent units (also structural units), copolymers may be classified based on how these units are arranged along the chain.

The term "block copolymers" is used herein to refer to a copolymer that includes two or more homopolymer subunits linked by covalent bonds in which the union of the homopolymer subunits may require an intermediate non-repeating subunit, known as a junction block. Block copolymers with two or three distinct blocks are referred to herein as "diblock copolymers" and "triblock copolymers," respectively.

The term "areal strain" is used herein to refer to the change in the surface area of a particle under an external force or tension divided by the original surface area of the particle prior to application of said external force or tension (denoted by "A" and expressed as %).

The term "critical lysis tension" or "Tc" is used herein to refer to the tension at which a particle ruptures when subject to an external force as measured by micropipette aspiration and expressed as milliNewtons/meter (mN/m).

The term "critical areal strain" or "Ac" is used herein to refer to the areal strain realized by the oxygen carrier or polymersome at the critical lysis tension.

The term "loading ratio" is used herein to refer to a measurement of a oxygen biding carrier and may be defined as the weight of oxygen binding agent within the oxygen carrier divided by the dry weight of the inert vehicle.

The term "myoglobin loading capacity" is used herein to refer to a measurement of a myglobin-based oxygen carrier and may be defined as the weight of myoglobin within the oxygen carrier divided by the total weight of carrier.. The term "myoglobin loading efficiency" is used herein to refer to a measurement of a myoglobin-based oxygen carrier and may be defined as the weight of myoglobin that is encapsulated and/or incorporated within a carrier suspension divided by the weight of the original myoglobin in solution prior to encapsulation (expressed as a %).

The term a "unit dose" is used herein to refer to a discrete amount of the pharmaceutical composition comprising a predetermined amount of the active ingredient.

It should be understood that P50 is the partial pressure of oxygen (pO2) at which the oxygen-binding compound becomes 50% saturated with oxygen. As the P50 decreases, oxygen affinity increases, and visa verse. Normal adult Hemoglobin A has a P50 of 26.5 mm Hg while Fetal Hemoglobin F has a P50 of 20 mm Hg and sickle cell anemia Hemoglobin S has a P50 of 34 mm Hg.

The various embodiments provide a nanoparticle-based therapeutic carrier suitable to deliver myoglobin as high-oxygen affinity agent to tumors in order to increase intratumoral pO2, to stunt their aggressive molecular phenotypes, and to increase the efficacy of radiation and chemo-therapies directed against the tumor.

Generally, radiation treatment may be augmented by increasing the oxygen levels of tumors, in order to generate more oxygen-based free radicals with concomitant radiation therapy, or by delivering another non-O₂ dependent radiation sensitizer to tumor-specific sites. Conventional methods for manipulating the oxygen levels of tumors are reliant upon increasing the systemic level of oxygen in order to eventually deliver this increased oxygen capacity to the tumor. One such method that delivers artificial blood substitutes using natural hemoglobin (Hb) is disclosed in U.S. Patent Application No. 13/090,076 entitled "Biodegradable Nanoparticles as Novel Hemoglobin-Based Oxygen Carriers and Methods of Using the Same" filed on April 19, 2011.

Hemoglobin is an oxygen-transporting protein in human red blood cells. Hemoglobin's structure makes it efficient at binding to oxygen, and efficient at unloading the bound oxygen in human tissues/blood stream. Hemoglobin consists of two pairs of globin dimers held together by non-covalent bonds to form a larger four subunit (tetrameric) hemoglobin molecule. The oxygen binding capacity of tetrameric hemoglobin depends on the presence of a non-protein unit called the heme group (i.e., one molecule of hemoglobin can bind with four oxygen molecules).

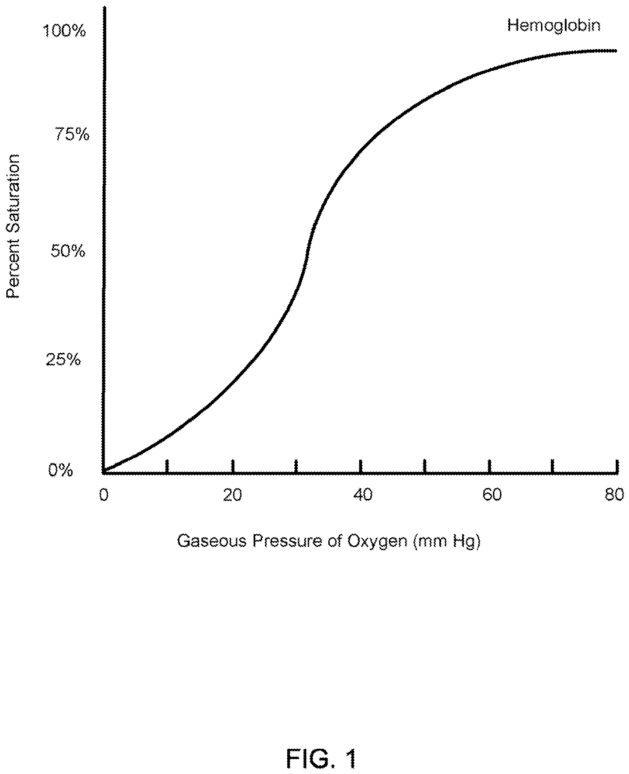

FIG. 1 illustrates the oxygen dissociation curve of hemoglobin. Cooperative binding of oxygen to hemoglobin gives native hemoglobin a sigmoidal-shaped oxygen dissociation curve and allows oxygen to be bound and released within a narrow physiological range of pO2s (from 40-100 mmHg). Conventional methods for manipulating the oxygen levels of tumors have attempted to use hemoglobin or agents (proteins, molecules, etc.) having a similar oxygen dissociation curve as native hemoglobin. Others have attempted to use agents having oxygen dissociation curves that are shifted to the right of the hemoglobin oxygen dissociation curve (i.e., agents having a lower affinity for oxygen than hemoglobin).

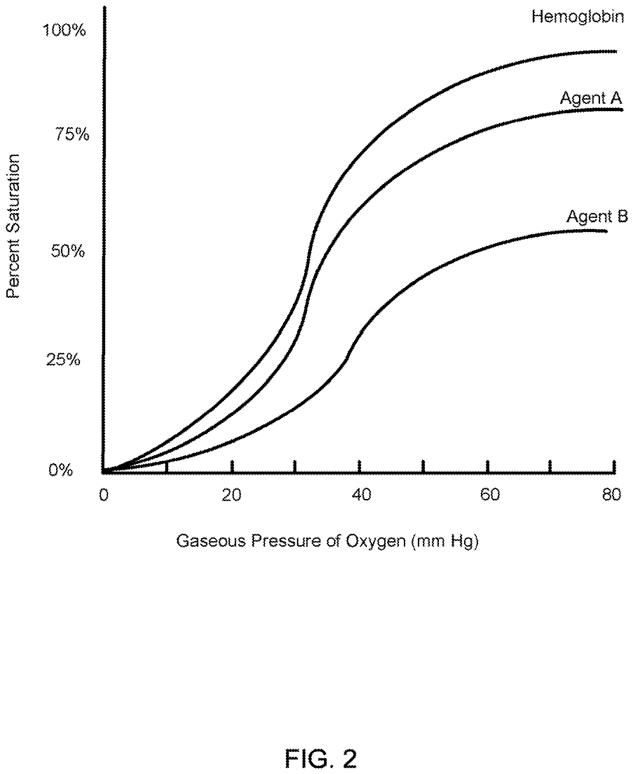

FIG. 2 illustrates the oxygen dissociation curves of hemoglobin and two other example agents (e.g., Agent A, Agent B) which may be used to manipulate oxygen levels in tumors. Specifically, FIG. 2 illustrates that the example agents (Agent A, Agent B) have oxygen dissociation curves that are shifted to the right of the hemoglobin oxygen dissociation curve, which increases the amount of oxygen delivered by the example agents.

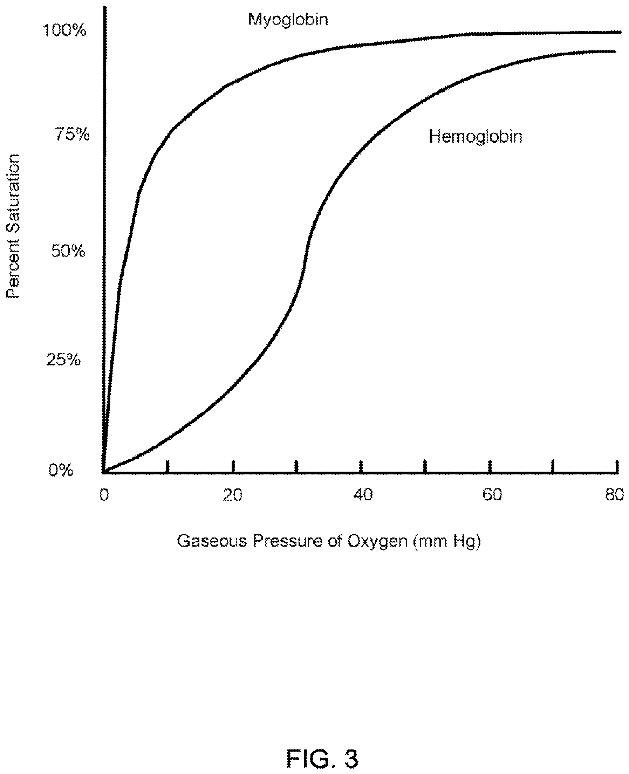

FIG. 3 illustrates the oxygen dissociation curves of hemoglobin and myoglobin, a ubiquitous protein involved in regulating oxygen levels in muscle tissues. FIG. 3 shows that the oxygen dissociation curve of myoglobin is a rectangular hyperbola with a very low P50 that lies to the left of the sigmoid-shaped hemoglobin oxygen dissociation curve (i.e., myoglobin has a much higher affinity for oxygen than hemoglobin). That is, in contrast to the example agents (e.g., Agent A, Agent B) discussed above with reference to FIG. 2, myoglobin has an oxygen dissociation curve that is shifted to the left of the hemoglobin oxygen dissociation curve. This is due in part to the fact that, unlike hemoglobin (which has affinity for oxygen of about 20 to 50 mmHg), myoglobin binds oxygen very tightly and only releases it at a very low oxygen tension (2 to 3 mmHg). The various embodiments benefit from the fact that a similar level of oxygen tension (2 to 5 mmHg) exists in the center of solid tumors, and this same level (2 to 5 mmHg) has been shown to be the level at which the efficacy of radiation therapy falls below fifty percent of its maximal value.

The various embodiments provide compositions comprising myoglobin as high-oxygen affinity agent, and being suitable for delivery to solid tumors to improve the efficacy of radiation therapy. Since the oxygen tension at the center of a solid tumor is similar to the oxygen tension (2 to 3 mmHg) at which oxygen releases from myoglobin, the use of high-oxygen affinity agents ensures that the oxygen is not released from the agents until they are positioned around or within the tumor.

As mentioned above, the oxygen tensions at the center of solid tumors are similar to the oxygen tensions (2 to 3 mmHg) at which oxygen releases from myoglobin. As also mentioned above, the efficacy of radiation treatment may be improved by increasing the oxygen levels of tumors, and conventional methods for manipulating the oxygen levels are reliant upon increasing the systemic level of oxygen. For example, existing techniques for delivering oxygen to tumors may involve increasing the amount of blood flow to the tumor, increasing the amount of dissolved oxygen in blood, or increasing the overall blood concentration of hemoglobin. Conventional methods achieve this by using agents having a similar affinity for oxygen as natural red blood cells (e.g., derivatives of human or xenotic hemoglobin and/or other agents having the same or less affinity for oxygen as natural human hemoglobin) in order to increase the oxygen carrying capacity of the blood in hopes that this may translate to increased tumor oxygen delivery. In contrast to these conventional treatment methods, the various embodiments describe compositions of matter and methodology to deliver oxygen to tumor tissues by utilizing myoglobin as an agent that has much higher affinity for oxygen than that of natural human hemoglobin. Since the partial pressure of oxygen at the center of a solid tumor is similar to the oxygen tension at which oxygen releases from myoglobin (2 to 3 mmHg), oxygen is not released until the high-oxygen affinity agents are around or within the tumor.

While delivering high-oxygen affinity agents (e.g., myoglobin, other synthetic proteins having similar oxygen affinity as myoglobin, etc.) to solid tumors may improve the efficacy of radiation therapy, in order to achieve proper localization to the tumor, a large amount of the high-oxygen affinity agents must be injected into the blood stream. Injecting a large amount of such proteins into the bloodstream is dangerous, as the injected agent (e.g., myoglobin) may be nephrotoxic and/or cause hypertensive urgency or emergency (via sequestration of the vasodilator nitric oxide that normally controls blood vessel tone). For example, in the case of myoglobin, such phenomena is commonly observed in people who have heart attacks, run marathons, engage in other strenuous exercises, and/or use various drugs such as cocaine. In such people, muscles may begin to break down very quickly, thereby releasing a large amount of myoglobin into the blood stream. This extra myoglobin may result in the protein getting trapped in the body's filter apparatus (i.e., kidney glomeruli) and/or cause a life threatening condition known as rhabdomyolysis. A large amount of myoglobin in the blood stream may also increase blood pressure and lead to organ damage. This is because, in the bloodstream, myoglobin and other free oxygen-binding proteins sequester nitric oxide (NO) that is a mediator of vascular tone and blood flow. Thus, when myoglobin floods in the bloodstream, it acts to extract nitric oxide from the blood vessel walls, causing the blood vessels to constrict. This may cause an increase in the overall blood pressure and possibly lead to a hypertensive crisis, damaging major organs such as the kidneys, the heart, and the brain. For these and other reasons, injecting an oxygen binding protein, such as myoglobin, directly into the blood stream in its free-form is dangerous.

To address these and other issues, various embodiments may encapsulate myoglobin as high-affinity oxygen binding agent in a carrier vehicle (e.g., a nanoparticle shell) that will protect the encapsulated agents from being released into the blood stream or interacting with biological components (e.g. proteins, cells, and the blood vessel walls) while in the blood circulation. It should be noted that the various embodiments are not necessarily limited to nanoparticle encapsulation or to any particular carrier vehicle unless expressly recited as such in the claims. In some embodiments, the inert carrier may be a PEGylated or polymerized version of myoglobin.

In various embodiments, myoglobin may be encapsulated in carrier vehicles having characteristics that allow for its accumulation around tumor regions and/or are capable of targeting tumors experiencing low oxygen tension. The carrier vehicle may also have characteristics such that oxygen will diffuse from within the vehicle to regions of low oxygen tension (as exist in the center of tumors) while myoglobin remains encapsulated. The high-oxygen affinity agent myoglobin is suitable for releasing oxygen at the oxygen tension required to increase the efficacy of radiation due to the oxygen partial pressure gradient characteristics of myoglobin.

In various embodiments, the high-oxygen affinity agent myoglobin may be encapsulated in a vehicle comprised of biodegradable polymers (e.g., polymersomes, nanoparticles, etc.). Encapsulation of myoglobin as high oxygen affinity agent in biodegradable polymeric vehicles protects myoglobin from contact with blood and tissues, thereby reducing toxicity while maintaining high internal oxygen concentrations until the vehicles are positioned within hypoxic tumor tissues. In an embodiment, myoglobin may be encapsulated such that it is highly concentrated within the aqueous interior of the carrier vehicle. The agents may be encapsulated such that the carrier vehicle (e.g., nanoparticle shell) shields the encapsulated myoglobin from interacting with the blood vessel walls, preventing nitric oxide from being taken up into the nanoparticle and/or binding to encapsulated myoglobin.

In various embodiments, the high-affinity oxygen-binding agents may be unmodified human myoglobin, unmodified myoglobin from another biological species, chemically or genetically modified myoglobin from humans or from another biological species, that binds oxygen tightly at physiological oxygen binding tensions as found in the lungs and that releases it only at lowest oxygen tensions as found in hypoxic tumors. In various embodiments, the inert carrier vehicle may be any one or more of a liposome, polymersome, micelle, modified lipoprotein, solid nanoparticle, solid micron-sized particle, lipid or perflurocarbon emulsion, dendrimer, virus, or virus-like particle. In other embodiments, the inert carrier vehicle may be a PEGylated or polymerized version of myoglobin as high-affinity oxygen-binding agent. In a preferred embodiment, human myoglobin may be encapsulated within nanoparticles, polymer vesicles and/or polymersomes. In various embodiments, the nanoparticles, polymer vesicles and/or polymersomes may be constructed from one of a number of different bioderadable materials.

The high-affinity oxygen-binding agent comprises myoglobin. Myoglobin (Mb) is a cytoplasmic heme protein that plays a well-characterized role in O₂ transport and free radical scavenging in skeletal and cardiac muscle (two tissues, notably, with low incidences of malignancy).^{93, 94} Myoglobin's oxygen-related functions are multiple and include at least 3 different activities. First, myoglobin acts as an oxygen reservoir, possessing a much higher O₂ affinity than that of hemoglobin (Mb) (P50-Mb = 2.75 vs. P50-Hb = 25-50 mmHg). Myoglobin thus binds O₂ in aerobic conditions and releases it under hypoxic conditions,⁹⁵ as found in tumors. Second, myoglobin is capable of buffering intracellular O₂ by unloading its oxygen as cytoplasmic pO₂ falls to low levels, promoting continuous oxidative phosophorylation.⁹⁶ Third, myoglobin supplements simple O₂ delivery by working as a carrier in a process known as facilitated O₂ diffusion.⁹⁷

Myoglobin has recently been shown to be a modulator of tumor hypoxia.^{98, 99} Myoglobin gene transfer in a mouse xenotransplanted human lung tumor provided a valid model for studying the role of O₂ and ROS in tumor progression. By enabling oxidative phosphorylation under low pO₂, myoglobin further prevents baseline ROS formation under hypoxic conditions and mitigates the tumorigenic response.^{98, 99} In these mouse models of cancer, myoglobin expression resulted in delayed tumor implantation, reduced xenograft growth, and generated minimal HIF-1 levels.⁹⁸ Angiogenesis and invasion were also strongly inhibited.⁹⁸ These effects were not observed using point-mutated forms of myoglobin unable to bind O₂ but capable of scavenging free radicals.⁹⁸ Together, these data suggest that hypoxia is not just an epiphenomenon associated with dysregulated growth, but also a key factor driving tumor progression. They also suggest that the pleiotropic functions of myoglobin affect cancer biology in multiple ways.

While myoglobin has shown to modulate tumor hypoxia, its clinical utility as an O₂ therapeutic requires overcoming two major obstacles related to its free intravascular infusion: 1) vasoconstriction, hypertension, reduced blood flow, and vascular damage in animals due to entrapment of endothelium-derived nitric oxide (NO); and 2) nephrotoxicity as seen with rhabdomyolysis. In the various embodiments, the limitations of using myoglobin as an oxygen carrier may be overcome by encapsulating myoglobin within an appropriate polymeric vehicle (e.g., polymersome) to improve its tumor-specific delivery and to mitigate its systemic exposure.

Polymersomes^{38, 39} are synthetic polymer vesicles that are formed in nanometric dimensions (50 to 300 nm in diameter) and exhibit several favorable properties as cellular oxygen carriers. For example, polymersomes belong to the class of bi- and multi-layered vesicles that can be generated through self-assembly and can encapsulate hydrophilic compounds such as hemoglobin (Hb) and myoglobin (Mb) in their aqueous core.^{40, 41} Moreover, polymersomes offer several options to be designed from fully biodegradable FDA-approved components and exhibit no *in vitro* or acute *in vivo* toxicities.

Polymersomes exhibit several superior properties over liposomes and other nanoparticle-based delivery vehicles that make them effective myoglobin-based oxygen carriers MBOCs. For example, depending on the structure of their component copolymer blocks, polymersome membranes may be significantly thicker (∼ 9-22 nm) than those of liposomes (3-4 nm), making them 5-50 times mechanically tougher and at least 10 times less permeable to water than liposomes.^{46, 47} The circulatory half-life of polymersomes, with poly(ethylene oxide) (PEO) brushes ranging from 1.2-3.7 kDa, is analogous to that of poly (ethylene glycol)-based liposomes (PEG-lyposomes) of similar sizes (-24-48 hours) and can be further specifically tailored by using a variety of copolymers as composite building blocks.⁴⁸ Polymersomes have been shown to be stable for several months *in situ,* and for several days in blood plasma under well-mixed quasi-physiological conditions, without experiencing any changes in vesicle size and morphology.^{40, 48} They do not show in-surface thermal transitions up to 60 °C. ^{37, 48} In addition, early animal studies on PEO-b-PCL and poly(ethylene-oxide)-block-poly(butadiene)- (PEO-b-PBD-)based polymersomes formulations encapsulating doxorubicin have shown no acute or sub-acute toxicities. Finally, the production and storage of polymersomes is economical. Polymersomes may be readily produced and stored on a large-scale without requiring costly post-manufacturing purification processes.

Most promising biodegradable polymersome-encapsulated myoglobin (PEM) formulations have been hypothesized to be comprised of block copolymers that consist of the hydrophilic biocompatible poly(ethylene oxide) (PEO), which is chemically synonymous with PEG, coupled to various hydrophobic aliphatic poly(anhydrides), poly(nucleic acids), poly(esters), poly(ortho esters), poly(peptides), poly(phosphazenes) and poly(saccharides), including but not limited by poly(lactide) (PLA), poly(glycolide) (PLGA), poly(lactic-co-glycolic acid) (PLGA), poly(ε-caprolactone) (PCL), and poly (trimethylene carbonate) (PTMC). Polymersomes comprised of 100% PEGylated surfaces possess improved *in vitro* chemical stability, augmented *in vivo* bioavailablity, and prolonged blood circulatory half-lives.^{42, 43} For example, aliphatic polyesters, constituting the polymersomes' membrane portions, are degraded by hydrolysis of their ester linkages in physiological conditions such as in the human body. Because of their biodegradable nature, aliphatic polyesters have received a great deal of attention for use as implantable biomaterials in drug delivery devices, bioresorbable sutures, adhesion barriers, and as scaffolds for injury repair via tissue engineering.^{44, 45}

Compared to the other biodegradable aliphatic polyesters, poly(ε-caprolactone) (PCL) and its derivatives have several advantageous properties including: 1) high permeability to small drug molecules; 2) maintenance of a neutral pH environment upon degradation; 3) facility in forming blends with other polymers; and 4) suitability for long-term delivery afforded by slow erosion kinetics as compared to PLA, PGA, and PLGA.⁴⁵ Utilization of ε-caprolactone (or derivatives such as γ-methyl ε-caprolactone) as the membrane-forming shells in polymersome-encapsulated myoglobin (PEM) formulations promises that the resultant cellular myoglobin-based oxygen carriers (MBOCs) will have safe and complete *in vivo* degradation.

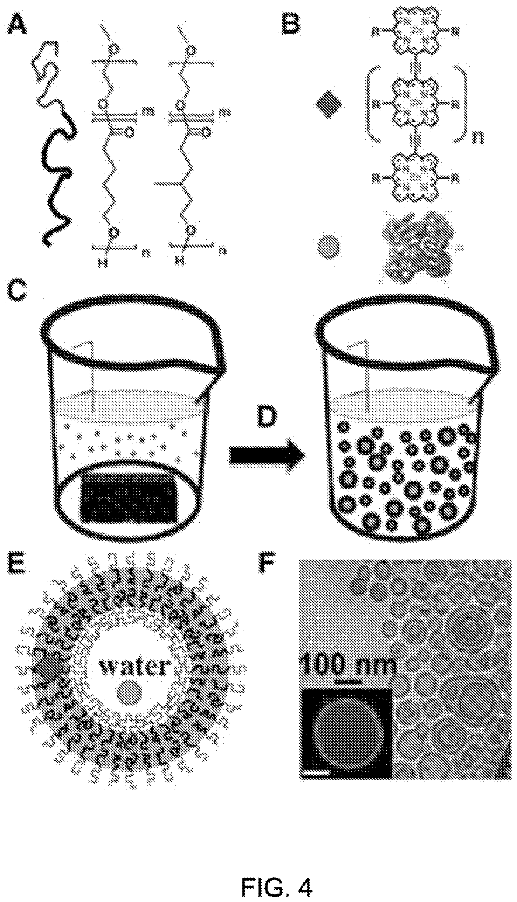

Fully biodegradable and bioresorbable polymersomes have previously been demonstrated to be generated via self-assembly upon aqueous hydration of amphiphilic diblock copolymers of PEO-b-PCL³⁹. Over 20 PEO-b-PCL copolymers, varying in molecular weights of the component building blocks, have previously been tested for the generation of stable bilayered polymersomes. However, as illustrated in FIG. 4(A), only diblock copolymers of PEO-b-PCL in which the PEO block was 1-5 kDa and 10-20% of the polymer mass by weight have demonstrated a consistent and significant yield of stable mono-dispersed polymersomes, with mean particle diameters of < 200 nm and membrane thicknesses of 9-22 nm after extrusion through 200-nm diameter pore cut-off membranes. PEO-b-PCL polymersomes have subsequently been shown to be capable of loading the anti-neoplastic drug doxorubicin (DOX) using an ammonium sulfate gradient. As illustrated in FIG. 4(B), the *in vitro* stability, mechanism of degradation, and rate of drug release from DOX-loaded PEO(2kDa)-b-PCL(12kDa) polymersomes were evaluated as a function of pH over 14 days. While the kinetics of release varied under neutral and acidic pH conditions (5.5 and 7.4, at 37 °C), an initial burst release phase (approx. 20% of the initial payload within the first 8 h) was observed at both pH conditions followed by a more controlled, pH-dependent release over the several days. At a pH of 7.4, kinetic release studies suggest that the encapsulated molecules initially escape the polymersome through passive diffusion of the drug across intact poly(ε-caprolactone) (PCL) membrane (days 1-4), and subsequently through hydrolytic matrix degradation of PCL (days 5-14). At a pH of 5.5, however, it appears that the dominant mechanism of release, at both short and long times, is acid-catalyzed hydrolysis of the PCL membrane. Notably, these fully-biodegradable polymersomes have a half-life (τ_{1/2}) of circulation (24-48 h) that is much shorter than their half-life (τ_{1/2}) of release (2 weeks at pH 7.4).

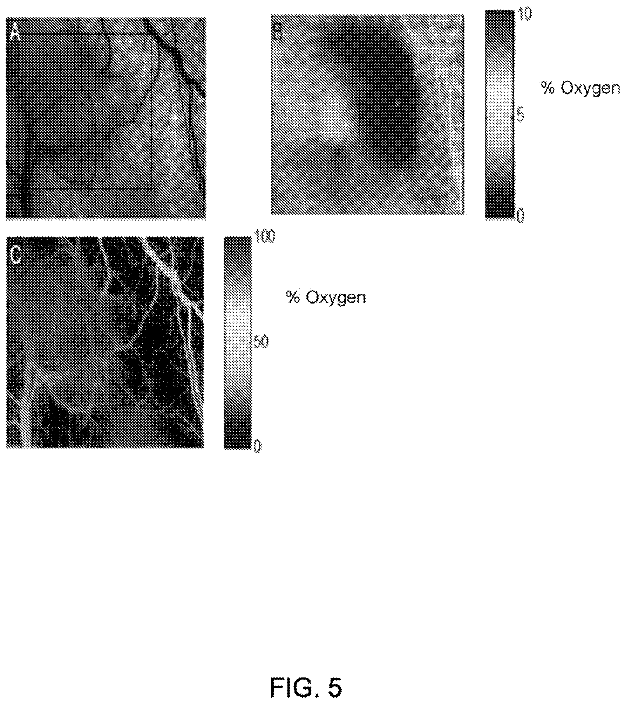

FIG. 5 illustrates the (A) bright field, (B) oxygen tension in % oxygen, and (C) functional blood vasculature for a window chamber tumor. In this illustration the tumor is the relatively dark region in panel A in the center-left. The oxygen saturation (C) is shown on a color scale whose brightness is modulated by the total O2 content (thus well vascularized regions appear bright.) The tumor region displays highly heterogeneous oxygen concentration (B), with a central peak in oxygen tension, as well as a peripheral (upper and right) region that is highly hypoxic. The composite map shows significant contrast with the surrounding normal tissue due to angiogenesis throughout the tumor, making it appear hazy bright. As is evident in the illustrated example of FIG. 5, the O2 content (as measured by the partial pressure of oxygen at various points) is heterogeneous throughout the tumor parenchyma but the lowest oxygen-tensions (darkest areas as demarcated by pO2 of < 10mmHg) can be found within the center of the tumor. It is within these low pO2 laden areas where tumors tend to up-regulate the HIF-1 signaling cascade, leading to a more aggressive tumorigenic phenotype that is resistant to radiation and chemotherapies and that has a higher tendency to metastasize to other locations. As such, increasing the minimum oxygen tensions found within the heterogeneous tumor may be as important as increasing the overall tumor pO2 when it comes to a therapeutic goal.

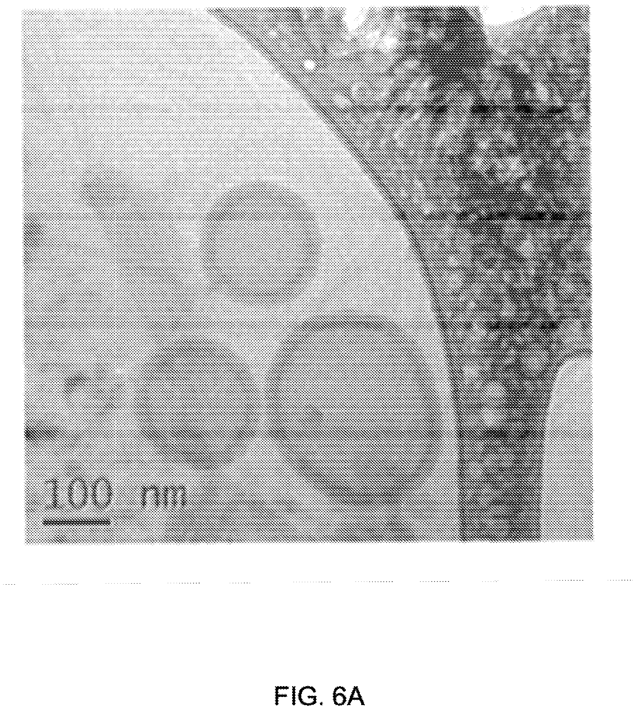

FIG. 6A illustrates that only diblock copolymers of poly(ethylene oxide)-block-poly(ε-caprolactone) (PEO-b-PCL) in which the PEO block was 1-5 kDa and 10-20% of the polymer mass by weight have demonstrated a consistent and significant yield of stable mono-dispersed polymersomes, with mean particle diameters of < 200 nm and membrane thicknesses of 9-22 nm after extrusion through 200-nm diameter pore-cutoff membranes. PEO-b-PCL polymersomes have subsequently been shown to be capable of loading the anti-neoplastic drug doxorubicin (DOX) using an ammonium sulfate gradient.

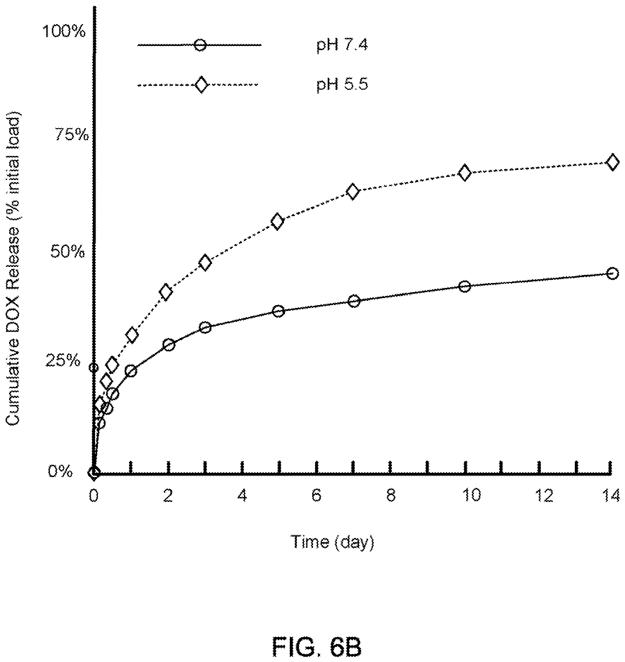

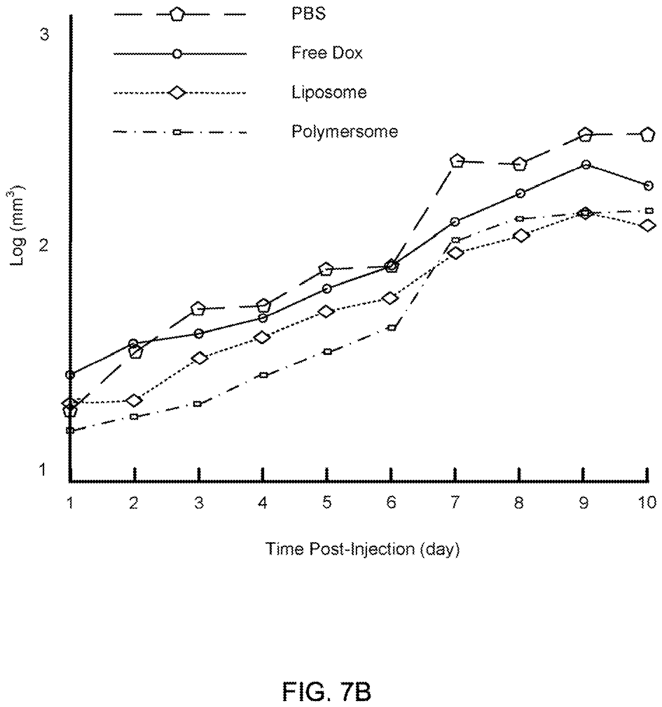

FIG. 6B illustrates the *in vitro* stability, mechanism of degradation and rate of drug release from DOX-loaded PEO(2kDa)-b-PCL(12kDa) polymersomes evaluated as a function of pH over 14 days. FIG. 7B shows that, while the kinetics of release varied under neutral and acidic pH conditions (5.5 and 7.4, at 37 °C), an initial burst release phase (approx. 20% of the initial payload within the first 8 h) was observed at both pH conditions followed by a more controlled, pH-dependent release over the several days. At a pH of 7.4, kinetic release studies show that the encapsulated molecules initially escape the polymersome through passive diffusion of the drug across the intact PCL membrane (days 1-4), and subsequently through hydrolytic matrix degradation of PCL (days 5-14). At a pH of 5.5, however, the dominant mechanism of release, at both short and long times, is acid-catalyzed hydrolysis of the PCL membrane. Notably, these fully-biodegradable polymersomes have a t1/2 half-life of circulation (24-48 h) that is much shorter than their t1/2 half-life of release (2 weeks at pH 7.4). As such, polymersomes can be expected to circulate in the blood stream relatively intact and will release their encapsulated contents in an accelerated fashion only when exposed to lower pH environments, as found in hypoxic tumors.

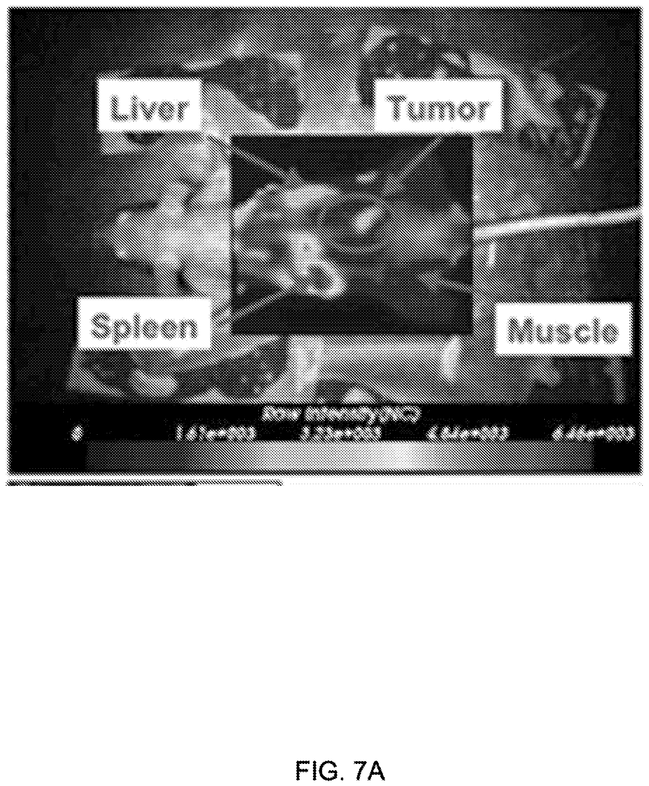

FIG. 7A illustrates the accumulation of an embodiment carrier (Polymersomes) in tumors as demonstrated through *in vivo* optical imaging of oligo(porphyrin)-based near-infrared (NIR) fluorophores (NIRFs) that are incorporated within the membrane shells of the polymersomes. This figure illustrates that polymersomes may accumulate around the tumor through a passive targeting modality due to the enhanced permeation and retention effect (EPR) associated with leaky tumor microvasculature. Further increases in polymersome accumulation may be aided through the inclusion of targeting molecules that will enhance the concentration of polymersomes at the tumor site.
FIG. 7B is a line chart of *in vivo* tumor growth as inhibited by phosphate buffered saline (PBS), doxorubicin (Dox), liposome, and Polymersome. This figure illustrates that not only are polymersomes able to accumulate around tumors (as seen in Fig 7a) but that they do so in sufficient quantities and with preserved intravascular stabilities so as to enable effective release of their encapsulant payload at the tumor site so as to alter tumor biology. When comparing different biodegradable delivery vehicles (e.g. polymersomes vs. liposomes vs. free drug), the superior ability of polymersomes to achieve these outcomes is evident.

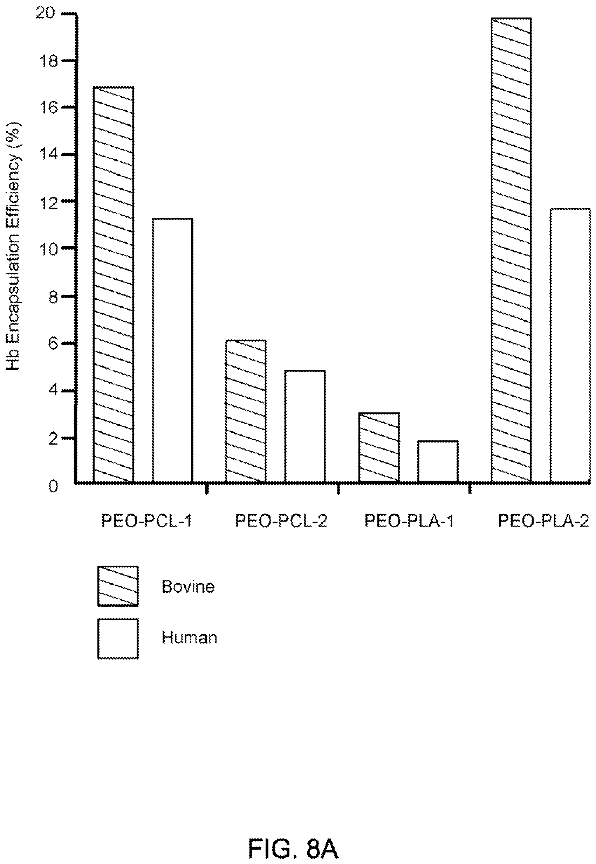

FIG. 8A illustrates the hemoglobin encapsulation efficiencies of four polymersome-encapsulated agent formulations extruded through 200 nm diameter polycarbonate membranes. Specifically, FIG. 8A illustrates the hemoglobin encapsulation efficiency of PEO-b-PCL-1(1.65 KDa), PEO-b-PCL-2(15 KDa), PEO-b-PLA-1(10 kDa) and PEO-b-PLA-2 (2.45 KDa). As discussed above, the various embodiments provide methodology for generating constructs that have an average radius between 100-125 nm with polydispersity index < 1.1 and a hemoglobin encapsulation efficiency > 50%.

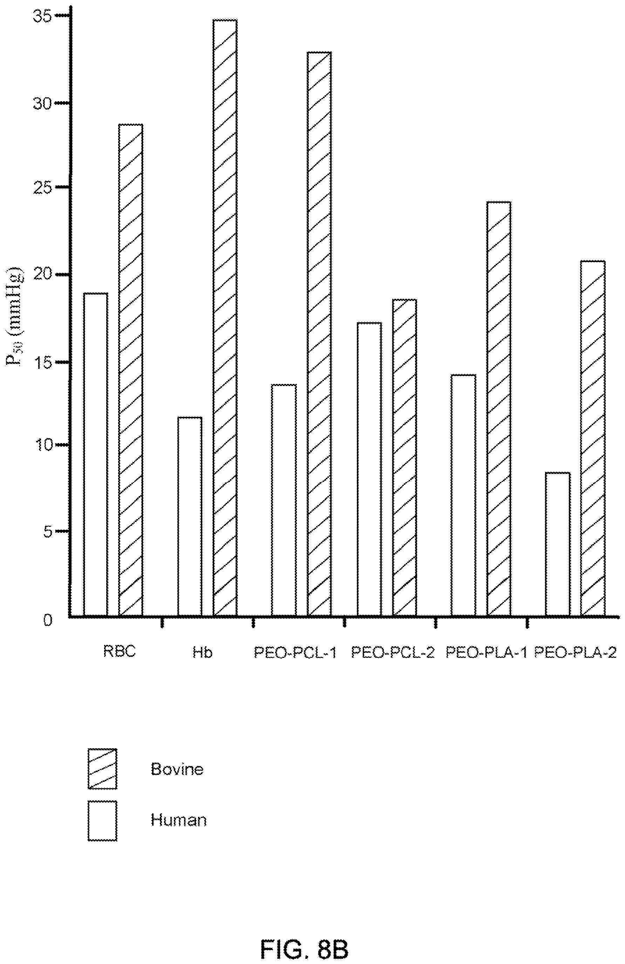

FIG. 8B illustrates the P₅₀ (mmHg) of red blood cells, hemoglobin and four polymersome-encapsulated hemoglobin formulations (PEO-b-PCL-1(1.65 KDa), PEO-b-PCL-2(15 KDa), PEO-b-PLA-1(10 kDa) and PEO-b-PLA-2 (2.45 KDa)) extruded through 200 nm diameter polycarbonate membranes.

As discussed above, polymers are macromolecules comprising chemically conjugated monomeric molecules, wherein the monomeric units being either of a single type (homogeneous) or of a variety of types (heterogeneous). The physical behavior of polymers may be dictated by several factors, including: the total molecular weight, the composition of the polymer (e.g., the relative concentrations of different monomers), the chemical identity of each monomeric unit and its interaction with a solvent, and the architecture of the polymer (whether it is single chain or consists of branched chains). For example, in polyethylene gylcol (PEG), which is a polymer of ethylene gylcol (EG), the chain lengths of which, when covalently attached to a phospholipid, optimize the circulation life of a liposome, is known to be in the approximate range of 34 -114 covalently linked monomers (EG34 to EG114). The preferred embodiments comprise hydrophilic copolymers of polyethylene oxide (PEO), a polymer that is related to PEG), and one of several hydrophobic blocks that drive self-assembly of the polymersomes, up to microns in diameter, in water and other aqueous media.

As discussed above, an amphiphilic substance is one containing both polar (water-soluble) and hydrophobic (water-insoluble) groups. To form a stable membrane in water, a potential minimum requisite molecular weight for an amphiphile must exceed that of methanol HOCH3, which is the smallest canonical amphiphile, with one end polar (HO-) and the other end hydrophobic (-CH3). Formation of a stable lamellar phase requires an amphiphile with a hydrophilic group whose projected area is approximately equal to the volume divided by the maximum dimension of the hydrophobic portion of the amphiphile.

In some embodiments, the oxygen carrier, nanoparticle and/or polymersome does not include polyethylene glycol (PEG) or polyethylene oxide (PEO) as one of its plurality of polymers. In some embodiments, the oxygen carrier, nanoparticle and/or polymersome include least one hydrophilic polymer that is polyethylene glycol (PEG) or polyethyelene oxide (PEO). In some embodiments, the PEG or PEO polymer may vary in molecular weight from about 5 kDaltons (kDa) to about 50 kDa in molecular weight.

The most common lamellae-forming amphiphiles may have a hydrophilic volume fraction between 20 and 50%. In some embodiments, the hydrophilic volume fraction of the oxygen carriers, nanoparticles and/or polymersomes is up to about 20%. In some embodiments, the hydrophilic volume fraction of the oxygen carriers, nanoparticles and/or polymersomes is up to about 19%. In some embodiments, the hydrophilic volume fraction of the oxygen carriers, nanoparticles and/or polymersomes is up to about 18%. In some embodiments, the hydrophilic volume fraction of the oxygen carriers, nanoparticles and/or polymersomes is up to about 17%. In some embodiments, the hydrophilic volume fraction of the oxygen carriers, nanoparticles and/or polymersomes is up to about 16%. In some embodiments, the hydrophilic volume fraction of the oxygen carriers, nanoparticles and/or polymersomes is up to about 15%. In some embodiments, the hydrophilic volume fraction of the oxygen carriers, nanoparticles and/or polymersomes is less than 20%. In some embodiments, the hydrophilic volume fraction of the oxygen carriers, nanoparticles and/or polymersomes is from about 1% to about 20%. It should be noted that the ability of amphiphilic and super-amphiphilic molecules to self-assemble can be largely assessed, without undue experimentation, by suspending the synthetic super-amphiphile in aqueous solution and looking for lamellar and vesicular structures as judged by simple observation under any basic optical microscope, cryogenic transmission electron microscope, or through the scattering of light.

The effective amount of the composition may be dependent on any number of variables, including without limitation, the species, breed, size, height, weight, age, overall health of the subject, the type of formulation, the mode or manner of administration, the type and/or severity of the particular condition being treated, or the need to modulate the activity of the molecular pathway induced by association of the analog to its receptor. The appropriate effective amount can be routinely determined by those of skill in the art using routine optimization techniques and the skilled and informed judgment of the practitioner and other factors evident to those skilled in the art.

A therapeutically effective dose of the oxygen carriers of the various embodiments may provide partial or complete biological activity as compared to the biological activity of a patient's or subject's physiologically mean, median or minimum tissue oxygenation. A therapeutically effective dose of the oxygen carriers of the various embodiments may provide a complete or partial amelioration of symptoms associated with a disease, disorder or ailment.

The oxygen carriers of the various embodiments may delay the onset or lower the chances that a subject develops one or more symptoms associated with the disease, disorder, or ailment. In some embodiments, an effective amount is the amount of a compound suitable to treat or prevent a consequence resulting from low or poor tissue oxygenation. According to the various embodiments, the effective amount of active compound(s) suitable for use in the therapeutic treatment of conditions caused by or contributing to low or poor tissue oxygenation varies depending upon the manner of administration, the age, body weight, and general health of the patient.

Soluble amphiphiles, proteins, ligands, allosteric effectors, oxygen binding compounds can bind to and/or intercalate within a membrane. Such a membrane must also be semi-permeable to solutes, sub-microscopic in its thickness (d), and result from a process of self-assembly or directed assembly. The membrane can have fluid or solid properties, depending on temperature and on the chemistry of the amphiphiles from which it is formed. At some temperatures, the membrane can be fluid (having a measurable viscosity), or it can be solid-like, with an elasticity and bending rigidity. The membrane can store energy through its mechanical deformation, or it can store electrical energy by maintaining a transmembrane potential. Under some conditions, membranes can adhere to each other and coalesce (fuse).

Myoglobin is used as the oxygen-binding compound. In some embodiments, the oxygen-binding compound is genetically- or chemically-modified myoglobin.

In some embodiments, the oxygen carrier transports an effective amount of oxygen suitable for treating a subject or for preventing a subject from suffering from a disease or disorder in which their blood does not carry or release sufficient levels of oxygen to tissues. In some embodiments the oxygen carrier comprises an effective amount of oxygen suitable for treating or preventing the spread of cancer in a subject in need thereof.

In some embodiments, the allostreic effector is 2,3-Bisphosphoglycerate or an isomer derived there from. Allosteric effectors such as 2,3-Bisphosphoglycerate may increase the offload of oxygen from the oxygen carrier or polymersome of the various embodiments to a tissue or cell that is deoxygenated within a subject.

As mentioned above, critical lysis tension (Tc) is the tension at which a particle ruptures when subject to an external force, as measured by micropipette aspiration and expressed as milliNewtons/meter (mN/m). The change in critical lysis tension of an oxygen carrier or polymersome may be measured before and after loading of the oxygen carrier, nanoparticle and/or polymersome with myoglobin.

In various embodiments, the oxygen carriers, nanoparticles and/or polymersomes have a change of critical lysis tension of no more than 20%. In various embodiments, the oxygen carriers, nanoparticles and/or polymersomes have a change of critical lysis tension of no more than 19%. In various embodiments, the oxygen carriers, nanoparticles and/or polymersomes have a change of critical lysis tension of no more than 18%. In various embodiments, the oxygen carriers, nanoparticles and/or polymersomes have a change of critical lysis tension of no more than 17%. In various embodiments, the oxygen carriers, nanoparticles and/or polymersomes have a change of critical lysis tension of no more than 16%. In various embodiments, the oxygen carriers, nanoparticles and/or polymersomes have a change of critical lysis tension of no more than 15%. In various embodiments, the oxygen carriers, nanoparticles and/or polymersomes have a change of critical lysis tension of no more than 14%. In various embodiments, the oxygen carriers, nanoparticles and/or polymersomes have a change of critical lysis tension of no more than 13%. In various embodiments, the oxygen carriers, nanoparticles and/or polymersomes have a change of critical lysis tension of no more than 12%. In various embodiments, the oxygen carriers, nanoparticles and/or polymersomes have a change of critical lysis tension of no more than 11%. In various embodiments, the oxygen carriers, nanoparticles and/or polymersomes have a change of critical lysis tension of no more than 10%. In various embodiments, the oxygen carriers, nanoparticles and/or polymersomes have a change of critical lysis tension from about 5% to about 10%. In various embodiments, the oxygen carriers, nanoparticles and/or polymersomes may have a change of critical lysis tension from about 10% to about 15%. In various embodiments, the oxygen carriers, nanoparticles and/or polymersomes have a change of critical lysis tension from about 15% to about 20%. In various embodiments, the oxygen carriers, nanoparticles and/or polymersomes have a change of critical lysis tension from about 1% to about 5%.

As mentioned above, critical areal strain (Ac) is the areal strain realized by the oxygen carriers, nanoparticles and/or polymersomes at the critical lysis tension. In various embodiments, the oxygen carriers, nanoparticles and/or polymersomes have a critical areal strain from about 20% to about 50%. In various embodiments, the oxygen carriers, nanoparticles and/or polymersomes have a critical areal strain from about 20% to about 25%. In various embodiments, the oxygen carriers, nanoparticles and/or polymersomes have a critical areal strain from about 25% to about 30%. In various embodiments, the oxygen carriers, nanoparticles and/or polymersomes have a critical areal strain from about 30% to about 35%. In various embodiments, the oxygen carriers, nanoparticles and/or polymersomes have a critical areal strain from about 35% to about 40%. In various embodiments, the oxygen carriers, nanoparticles and/or polymersomes have a critical areal strain from about 40% to about 45%. In various embodiments, the oxygen carriers, nanoparticles and/or polymersomes have a critical areal strain from about 45% to about 50%.

As mentioned above, a "myoglobin loading capacity" is a measurement of a myglobin-based oxygen carrier and is defined as the weight of myoglobin within the oxygen carrier divided by the total weight of carrier. In various embodiments, the oxygen carriers, nanoparticles and/or polymersomes have a myoglobin loading capacity of greater than about 5. In various embodiments, the oxygen carriers, nanoparticles and/or polymersomes have a myoglobin loading capacity of greater than 10. In various embodiments, the oxygen carriers, nanoparticles and/or polymersomes have a myoglobin loading capacity of greater than 15. In various embodiments, the oxygen carriers, nanoparticles and/or polymersomes have a myoglobin loading capacity of greater than 20. In various embodiments, the oxygen carriers, nanoparticles and/or polymersomes have a myoglobin loading capacity of greater than 25. In various embodiments, the oxygen carriers, nanoparticles and/or polymersomes have a myoglobin loading capacity of greater than 26. In various embodiments, the oxygen carriers, nanoparticles and/or polymersomes have a myoglobin loading capacity of greater than 27. In various embodiments, the oxygen carriers, nanoparticles and/or polymersomes have a myoglobin loading capacity of greater than 28. In various embodiments, the oxygen carriers, nanoparticles and/or polymersomes have a myoglobin loading capacity of greater than 29. In various embodiments, the oxygen carriers, nanoparticles and/or polymersomes have a myoglobin loading capacity of greater than 30.

As mentioned above, a "myoglobin loading efficiency" is a fundamental measurement of a myoglobin-based oxygen carrier and is defined as the weight of myoglobin that is encapsulated and/or incorporated within a carrier suspension divided by the weight of the original myoglobin in solution prior to encapsulation (expressed as a %). In various embodiments, the oxygen carriers, nanoparticles and/or polymersomes have a myoglobin loading efficiency of greater than about 10%. In various embodiments, the oxygen carriers, nanoparticles and/or polymersomes have a myoglobin loading efficiency of greater than about 11%. In various embodiments, the oxygen carriers, nanoparticles and/or polymersomes have a myoglobin loading efficiency of greater than about 12%. In various embodiments, the oxygen carriers, nanoparticles and/or polymersomes have a myoglobin loading efficiency of greater than about 13%. In various embodiments, the oxygen carriers, nanoparticles and/or polymersomes have a myoglobin loading efficiency of greater than about 14%. In various embodiments, the oxygen carriers, nanoparticles and/or polymersomes have a myoglobin loading efficiency of greater than about 15%. In various embodiments, the oxygen carriers, nanoparticles and/or polymersomes have a myoglobin loading efficiency of greater than about 16%. In various embodiments, the oxygen carriers, nanoparticles and/or polymersomes have a myoglobin loading efficiency of greater than about 17%. In various embodiments, the oxygen carriers, nanoparticles and/or polymersomes have a myoglobin loading efficiency of greater than about 18%. In various embodiments, the oxygen carriers, nanoparticles and/or polymersomes have a myoglobin loading efficiency of greater than about 19%. In various embodiments, the oxygen carriers, nanoparticles and/or polymersomes have a myoglobin loading efficiency of greater than about 20%. In various embodiments, the oxygen carriers, nanoparticles and/or polymersomes have a myoglobin loading efficiency of greater than about 21%. In various embodiments, the oxygen carriers, nanoparticles and/or polymersomes have a myoglobin loading efficiency of greater than about 22%. In various embodiments, the oxygen carriers, nanoparticles and/or polymersomes have a myoglobin loading efficiency of greater than about 23%. In various embodiments, the oxygen carriers, nanoparticles and/or polymersomes have a myoglobin loading efficiency of greater than about 24%. In various embodiments, the oxygen carriers, nanoparticles and/or polymersomes have a myoglobin loading efficiency of greater than about 25%. In various embodiments, the oxygen carriers, nanoparticles and/or polymersomes have a myoglobin loading efficiency of greater than about 26%. In various embodiments, the oxygen carriers, nanoparticles and/or polymersomes have a myoglobin loading efficiency of greater than about 27%. In various embodiments, the oxygen carriers, nanoparticles and/or polymersomes have a myoglobin loading efficiency of greater than about 28%. In various embodiments, the oxygen carriers, nanoparticles and/or polymersomes have a myoglobin loading efficiency of greater than about 29%. In various embodiments, the oxygen carriers, nanoparticles and/or polymersomes have a myoglobin loading efficiency of greater than about 30%.

In various embodiments, the oxygen carriers, nanoparticles and/or polymersomes have a myoglobin loading efficiency from about 10% to about 35%. In various embodiments, the oxygen carriers, nanoparticles and/or polymersomes have a myoglobin loading efficiency from about 15% to about 35%. In various embodiments, the oxygen carriers, nanoparticles and/or polymersomes have a myoglobin loading efficiency from about 18% to about 35%. In various embodiments, the oxygen carriers, nanoparticles and/or polymersomes have a myoglobin loading efficiency from about 20% to about 35%. In various embodiments, the oxygen carriers, nanoparticles and/or polymersomes have a myoglobin loading efficiency from about 22% to about 35%. In various embodiments, the oxygen carriers, nanoparticles and/or polymersomes have a myoglobin loading efficiency from about 24% to about 35%. In various embodiments, the oxygen carriers, nanoparticles and/or polymersomes have a myoglobin loading efficiency from about 26% to about 35%. In various embodiments, the oxygen carriers, nanoparticles and/or polymersomes have a myoglobin loading efficiency from about 28% to about 35%. In various embodiments, the oxygen carriers, nanoparticles and/or polymersomes have a myoglobin loading efficiency from about 30% to about 35%.

The various embodiments include compositions and methods for making and storing oxygen carriers comprising of an oxygen-binding compound encapsulated in a nanoparticle such as a polymersome. The oxygen-binding compound comprises myoglobin.

The various embodiments include compositions and methods for making and storing oxygen carriers comprising myoglobin as oxygen-binding compound encapsulated in a vehicle such as a polymersome.

Some embodiments may further inlcude compositions and methods for developing polymersome-encapsulated myoglobin (PEM) as oxygen carriers. In various embodiments, the PEM may include polymersomes comprising of poly(ethylene oxide)-block-poly(ε-caprolactone) (PEO-b-PCL) and related diblock copolymers of poly(ethylene oxide)-block-poly(y-methyl ε-caprolactone) (PEO-b-PMCL). PEO may provide the polymersomes improved *in vitro* chemical stability, augmented *in vivo* bioavailability and prolonged blood circulation half-lives. Both PEO-b-PCL and PEO-b-PMCL may afford complete and safe *in vivo* biodegradation of polymersome membranes via hydrolysis of their ester linkages. In various embodiments, the biodegradable polymersome-encapsulated myoglobin (PEM) dispersions may be comprised of diblock copolymers of PEO-b-PCL with a PEO block size of ∼1.5-2kDa and with a block fraction of -10-20% by weight. In various embodiments, the biodegradable polymersome-encapsulated myoglobin (PEM) dispersions may be comprised of diblock copolymers of PEO-b-PCL with a PCL block size of ∼8kDa-23kDa and with a block weight fraction of about ∼ 50 to 85 percent. In other embodiments, the PEM dispersions may be comprised of diblock copolymers of PEO-b-PMCL. PEO-b-PCL and PEO-b-PMCL polymersomes may be preferred cellular myoglobin-based oxygen carriers (MBOCs) and possess all the requisite properties for effective oxygen delivery, including tunable oxygen-binding capacities, uniform and appropriately small size distributions, human bloodlike viscosities and oncotic properties, as well as ease of mass production and affordable storage.

In an embodiment, a supramolecular self-assembly approach may be used to prepare mono-disperse unilamellar polymersomes (50-300 nm diameter) that incorporate high quantum yield oligo(porphyrin)-based near-infrared (NIR) fluorophores (NIRFs) within their bilayer membranes^{124, 130, 114-111}. These bright, NIR-emissive polymersomes may possess the requisite photophysical properties and biocompatibility for ultra-sensitive *in vivo* optical imaging.^{111, 124, 164, 168} Imaging studies of tumor-bearing mice have shown that non-targeted polymersomes are able to accumulate in tumors after intravascular injection due to the Enhanced Permeability and Retention (EPR) effect associated with leaky tumor microvasculature; quantitative fluorescence analysis has shown that a greater than two times tumor accumulation is readily achieved (FIGURE 7A).¹⁶⁴ Tumor-specific accumulation may further be enhanced by modifying polymersome surfaces through chemical conjugation to targeting ligands, such as small molecules, peptides, proteins (e.g. antibodies), and nucleic acids.^{111, 127, 164}

Some embodiments include an operating methodology to synthesize PEM dispersions that consistently meet the following standard characteristics: (i) average radius between 100-200 nm with polydispersity index < 1.1 (ii) Mb encapsulation efficiency > 50 mol%; (iii) weight ratio of encapsulated Mb:polymer > 2; (iv) solution metMb level < 5%; (v) suspension viscosity between 3-4 cP; (vi) P50 between 2-3 mm Hg; and, (vii) at least an order of magnitude smaller NO binding rate constant as that measured for free Mb at similar weight per volume of distribution; (viii) final suspension concentration of between 80 to 180 mg Mb/mL solution; and (ix) excellent stability under different storage and flow conditions as determined by intact morphology, change in average particle diameter < 5 nm and unaltered Mb concentration (change < 0.5 g/dL) and unchanged metMb level (change < 2%).

The various embodiment PEMs may differ in their combination of particle size, deformability and concentration. Each of these parameters may independently affect the amount of Mb per particle, particle stability, and the numbers of particles that will accumulate at the tumor site. PEMs may be formed that are either 100 nm or 200 nm in mean particle diameter. Polymersomes, like other nanoparticles that are smaller than 250 nm in diameter may accrue in solid tumors due to the EPR effect.^{111, 114, 164} Although polymersomes with 200 nm mean particular diameter may deliver more Mb per particle, those that are -100 nm in diameter may exhibit longer blood circulation half-lives (before eventual clearance by the RES)^{)102, 111, 114, 136, 172} and may demonstrate enhanced tumor accumulation by traversing plasma channel^{s173} (small microvessels that exclude RBCs).

An embodiment PEM may be constructed from either PEO-b-PCL, poly(ethylene oxide)-block-poly(y-methyl ε-caprolactone) (PEO-b-PMCL), and/or poly(ethylene oxide)-block-poly(trimethylcarbonate) (PEO-b-PTMC) diblock copolymers in order to determine the ultimate balance of particle stability versus deformability that may maximize *in vivo* tumor delivery. PMCL, as a derivative of PCL, similarly forms fully bioresorbable polymersomes that degrade via non-enzymatic hydrolysis of ester linkages.¹⁷⁴ PEO-b-PCL, however, may yield ultra-stable, solid vesicle membranes while PEO-b-PMCL and PEO-b-TMC may generate more deformable polymersomes, a characteristic that may aid in PEM passage through tortuous tumor blood vessels.

The various embodiments may include a polymersomes nanoparticle, or other oxygen carriers with varying sizes. In various embodiments, the polymersome or oxygen carrier includes a roughly spherical shape and has a diameter of about 50 nm to about 1 µm. In various embodiments, the polymersome or oxygen carrier has a diameter of about 50 nm to about 250 nm. In various embodiments, the polymersome or oxygen carrier has a diameter of about 100 nm to about 200 nm. In various embodiments, the polymersome or oxygen carrier has a diameter of about 200 nm to about 300 nm. In various embodiments, the polymersome or oxygen carrier has a diameter of about 300 nm to about 400 nm. In various embodiments, the polymersome or oxygen carrier has a diameter of about 400 nm to about 500 nm. In various embodiments, the polymersome or oxygen carrier has a diameter of about 500 nm to about 600 nm. In various embodiments, the polymersome or oxygen carrier has a diameter of about 600 nm to about 700 nm. In various embodiments, the polymersome or oxygen carrier has a diameter of about 700 nm to about 800 nm. In various embodiments, the polymersome or oxygen carrier has a diameter of about 800 nm to about 900 nm. In various embodiments, the polymersome or oxygen carrier has a diameter of about 900 nm to about 1 µm.

In various embodiments, the oxygen carrier consists of a nanoparticle that has a diameter of about 5 nm to about 100 nm. In various embodiments, the oxygen carrier has a diameter of about 5 nm to about 10 nm. In various embodiments, the oxygen carrier has a diameter of about 10 nm to about 50 nm. In various embodiments, the oxygen carrier has a diameter of about 50 nm to about 100 nm. In various embodiments, the oxygen carrier has a diameter of about 100 nm to about 300 nm. In various embodiments, the oxygen carrier has a diameter of about 300 nm to about 500 nm. In various embodiments, the oxygen carrier has a diameter of about 500 nm to about 1 µm.

In a further embodiment, the oxygen carriers, nanoparticle and/or polymersomes may include varying membrane thicknesses. The thickness of the membrane may depend upon the molecular weight of the polymers and the types of polymers used in the preparation of the oxygen carriers or polymersomes. In various embodiments, the membrane may be a single, double, triple, quadruple, or more layers of polymers. In various embodiments, the oxygen carriers, nanoparticle and/or polymersomes have a polymer membrane thickness from about 5 nm to about 35 nm. In various embodiments, the oxygen carriers, nanoparticle and/or polymersomes have a membrane thickness from about 5 nm to about 10 nm. In various embodiments, the oxygen carriers, nanoparticle and/or polymersomes have a membrane thickness from about 10 nm to about 15 nm. In various embodiments, the oxygen carriers, nanoparticle and/or polymersomes have a membrane thickness from about 15 nm to about 20 nm. In various embodiments, the oxygen carriers, nanoparticle and/or polymersomes have a membrane thickness from about 20 nm to about 25 nm. In various embodiments, the oxygen carriers, nanoparticle and/or polymersomes have a membrane thickness from about 25 nm to about 30 nm. In various embodiments, the oxygen carriers, nanoparticle and/or polymersomes have a membrane thickness from about 30 nm to about 35 nm. In various embodiments, the oxygen carriers, nanoparticle and/or polymersomes have a polymer membrane that is no more than about 5 nm in thickness. In various embodiments, the oxygen carriers, nanoparticle and/or polymersomes have a polymer membrane that is no more than about 10 nm in thickness. In various embodiments, the oxygen carriers, nanoparticle and/or polymersomes have a polymer membrane that is no more than about 15 nm in thickness. In various embodiments, the oxygen carriers, nanoparticle and/or polymersomes have has a polymer membrane that is no more than about 20 nm in thickness. In various embodiments, the oxygen carriers, nanoparticle and/or polymersomes have a polymer membrane that is no more than about 25 nm in thickness. In various embodiments, the oxygen carriers, nanoparticle and/or polymersomes have a polymer membrane that is no more than about 30 nm in thickness. In various embodiments, the oxygen carriers, nanoparticle and/or polymersomes have a polymer membrane that is no more than about 35 nm in thickness.

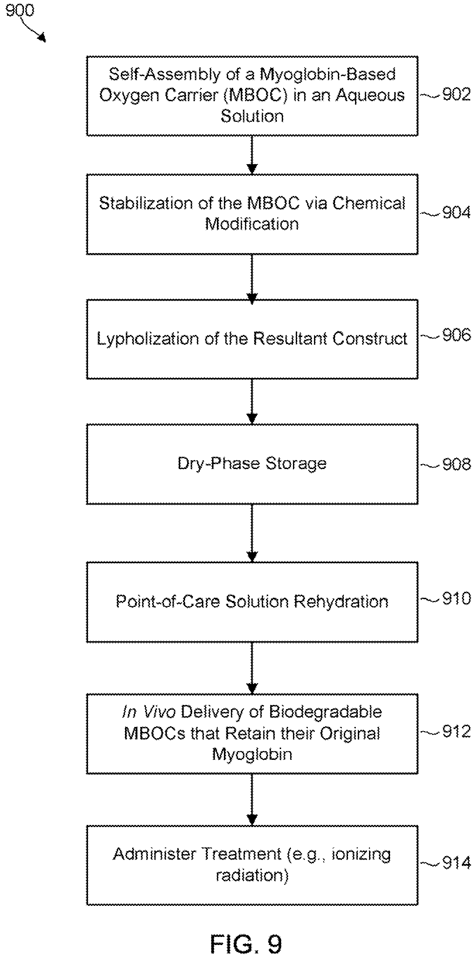

FIG. 9 illustrates a method 900 for MBOC preparation. In step 902, the myoglobin-based oxygen carrier (MBOC) is self-assembled in aqueous solution. In step 904, the myoglobin-based oxygen carrier is stabilized via chemical modification. In step 906, the resultant construct is lypholized. In step 908, the resultant construct is stored via dry-phase storage. In step 910, point-of-care solution rehydration. In step 912, biodegradable MBOCs that retain their original myoglobin may be delivered *in vivo.* As a non-limiting example, polymersome-encapsulated Mb may be prepared and generated via such an MBOC preparation method. In step 914, MBOC and/or high-oxygen affinity agent comprising myoglobin and ionizing radiation may be administered to the tumor of a patient.

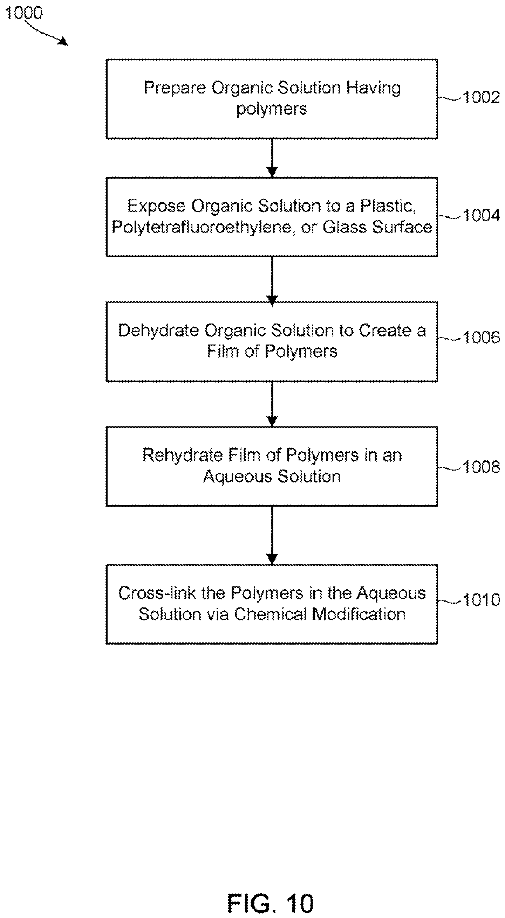

FIG. 10 illustrates a method 1000 for preparing a polymersome comprising at least one biocompatible polymer and at least one biodegradable polymer. It should be noted that FIG. 10 provides a high-level overview of the method steps and that details for each step are provided further below. In step 1002, an organic solution having a plurality of polymers may be prepared. In step 1004, the organic solution comprising the plurality of polymers may be exposed to a plastic, polytetrafluoroethylene (i.e., Teflon™) (herein "PTFE"), or glass surface. In step 1006, the organic solution may be dehydrated on the plastic, PTFE, or glass surface to create a film of polymers. In step 1008, the film of polymers may be rehydrated in an aqueous solution. In step 1010, the polymers may be cross-linked in the aqueous solution via chemical modification.

Polymersomes of the various embodiment PEM may comprise copolymers that are synthesized to include polymerizable groups within either their hydrophilic or hydrophobic blocks. The polymerizable biodegradable polymers may be utilized to form polymersomes that co-incorporate Mb and a water-soluble initiator in their aqueous interiors, or alternatively, by compartmentalizing Mb in their aqueous cavities and a water-insoluble initiator in their hydrophobic membranes.

The various embodiments may further include a method for preparing a polymersome comprising at least one biocompatible polymer and at least one biodegradable polymer comprising: (a) preparing an organic solution comprising a plurality of polymers and exposing the organic solution comprising the plurality of polymers to a plastic, polytetrafluoroethylene (PTFE) (a.k.a. Teflon®), or glass surface; (b) dehydrating the organic solution on the plastic, Teflon®, or glass surface to create a film of polymers; and (c) rehydrating the film of polymers in an aqueous solution of myoglobin; (d) cross-linking the polymers in the aqueous solution via chemical modification.

The compositions of the various embodiments may be made by direct hydration methods as described in O'Neil, et al.,, Langmuir 2009, 25(16), 9025-9029. Briefly, polymersomes of the various embodiments may be made and encapsulated using the following method: To prepare formulations, 20 total mgs of polymer may be weighed into a 1.5mL centrifuge tube, heated at 95 °C for 20 min, and mixed. After the samples are cooled to room temperature (15 min minimum), 10 µL of protein solution may be added and diluted with 20, 70, and 900 µL of 10 mmol phosphate buffered saline (PBS), pH 7.4, with mixing after each addition. As a control, the polymersomes may be formed via dilution with PBS (10, 20, 70, 890 µL of PBS with mixing after each addition) and finally add 10 µL of myoglobin solution after the formation of the polymersomes. In this way, the encapsulation efficiency and loading may be calculated by subtraction. All samples may be prepared in triplicate. Encapsulation efficiencies may be quantified from standard curves generated from the fluorescently labeled crosslinked to the polymers of choice under investigation.

In a further embodiment method, polymersome preparation may involve large-scale fractionation of vesicular particles. Briefly, a total of 1.25 g of diblock copolymer may be hydrated with 25mL of 10mM phosphate buffer (PB) at pH 7.3. Because of the lows solubility of diblock copolymers in PB, the aqueous polymer mixture may be sonicated (Branson Sonifier 450, VWR Scientific,West Chester, PA) for 8-10 h at room temperature to yield the stock copolymer solution. The stock copolymer solution may be then mixed with 25mL of purified Mb (250-300 g/L) to yield a copolymer concentration of 12.5 mg/mL in the Mb copolymer mixture. Empty polymersomes may be prepared by diluting the stock copolymer solution in PB, instead of purified Mb solution, to yield a copolymer concentration of 12.5 mg/mL. For the 1 mL volume manual extrusion method, the Mb-copolymer/copolymer mixture may be extruded 20 times through either 100 nm or 200 nm diameter polycarbonate membranes (Avanti PolarLipids, Alabaster, AL). However, for the large scale Hollow Fiber (HF) extrusion method (Figures 4 and 5), the Mb-copolymer/copolymer mixture may be extruded through a 0.2 µm HF membrane (Spectrum Laboratories Inc., Rancho Dominguez, CA). For both extrusion methods, extruded PEM dispersions may be dialyzed overnight using 300 kDa molecular weight cutoff (MWCO) dialysis bags (Spectrum Laboratories Inc., Rancho Dominguez, CA) in PB at 4 °C at a 1:1000 Volume/Volume ration (v/v)(extruded PEM/PB) ratio to remove unencapsulated Mb from the vesicular dispersion. An Eclipse asymmetric flow field-flow fractionator (Wyatt Technology Corp., Santa Barbara,CA) coupled in series to an 18 angle Dawn Heleos multi-angle static light scattering photometer (Wyatt Technology Corp., Santa Barbara, CA) may be used to measure the size distribution of empty polymersomes and PEM particles. The light scattering photometer is equipped with a 30 mW GaAs laser operating at a laser wavelength of 658 nm. Light scattering spectra may be analyzed using the ASTRA software package (Wyatt Technology Corp., Santa Barbara, CA) to calculate the particle size distribution. The elution buffer consisted of 10 mM PB at pH 7.3.

It should be noted that while diameter rages are given above, the final diameter of polycarbonate membrane through which polymersomes are extruded will define the ultimate size distribution (diameter) of the polymersomes in that suspension.

Mb Encapsulation in PEM: To measure the amount of Mb that was encapsulated inside PEM particles, dialyzed PEM dispersions were first lysed using 0.5% v/v Triton X100 (Sigma-Aldrich, St. Louis, MO) in PB. Lysed PEM samples may be centrifuged at 14,000 rpm for 15 min, and the supernatant collected for analysis. The concentration of encapsulated Mb obtained after lysing the PEM particles (mg/mL) may be measured using the Bradford method via the Coomassie Plus protein assay kit (Pierce Biotechnology, Rockford, IL).

As a consequence of the reaction of two or more of the polymerizable groups facilitated by the initiator, stabilized PEM dispersions may be generated via formation of covalent bonds between chains of the copolymers forming the polymersome membranes. These stabilized PEM constructs may be further dried via well-established lyophilization protocols without disrupting the formed polymersome structure or losing the encapsulated Mb. In various embodiments, the polymersomes are administered in the aqueous solution. If lyophilized, in various embodiments, the polymersomes are reconstituted in an appropriate aqueous solution and administered to a subject. Lyophilized biodegradable PEM may be stored in a dessicator (free of O₂) at 4 °C for varying periods of time without polymer or Mb degradation as the dried suspensions are free of aqueous free radicals, protons, etc. The polymersomes may be rehydrated at point-of-care prior to delivery.

To generate stabilized polymersomes, polymerizable units may be chemically linked to either the hydrophilic or hydrophobic ends of the copolymer after synthesis. One or more cross-links between multiblock copolymer chains may be formed between the polymerizable units and the hydrophilic or hydrophobic polymers of the various embodiments. These cross-links may be suitably formed by introducing a composition having multiple polymerizable groups to the chains of multiblock copolymer, although in various cases, the multiblock copolymer itself includes multiple polymerizable groups. In various embodiments, the multiple polymerizable groups are chosen from acrylates, methacrylates, acrylamides, methacrylamides, vinyls, vinyl sulfone units or a combination thereof. In various embodiments, the oxygen carriers, nanoparticle and/or polymersomes comprise the polymerizable groups from about 0 weight (wt) % to about 5 wt % of the total weight of the composition. In various embodiments, the oxygen carriers, nanoparticle and/or polymersomes comprise the polymerizable groups from about 5 wt % to about 10 wt % of the total weight of the composition. In various embodiments, the oxygen carriers, nanoparticle and/or polymersomes comprise the polymerizable groups from about 10 wt % to about 20 wt % of the total weight of the composition. In various embodiments, the oxygen carriers, nanoparticle and/or polymersomes comprise the polymerizable groups from about 20 wt % to about 30 wt % of the total weight of the composition. In various embodiments, the oxygen carriers, nanoparticle and/or polymersomes comprise the polymerizable groups from about 30 wt % to about 40 wt % of the total weight of the composition. In various embodiments, the oxygen carriers, nanoparticle and/or polymersomes comprise the polymerizable groups from about 40 wt % to about 50 wt % of the total weight of the composition. In various embodiments, the oxygen carriers, nanoparticle and/or polymersomes comprise the polymerizable groups from about 50 wt % to about 60 wt% of the total weight of the composition. In various embodiments, the oxygen carriers, nanoparticle and/or polymersomes comprise the polymerizable groups from about 60 wt % to about 70 wt % of the total weight of the composition. In various embodiments, the oxygen carriers or the polymersomes comprise the polymerizable groups from about 70 wt % up to about 80 wt % of the total weight of the composition. In various embodiments, the oxygen carriers, nanoparticle and/or polymersomes comprise the polymerizable groups from about 80 wt % up to about 90 wt % of the total weight of the composition. In various embodiments, the oxygen carriers, nanoparticle and/or polymersomes comprise the polymerizable groups from about 90 wt % up to about 95 wt % of the total weight of the composition. In various embodiments, the oxygen carriers, nanoparticle and/or polymersomes comprise the polymerizable groups from about 95 wt % up to about 100 wt % of the total weight of the composition.Cross-linking between chains of a membrane is achieved via activation of the polymerization reaction by an initiator and results in enhancing the rigidity of the polymersome composition. In certain embodiments, the polymerizable group may be conjugated to copolymer's hydrophilic block consisting of either poly(ethylene oxide), poly(ethylene glycol), poly(acrylic acid), and the like. In other embodiments, the polymerizable group may be conjugated to the copolymer's hydrophobic block consisting of either poly(ε-caprolactone), poly(y-methyl ε-caprolactone), poly(trimethylcarbonate), poly(menthide), poly(lactide), poly(glycolide), poly(methylglycolide), poly(dimethylsiloxane), poly(isobutylene), poly(styrene), poly(ethylene), poly(propylene oxide), etc. The initiator may be a molecule that generates/reacts to heat, light, pH, solution ionic strength, osmolarity, pressures, etc. In various embodiments, the initiator may be photoreactive and cross-links the polymers of the oxygen carrier or polymersome via exposure to ultraviolet light.

The compositions of the various embodiments may be prepared without the use of organic solvents. The compositions of the various embodiments may include polymersomes comprising poly(ethylene oxide)-block-poly(ε-caprolactone), poly(ethylene oxide)-block-poly(y-methyl ε-caprolactone) and/or), and/or poly(ethylene oxide)-block-poly(trimethylcarbonate) copolymers that have been modified with an acrylate moiety at the hydrophobic block terminus. In various embodiments, the oxygen carriers, nanoparticle and/or polymersomes may comprise cross-linked polymers formed between the hydrophobic block terminus and a diacrylate using a UV initiator, such as 2,2-dimethoxy-2-phenylacetophenone (DMPA). In various embodiments, DMPA is compartmentalized in the polymersome membrane during polymersome assembly while Mb occupies the internal aqueous compartment of the carrier.

The composition of the various embodiments may also comprise polymersomes, nanoparticles or oxygen carriers that have increased degradative half-lives. Circulation times of oxygen carrier and polymersomes may be generally limited to hours (or up to one day) because of either rapid clearance by the mononuclear phagocytic system (MPS) of the liver and spleen, or by excretion. Clinical studies have shown that circulation times of spherical carriers may be generally extended threefold in humans over rats. As proposed for clinically used drug formulations of PEG-liposomes, oxygen carriers and polymersomes with long circulating lifetime may increase the drug exposure to cancer cells, low oxygenated tissues, or healing wounds, and thereby increase the time-integrated dose, commonly referred to in drug delivery as "the area under the curve." Additionally, the enhanced permeation and retention effect that allows small solutes and micelles to permeate the leaky blood vessels of a rapidly expanding tumor might also allow oxygen carriers and polymersomes to transport into the tumor stroma. Persistent circulation of the oxygen carriers and polymersomes has many practical applications because these vehicles can increase exposure of drugs to cancer cells, low or poor oxygenated tissues, or healing wounds.

The compositions of the various embodiments may comprise polymersomes, nanoparticles or oxygen carriers that have increased circulatory half-lives. In various embodiments, the compositions have a certain percent mass composition of polymer designed to have a circulatory half-life from about 12 hours to about 36 hours, and a degradative half-life from about 38 to about 60 hours.

In various embodiments, the compositions have a certain percent mass composition of polymer designed to have circulatory half-life about 12 hours less than the degradative half-life of the oxygen-carrier or polymersome. This delay in degradation may vary depending upon the route of administration and/or the targeted micro-compartment, the size of the oxygen-carrier or polymesome or subcellular microenvironment. In various embodiments, the compositions comprising polymersomes, nanoparticles or oxygen carriers have circulatory half-life about 11 hours less than the degradative half-life of the oxygen-carrier or polymersome. In various embodiments, the compositions comprising polymersomes, nanoparticles or oxygen carriers have circulatory half-life about 10 hours less than the degradative half-life of the oxygen-carrier or polymersome. In various embodiments, the compositions comprising polymersomes, nanoparticles or oxygen carriers have circulatory half-life about 9 hours less than the degradative half-life of the oxygen-carrier or polymersome. In various embodiments, the compositions comprising polymersomes, nanoparticles or oxygen carriers have circulatory half-life about 8 hours less than the degradative half-life of the oxygen-carrier or polymersome. In various embodiments, the compositions comprising polymersomes, nanoparticles or oxygen carriers have circulatory half-life about 7 hours less than the degradative half-life of the oxygen-carrier or polymersome. In various embodiments, the compositions comprising polymersomes, nanoparticles or oxygen carriers have circulatory half-life about 6 hours less than the degradative half-life of the oxygen-carrier or polymersome. In various embodiments, the compositions comprising polymersomes, nanoparticles or oxygen carriers have circulatory half-life about 5 hours less than the degradative half-life of the oxygen-carrier or polymersome. In various embodiments, the compositions comprising polymersomes, nanoparticles or oxygen carriers have circulatory half-life about 4 hours less than the degradative half-life of the oxygen-carrier or polymersome. In various embodiments, the compositions comprising polymersomes, nanoparticles or oxygen carriers have circulatory half-life about 3 hours less than the degradative half-life of the oxygen-carrier or polymersome. In various embodiments, the compositions comprising polymersomes, nanoparticles or oxygen carriers have circulatory half-life about 2 hours less than the degradative half-life of the oxygen-carrier or polymersome. In various embodiments, the compositions comprising polymersomes, nanoparticles or oxygen carriers have circulatory half-life about 1 hours less than the degradative half-life of the oxygen-carrier or polymersome. In various embodiments, the compositions comprising polymersomes, nanoparticles or oxygen carriers have circulatory half-life about 14 hours less than the degradative half-life of the oxygen-carrier or polymersome. In various embodiments, the compositions comprising polymersomes, nanoparticles or oxygen carriers have circulatory half-life from about 1 hour to about 20 hours less than the degradative half-life of the oxygen-carrier or polymersome. In various embodiments, the compositions comprising polymersomes, nanoparticles or oxygen carriers have a certain percent mass composition of polymer designed to have a circulatory half-life of about 36 hours, and a degradative half-life greater than about 48 hours. In various embodiments, the compositions comprising polymersomes, nanoparticles or oxygen carriers have a certain percent mass composition of polymer designed to have a circulatory half-life from about 24 hours to about 36 hours, and a degradative half-life from about 38 to about 60 hours. In various embodiments, the compositions comprising polymersomes, nanoparticles or oxygen carriers have a certain percent mass composition of polymer designed to have a circulatory half-life from about 28 hours to about 36 hours, and a degradative half-life from about 38 to about 60 hours. In various embodiments, the compositions comprising polymersomes, nanoparticles or oxygen carriers have a certain percent mass composition of polymer designed to have a circulatory half-life from about 30 hours to about 36 hours, and a degradative half-life from about 38 to about 60 hours. In various embodiments, the compositions comprising polymersomes, nanoparticles or oxygen carriers have a certain percent mass composition of polymer designed to have a circulatory half-life of no more than 36 hours, and a degradative half-life from about 38 to about 60 hours.

In various embodiments, the degradation half-life is 6 hours greater than the circulatory half-life. In various embodiments, the degradation half-life is between 6 hours and 24 hours greater than the circulatory half-life. In various embodiments, the degradation half-life is more than 24 hours greater than the circulatory half-life. In various embodiments, the degradation half-life is about 6 hours greater than the circulatory half-life. In various embodiments, the degradation half-life is about 7 hours greater than the circulatory half-life. In various embodiments, the degradation half-life is about 8 hours greater than the circulatory half-life. In various embodiments, the degradation half-life is about 9 hours greater than the circulatory half-life. In various embodiments, the degradation half-life is about 10 hours greater than the circulatory half-life. In various embodiments, the degradation half-life is about 11 hours greater than the circulatory half-life. In various embodiments, the degradation half-life is about 12 hours greater than the circulatory half-life. In various embodiments, the degradation half-life is about 13 hours greater than the circulatory half-life. In various embodiments, the degradation half-life is about 14 hours greater than the circulatory half-life. In various embodiments, the degradation half-life is about 15 hours greater than the circulatory half-life. In various embodiments, the degradation half-life is about 16 hours greater than the circulatory half-life. In various embodiments, the degradation half-life is about 17 hours greater than the circulatory half-life. In various embodiments, the degradation half-life is about 18 hours greater than the circulatory half-life. In various embodiments, the degradation half-life is about 19 hours greater than the circulatory half-life. In various embodiments, the degradation half-life is about 20 hours greater than the circulatory half-life. In various embodiments, the degradation half-life is about 21 hours greater than the circulatory half-life. In various embodiments, the degradation half-life is about 22 hours greater than the circulatory half-life. In various embodiments, the degradation half-life is about 23 hours greater than the circulatory half-life. In various embodiments, the degradation half-life is about 24 hours greater than the circulatory half-life. In various embodiments, the degradation half-life is about 36 hours greater than the circulatory half-life. In various embodiments, the degradation half-life is about 48 hours greater than the circulatory half-life. In various embodiments, the degradation half-life is about 60 hours greater than the circulatory half-life. In various embodiments, the degradation half-life is about 72 hours greater than the circulatory half-life. In various embodiments, the degradation half-life is more than 96 hours greater than the circulatory half-life.

In vivo delivery may be achieved by intravenous, inhalational, transmucosal (e.g. buccal) or transcutaneous routes of administration. Dosages for a given host may be determined using conventional considerations, e.g., by customary comparison of the differential activities of the subject preparations and a known appropriate, conventional pharmacological protocol.

In an embodiment, different final concentrations of PEMs may be used in order to test the effects of Mb dose on improving oxygenation and mitigating tumor hypoxia. 100 uL injections of PEMs that contain either 90 or 180 mg Mb/mL may result in 450 or 900 mg/kg injection doses of Mb, respectively, assuming a 20 g mouse. These doses correspond to the total hemoglobin injection dose found in 0.5 and 1 unit of whole blood, assuming 15 g/dL blood concentrations, 450 mL blood/unit, and a 70 kg human. While larger PEM doses may likely enhance Mb tumor delivery, increased amounts of free Mb (released during PEM degradation) may also result in local NO uptake, decreased microperfusion, and ineffective oxygenation.¹⁷⁵ In a preferred embodiment, the associated polymer concentrations and subject injection doses may range between 2.5-18 mg/mL and 12.5-90 mg/kg, assuming a final weight ratio of encapsulated Mb:polymer ranging between 10-35, both of which fall well within the range of previous animal studies that demonstrated no subacute or acute in vivo toxicities from various polymersome compositions.^{111, 122, 164, 168, 171}

The pharmaceutical composition of the various embodiments may be an oxygen carrier that possess different "loading ratios" of myoglobin as oxygen binding agent to inert vehicle. In various embodiments, the pharmaceutical composition comprises < 5 mg myoglobin/mg inert vehicle. In various embodiments, the pharmaceutical composition comprises from about 5 to about 40 mg myoglobin/mg inert vehicle. In various embodiments, the pharmaceutical composition comprises from about 10 to about 40 mg myoglobin/mg polymer inert vehicle. In various embodiments, the pharmaceutical composition comprises from about 20 to about 40 mg myoglobin/mg inert vehicle. In various embodiments, the pharmaceutical composition comprises from about 30 to about 40 mg myoglobin/mg inert vehicle. In various embodiments, the pharmaceutical composition comprises from about 35 to about 40 mg myoglobin/mg inert vehicle. In various embodiments, the pharmaceutical composition comprises from about 25 to about 40 mg myoglobin/mg inert vehicle. In various embodiments, the pharmaceutical composition comprises from about 25 to about 35 mg myoglobin/mg inert vehicle. In various embodiments, the pharmaceutical composition comprises from about 25 to about 30 mg myoglobin/mg inert vehicle. In various embodiments, the pharmaceutical composition comprises from about 20 to about 25 mg myoglobin/mg inert vehicle. In various embodiments, the pharmaceutical composition comprises from about 10 to about 15 mg myoglobin/mg inert vehicle.

In various embodiments, the pharmaceutical composition comprises from about 5 to about 35 mg Mb/mg polymer. In various embodiments, the pharmaceutical composition comprises from about 10 to about 35 mg Mb/mg polymer. In various embodiments, the pharmaceutical composition comprises from about 20 to about 35 mg Mb/mg polymer. In various embodiments, the pharmaceutical composition comprises from about 30 to about 35 mg Mb/mg polymer. In various embodiments, the pharmaceutical composition comprises from about 25 to about 35 mg Mb/mg polymer. In various embodiments, the pharmaceutical composition comprises from about 25 to about 30 mg Mb/mg polymer. In various embodiments, the pharmaceutical composition comprises from about 20 to about 25 mg Mb/mg polymer. In various embodiments, the pharmaceutical composition comprise from about 10 to about 15 mg Mb/mg polymer. In various embodiments, the pharmaceutical composition comprise from about 5 to about 10 mg Mb/mg polymer. In various embodiments the Mb dosages may be replaced by the same weight of Mb.

In various embodiments, the pharmaceutical composition is a liquid formulation, wherein the dosage may be from about 1 unit of compositions to about 50 units of oxygen-carrier suspension, wherein a unit of suspension comprises from about 40 g of Mb to about 85 g of Mb. In an embodiment, the high-oxygen affinity agent/compound has a P50 for oxygen that is less than 5 mmHg.

In various embodiments, a unit of a liquid formulation comprising the pharmaceutical composition comprises about 41 grams of Mb. In various embodiments, a unit of a liquid formulation comprising the pharmaceutical composition comprises about 45 grams of Mb. In various embodiments, a unit of a liquid formulation comprising the pharmaceutical composition comprises about 50 grams of Mb. In various embodiments, a unit of a liquid formulation comprising the pharmaceutical composition comprises about 55 grams of Mb. In various embodiments, a unit of a liquid formulation comprising the pharmaceutical composition comprises about 60 grams of Mb. In various embodiments, a unit of a liquid formulation comprising the pharmaceutical composition comprises about 65 grams of Mb. In various embodiments, a unit of a liquid formulation comprising the pharmaceutical composition comprises about 70 grams of Mg. In various embodiments, a unit of a liquid formulation comprising the pharmaceutical composition comprises about 75 grams of Mb. In various embodiments, a unit of a liquid formulation comprising the pharmaceutical composition comprises about 80 grams of Mb. In various embodiments, a unit of a liquid formulation comprising the pharmaceutical composition comprises about 85 grams of Mb.

Generally, a pharmaceutical composition according to the various embodiments may comprise a dose of myoglobin as oxygen-binding protein that may be suspended within a solution and administered in units, where a unit is equal to 81 grams of oxygen-binding protein. If a subject undergoes surgery or experiences blood loss, the pharmaceutical composition may be administered to the subject according to the following dosing regimen, where blood is replaced with units of liquid formulation: In various embodiments, the pharmaceutical composition comprises from about 40 g of oxygen binding protein/unit of solution to about 81 g of oxygen binding protein/unit of solution.

| **Examples Of Blood Use** | **Average # Unites Required per Patient** |
|---|---|
| Automobile Accident | 50 units of blood |
| Heart Surgery | 6 units of blood |
| | 6 units of platelets |
| Organ Transplant | 40 units of blood |
| | 30 units of platelets |
| | 20 bags of cryoprocipitate |
| | 25 units of fresh frozen plasma |
| Bone Marrow Transplant | 120 units of platelets |
| | 20 units of blood |

In various embodiments, the pharmaceutical composition comprises a dose from about 40 g of Mb/unit of solution to about 80 g of Mb/unit of solution. In various embodiments, the pharmaceutical composition comprises a dose from about 50 g of Mb/unit of solution to about 80 g of Mb/unit of solution. In various embodiments, the pharmaceutical composition comprises a dose from about 60 g of Mb/unit of solution to about 80 g of Mb/unit of solution. In various embodiments, the pharmaceutical composition comprises a dose from about 70 g of Mb/unit of solution to about 80 g of Mb/unit of solution. In various embodiments, the pharmaceutical composition comprises a dose from about 60 g of Mb/unit of solution to about 70 g of Mb/unit of solution.

The dose of the pharmaceutical composition of the various embodiments may also be measured in grams of polymersome administered per kg of a subject. In various embodiments, the total dose comprises from about 12.5 mg of polymer to about 90 mg of polymer per kg of a subject. In various embodiments, the total dose comprises from about 15 mg of polymer to about 90 mg of polymer per kg of a subject. In various embodiments, the total dose comprises from about 25 mg of polymer to about 90 mg of polymer per kg of a subject. In various embodiments, the total dose comprises from about 35 mg of polymer to about 90 mg of polymer per kg of a subject. In various embodiments, the total dose comprises from about 45 mg of polymer to about 90 mg of polymer per kg of a subject. In various embodiments, the total dose comprises from about 55 mg of polymer to about 90 mg of polymer per kg of a subject. In various embodiments, the total dose comprises from about 65 mg of polymer to about 90 mg of polymer per kg of a subject. In various embodiments, the total dose comprises from about 75 mg of polymer to about 90 mg of polymer per kg of a subject. In various embodiments, the total dose comprises from about 80 mg of polymer to about 90 mg of polymer per kg of a subject. In various embodiments, the total dose comprises from about 85 mg of polymer to about 90 mg of polymer per kg of a subject.

In various embodiments, the pharmaceutical composition is a liquid formation that comprises an allosteric effector such as 2,3-Bisphosphoglycerate, wherein the formulation comprises from about 1 to about 100 mmol/L of formulation. In various embodiments, the formulation comprises from about 1 to about 100 mmol of a isomer of 2,3-Bisphosphoglycerate per L of formulation. In various embodiments, the formulation comprises from about 1 to about 10 mmol of a isomer of 2,3Bisphosphoglycerate per L of formulation. In various embodiments, the formulation comprises about 5 mmol of 2,3-Bisphosphoglycerate or isomer derived thereof per L of formulation. In various embodiments, the formulation comprises about 2.25 mmol of 2,3-Bisphosphoglycerate or isomer derived thereof per Unit (450 mL) of formulation.

The pharmaceutical compositions may be prepared, packaged, or sold in the form of a sterile, injectable, aqueous or oily suspension or solution. This suspension or solution may be formulated according to the known art, and may comprise, in addition to myoglobin, additional ingredients such as the dispersing agents, wetting agents, or suspending agents described herein. Such sterile injectable formulations may be prepared using a nontoxic parenterally acceptable diluent or solvent, such as water or 1,3 butane diol, for example. Other acceptable diluents and solvents include, but are not limited to, Ringer's solution, isotonic sodium chloride solution, and fixed oils such as synthetic mono or di-glycerides. Other parentally-administrable formulations which are useful include those which comprise myoglobin in a liposomal preparation, or as a component of biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The formulations described herein, are also useful for pulmonary delivery and the treatment of such cancers of the respiratory system or lung, are also useful for intranasal delivery of a pharmaceutical composition of the various embodiments. Such formulation suitable for intranasal administration is a coarse powder comprising myoglobin and having an average particle from about 0.2 to 500 micrometers, administered by rapid inhalation through the nasal passage from a container of the powder held close to the nares.

The various embodiment pharmaceutical compositions may be administered to deliver a dose of from about 0.1 g/kg/day to about 100 g/kg/day, where the gram measurement is equal to the total weight of Mb and polymer in the pharmaceutical composition. In various embodiments, the dosage is from about 0.1 to 1 g/kg/day. In another embodiment, the dosage is from about 0.5 g/kg/day to about 1.0 g/kg/day. In another embodiment, the dosage is from about 1.0 g/kg/day to about 1.5 g/kg/day. In another embodiment, the dosage is from about 1.5 g/kg/day to about 2.0 g/kg/day. In another embodiment, the dosage is from about 2.5 g/kg/day to about 3.0 g/kg/day. In another embodiment, the dosage is 1.0, 2.0, 5.0, 10, 15, 20, 25, 30, 35, 40, 45, or 50 g/kg/day, where the gram measurement is equal to the total weight of Mb and polymer in the pharmaceutical composition. In one embodiment, administration of a dose may result in a therapeutically effective concentration of myoglobin between 1 µM and 10 µM in a diseased or cancer-affected tissue, or tumor of a mammal when analyzed *in vivo.*

In an embodiment, a pharmaceutical composition, especially one used for prophylactic purposes, can comprise, in addition, a pharmaceutically acceptable adjuvant filler or the like. Suitable pharmaceutically acceptable carriers are well known in the art. Examples of typical carriers include saline, buffered saline and other salts, lipids, and surfactants. The oxygen carrier or polymersome may also be lyophilized and administered in the forms of a powder. Taking appropriate precautions not to denature any protein component disclosed herein, the preparations can be sterilized and if desired mixed with auxiliary agents, e.g., lubricants, preservatives, stabilizers, wetting agents, emulsifiers, salts for influencing osmotic pressure, buffers, and the like that do not deleteriously react with the oxygen carrier or polymersome discussed herein. They also can be combined where desired with other biologically active agents, e.g., antisense DNA or mRNA.

A pharmaceutical composition of the various embodiments may be prepared, packaged, or sold in bulk, as a single unit dose, or as a plurality of single unit doses. The amount of myoglobin is generally equal to the dosage of myoglobin which would be administered to a subject, or a convenient fraction of such a dosage, such as, for example, one-half or one-third of such a dosage, as would be known in the art.

The relative amounts of myoglobin, the pharmaceutically acceptable carrier, and any additional ingredients in a pharmaceutical composition of the various embodiments may vary, depending upon the identity, size, and condition of the subject to be treated and further depending upon the route by which the composition is to be administered. By way of example, the composition may comprise from about 0.1% to about 100% (w/w) active ingredient.

The compositions and methods described herein may be useful for the treatment of cancer in conjunction with radiation therapy.

The compositions and methods described herein can be useful for preventing the dissemination or improving the chemotherapy and/or radiation therapy of cancers including leukemias, lymphomas, meningiomas, mixed tumors of salivary glands, adenomas, carcinomas, adenocarcinomas, sarcomas, dysgerminomas, retinoblastomas, Wilms' tumors, neuroblastomas, melanomas, and mesotheliomas; as represented by a number of types of cancers, including but not limited to breast cancer, sarcomas and other neoplasms, bladder cancer, colon cancer, lung cancer, pancreatic cancer, gastric cancer, cervical cancer, ovarian cancer, brain cancers, various leukemias and lymphomas. One would expect that any other human tumor cell, regardless of expression of functional p53, would be subject to treatment or prevention by the methods discussed herein, although the particular emphasis is on mammary cells and mammary tumors.

Also useful in conjunction with the methods provided in the various embodiments may be chemotherapy, phototherapy, anti-angiogenic or irradiation therapies, separately or combined, which may be used before, contemporaneously, or after the enhanced treatments discussed here, but will be most effectively used after the cells have been sensitized by the present methods. As used herein, the phrase "chemotherapeutic agent" means any chemical agent or drug used in chemotherapy treatment, which selectively affects tumor cells, including but not limited to, such agents as adriamycin, actinomycin D, camptothecin, colchicine, taxol, cisplatinum, vincristine, vinblastine, and methotrexate. Other such agents are well known in the art.

The various embodiments may also include kits comprising any of the aforementioned compositions or pharmaceutical compositions comprising an oxygen carrier or a polymersome, wherein the oxygen carrier or a polymersome comprises myoglobin, at least one biocompatible polymer and at least one biodegradable polymer. According to various embodiments, the formulation may be supplied as part of a kit. The kit may comprise the pharmaceutical composition comprising an oxygen carrier or a polymersome. In another embodiment, the kit may comprise a lyophilized oxygen carrier or polymersome with an aqueous rehydration mixture. In another embodiment, the oxygen carrier or polymersome may be in one container while the rehydration mixture is in a second container. The rehydration mixture may be supplied in dry form, to which water may be added to form a rehydration solution prior to administration by mouth, venous puncture, injection, or any other mode of delivery. In various embodiments, the kit may further comprise a vehicle for administration of the composition such as tubing, a catheter, syringe, needle, and/or combination of any of the foregoing.

The various embodiments may be illustrated, but are not limited to, the following examples

### Example I

### Methods and materials to construct biodegradable PEM dispersions with varying physicochemical properties:

Poly(ethyleneoxide)-block-poly(ε-caprolactone) (PEO-b-PCL) possessing a PEO block size of -1.5-4kDa and with a PEO block fraction of -10-20% by weight are utilized to form biodegradable PEM dispersions. Poly(ethylene oxide)-block-poly(y-methyl ε-caprolactone) (PEO-b-PMCL) and Poly(ethylene oxide)-block-poly(trimethylcarbonate) (PEO-b-PTMC) copolymers of varying molecular weight, hydrophobic-to-hydrophilic block fraction, and resulting polymersomemembrane-core thickness are further incorporated to generate PEM constructs that are not only slowly biodegradable but also uniquely deformable, enabling passage through compromised capillary beds, via infra. PMCL, as a derivative of PCL, is a similarly fully bioresorbable polymer that degrades via non-enzymatic cleavage of its ester linkages. Polymersomes composed from PEO-b-PTMC and/or PEO-b-PMCL are spontaneously formed at lower temperatures, in greater yields, and possess more deformable and viscoelastic membranes as compared to those composed from PEO-b-PCL. They also similarly degrade much more slowly than vesicles formed from PEO-b-PGA, PEO-b-PLA, or PEO-b-PLGA. As such, PEO-b-PCL and PEO-b-PMCL-derived PEM dispersions demonstrate larger Mb-encapsulation efficiencies, smaller average particle diameters, and lower levels of metMb generation as compared to biodegradable cellular MBOCs claimed in the literature.

### Synthesis of PEM dispersions:

To synthesize PEM dispersions, Purified human Mb may be purchased from Sigma-Aldrich® to be used as starting materials. PEO-b-PCL, PEO-b-PMCL, and PEO-b-PTMC copolymers with PEO molecular weight ranging from 1kDa-4kDa have previously been shown to give a stable and high yield of polymersomes⁵³. For example, the PEO may have a molecular weight of 2kDa and the PMCL may have a molecular weight of 9.4kDa. By varying the initial amounts of polymer (from 5 mg - 20 mg per sample), as well as the initial Mb concentrations used in polymersome formation (from 100 mg/ml to 300 mg/ml), PEM dispersions that differ in the degree of Mb encapsulation are generated.

PEM dispersions will be formed by using three different methodologies: 1) "thin-film rehydration", which involves the deposition of an organic solution of dissolved polymer on a Teflon film, drying of the film under vaccum oven overnight to remove all organic solvent, immersion of the dry thin-film of polymer in an aqueous solution of purified Mb and subsequent high-frequency sonication with heat, and, finally, extruding through a series of different pore-size membranes in order to yield the desired nanometric PEM dispersion; 2) "direct hydration",^{116, 117} where dry polymer is mixed with an equal weight of PEG 500 DME at a 1:1 molar ratio, heated to 95 °C for 30 minutes, mixed vigorously, allowed to cool to room temperature for 20 minutes prior to addition of Mb solution, then followed by further vigorous mixing and sonication, extrusion through a series of different pore-size membranes in order to yield the desired nanometric PEM dispersion, and finally, by separation of PEG 500 by running on a size-exclusion column; and 3) thin-film direct hydration, where dry polymer is mixed with an equal weight of PEG 500 DME at a 1:1 molar ratio prior to deposition on Teflon, followed by then drying of this film over night, heating to 95 °C for 30 minutes, vigorously mixing, allowing to cool to room temperature for 20 minutes prior to addition of Mb solution, further vigorous mixing and sonication, extrusion through a series of different pore-size membranes in order to yield the desired nanometric PEM dispersion, and finally, by separation of PEG 500 by running on a size-exclusion column..

Each of these methods produces a high yield of stable polymersomes that can be effectively controlled through membrane extrusion to yield unilamellar, mono-dispersed suspensions of PEMs that vary from 100 nm - 1 µm in diameter in average size. Although thin-film rehydration may yields very narrow PEM size distribution, and relatively higher Mb encapsulation % due to larger core volumes available for encapsulation,¹¹⁶ the stability of Mb and the resultant PEM dispersions can be demonstrably lower;¹⁶³ these results may be due to the fact that the hydration and optimal sonication temperatures necessary for generating a given polymeric-composition of polymersomes may be close to the denaturation temperature of free Mb (e.g. 60 °C used to generate PEO-b-PCL-based PEM dispersions via thin-film rehydration). PEO-b-PMCL and PEO-b-PTMC polymersomes will be formed by direct or thin-film direct hydration at room temperature (under ambient pO₂) and expectedly enable a higher yield of PEMs with greater Mb encapsulation efficiency. For concomitant NIR imaging studies, NIR-emissive PEM constructs may be generated via co-incorporation of oligo(porphyrin)-based NIRFs with dried polymer (at a mol ratio of 1:40),¹⁶⁶ prior to exposure to the aqueous Mb solution. Unencapsulated Mb are separated from all PEM dispersions using dialysis, ultra-filtration, and/or size exclusion chromatography.

### Example II

### Characterization of physicochemical properties of PEM dispersions:

To verify PEM generation, each Mb/polymer formulation are characterized for particle size distribution using dynamic light scattering (DLS). PEM structure and morphology are directly visualized using cryogenic transmission electron microscopy (cryo-TEM). The viscosity of the various PEM dispersions are measured using a microviscometer. To measure Mb encapsulation%, two independent methods are used. In the first method, PEM dispersions are initially lysed with a detergent (e.g. triton X-100) and the UV absorbance of the resulting lysate is measured to determine the mass of Mb and subsequent Mb encapsulation% of the original PEM composition.¹⁶² While this calculation is relatively straight forward, it may overestimate the encapsulation% through some assumptions on total Mb dispersion volume. As such, an asymmetric field-flow fractionator coupled with a differential interferometric refractometer is used to measure the concentration of eluting, unencapsulated Mb from the encapsulation% is determined.^{162, 176} From these measurements, the final weight ratio of Mb:polymer in the various PEM dispersions is further calculated. The % metMb in each of the PEM dispersions is determined by analogous methodology to the well-established cyanometMb assay.^{162, 163}

### Example III

### Characterization of the oxygen-carrying properties of biodegradable PEM dispersions:

The oxygen binding properties of PEO-b-PCL and PEO-b-PMCL-based PEM dispersions are measured using established techniques. The equilibrium oxygen binding properties are thoroughly characterized as well as the diffusion kinetics of oxygen across polymersome membranes. With the aid of these measurements, oxygen permeabilities and oxygen-membrane diffusion coefficients for these various PEM dispersions are determined. These very fundamental parameters are critical for the optimal design of a successful cellular MBOC. Nitric oxide (NO) binding profiles of various PEO-b-PCL and PEO-bPMCL-based PEM dispersions are further determined. Acellular MBOCs can be expected to induce vasoconstriction, hypertension, reduced blood flow, and vascular damage in animals due to their entrapment of endothelium-derived NO. Mb-encapsulated in nanoparticles such as polymersomes, liposomes, micelles, etc, however, is not been expected to be similarly "vasoactive"; analogous to those of natural RBCs, liposome and polymersome membranes should effectively retard NO binding through effective Mb sequestration from the surrounding vascular environment. PEM dispersions will likely exhibit more resistance to NO scavenging owing to their thicker membranes and lower permeabilities. Finally, different measurements on PEO-b-PCL and PEO-b-PMCL-based PEM dispersions will be performed in order to test their stability and integrity under physiological conditions for extended durations of time.

### Experimental:

### Characterization of oxygen binding properties:

Equilibrium oxygen binding properties such as P₅₀ of PEO-b-PCL, PEO-b-PMCL-and PEO-b-PTMC-based PEM dispersions are measured using a Hemox-analyzer^{51, 52}. Dependence of these properties on the composition of PEM dispersions are determined using a series of Mb-loading concentrations, as well as by adding an allosteric effector such as inositol hexaphosphate into the aqueous phase of the polymersomes. This is especially important in order to determine the suitability of PEO-b-PCL, PEO-b-PMCL, and PEO-b-PTMC-based PEM constructs to deliver oxygen to tissues experiencing normal oxygenation as well as in low oxygenation conditions. Results of these experiments will be compared with respect to P₅₀ and n values of free Mb solution, as well as those values of Oxyglobin® (Biopure Corp., Cambridge, MA), which is the only oxygen therapeutic approved by the FDA for veterinary use.

In addition to these equilibrium measurements, the kinetics of oxygen diffusion across PEM membranes and binding to/release of Mb for different PEO-b-PCL, PEO-b-PMCL, and PEO-b-PTMC-based PEM dispersions are determined using a highly sensitive oxygen microelectrode. Measurements of various PEMs are compared to those from free Mb and empty polymersome dispersions (without Mb) in order to delineate the roles of diffusion and binding in O₂ take-up. The results of these experiments are analyzed with the help of a diffusion-reaction transport model to determine oxygen permeability of different polymersome membranes; a correlation between diffusive properties of various diblock copolymer membranes and measured oxygen binding properties of PEM formulations is expected.

### Characterization of NO binding properties:

NO binding of PEO-b-PCL, PEO-b-PMCL, and PEO-b-PTMC-based PEM dispersions under oxygenated and deoxygenated conditions are systematically studied using stopped flow spectroscopy.^{177, 178} The time-course of binding is measured by taking rapid absorbance scans of the various oxygenated or deoxygenated PEM dispersions rapidly mixed with NO-containing solution. A range of Mb loading concentrations, PEM dispersion concentrations, and PEM sizes are expected to alter the results of these experiments. Similarly, the roles of NO diffusion and binding in NO uptake by PEO-b-PCL, PEO-b-PMCL, and PEO-b-PTMC-based PEM constructs are further characterized by conducting experiments comparing PEM, free Mb, and empty polymersomes using a NO microelectrode. Through these comprehensive studies, the NO binding rate constants for PEO-b-PCL, PEO-b-PMCL, and PEO-b-PTMC-based PEM dispersions under different conditions are established and compared with the results for free Mb in solution, liposome encapsulated Mb (LEM), and Oxyglobin®.

### Characterization of the stability and integrity of PEM dispersions:

To test the stability of various PEO-b-PCL, PEO-b-PMCL, and PEO-b-PTMC-based PEM dispersions, they are stored in saline solution and in blood plasma at 4 °C and at 37 °C for several days; changes in PEM morphology and size distribution are assessed using cryo-TEM and DLS, respectively. Similarly, in situ changes in Mb concentration, metMb level, NO uptake, and Mb release from biodegradable PEMs under various solution conditions (e.g. temperature, pH, pO₂, and pNO) and at various time points is tested using techniques described herein. These studies utilize electronic absorption spectroscopy and concentration calculations based on known extinction coefficients for methylated, NO-bound, and oxygenated Mb.¹⁷⁹⁻¹⁸⁸

### Measurement of critical lysis tension, critical areal strain using micropipette aspiration:

Micropipet aspiration of Mb-encapsulating polymersomes follows analogous procedures to those described in previous references. Briefly, micropipets made of borosilicate glass tubing (Friedrich and Dimmock, Milville, NJ) are prepared using a needle/pipette puller (model 730, David Kopf Instruments, Tujunga, CA) and microforged using a glass bead to give the tip a smooth and flat edge. The inner diameters of the micropipets range from 1 um to 6 um and are measured using computer imaging software. The pipettes are used to pick up the Mb-loaded and unloaded polymersomes and apply tension to their membranes. Micropipets are filled with PBS solution and connected to an aspiration station mounted on the side of a Zeiss inverted microscope, equipped with a manometer, Validyne pressure transducer (models DP 15-32 and DP 103-14, Validyne Engineering Corp., Northridge, CA), digital pressure read-outs, micromanipulators (model WR-6, Narishige, Tokyo, Japan), and MellesGriot millimanipulators (course x,y,z control). Suction pressure is applied via a syringe connected to the manometer. Experiments are performed in PBS solutions that has osmolalities of 310-320 mOsm in order to make the polymersomes flaccid (internal vesicle solution was typically 290-300 mOsm sucrose). The osmolalities of the solutions are measured using an osmometer. Since sucrose and PBS have different densities and refractive indices, the polymersomes settle in solution and are readily visible under phase contrast or DIC optics.

### Example IV

### Development of PEO-b-PCL, PEO-b-PMCL, and PEO-b-PTMC-based PEM dispersions that are capable of dry storage, point-of-care rehydration, and in vivo delivery:

### Polymer Synthesis:

Acrylate-modified diblock copolymers (e.g. an acryl modified PEO-b-PCL-based polymer deemed PEO-b-PCL-acryl) are synthesized according to standard procedures using stannous octoate as the catalyst. For example, PEO-b-PCL-acryl is found to have a number average molecular weight of 14 kDa (12 and 2 kDa for the PCL and PEO blocks, respectively). These are determined by calibrating the NMR peaks to the terminal methoxy group on the PEO at approximately 3.4 ppm. The polydispersity of the polymer is < 1.5. Acrylation of the OH terminus of the PCL block does not lead to a significant change in the polymer size or distribution following the second purification. The acrylation efficiency has been found to be 99%.

### Formation of PEM Dispersions:

To synthesize PEM dispersions comprised of acryl-modified polymers (e.g. PEO-b-PCL-acryl-based PEM dispersions), pure human Mb is used as starting materials. Pure human Mb may be purchased from Sigma-Aldrich®. PEO(2k)-b-PCL(12k)-acryl polymer and 2,2dimethoxy-2-phenylacetophenone (DMPA) are dried on roughened Teflon® via dissolution in methylene chloride at a molar ratio of 1:1, deposition on Teflon®, and evaporation of the organic solvent. Varying the amount of acryl-modified polymer (e.g. PEO(2k)-b-PCL(12k)-acryl polymer, from 5 mg - 20 mg per sample), as well as the initial aqueous Mb concentrations used in polymersome formation (from 100 mg/ml to 300 mg/ml), PEM dispersions that compartmentalize DMPA in their membranes and that differ in the degree of aqueous Mb encapsulation are generated. PEM dispersions are formed by using three well-established methodologies: 1) thin-film rehydration, 2) direct hydration, and 3) thin-film direct hydration (see Example I). Each of these methods produces a high yield of stable polymersomes that can be effectively controlled through membrane extrusion to yield unilamellar, mono-dispersed suspensions of PEMs that vary from 100 nm - 1 µm in diameter in average size. Although thin-film rehydration may yields very narrow PEM size distribution, and possibly higher Mb encapsulation % due to larger core volumes available for encapsulation,¹¹⁶ the stability of Mb and the resultant PEO-b-PCL-based PEM dispersions can be demonstrably low;¹⁶³ these results may be due to the fact that the hydration temperature for PEO-b-PCL is close to the denaturation temperature of free Mb. PEO-b-PMCL and PEO-b-PTMC polymersomes are formed by direct hydration or thin-film direct hydration at room temperature (under ambient pO₂) and expectedly enable a higher yield of PEMs with greater Mb encapsulation efficiency. For concomitant NIR imaging studies, NIR-emissive PEM constructs are generated via co-incorporation of oligo(porphyrin)-based NIRFs with dried polymer (at a mol ratio of 1:40),¹⁶⁶ prior to exposure to the aqueous Mb solution. Unencapsulated Mb is separated from all PEM dispersions using dialysis, ultra-filtration, or size exclusion chromatography.

### Stabilization of PEM Membranes After Formation:

Once assembled, acryl-modified polymersomes comprising the membranes of the PEM dispersions (e.g. PEO-b-PCL-acryl) can be crosslinked via UV light exposure that induces a radical polymerization of the acryl groups via activation of the photoinitator DMPA incorporated in the polymersome membranes. This approach does not hinder hydrolysis of the biodegradable block (e.g. the PCL chain of PEO-b-PCL-acryl) and yields degraded monomers (e.g. oligo-caprolactone units), PEO, and kinetic chains of poly(acrylic acid) as the degradation products. Mb is protected from photo-induced degradation of metMb formation by co-ecapsulation of NAC or methylene blue with Mb within the polymersomess' aqueous core. Polymerization of the vescicles' membranes proceeds by exposure of the DMPA-incorporated acryl-modified polymers (e.g. PEO-b-PCL-acryl) that compose thePEM dispersions using UV light generated from an OmniCure Series 1000 spot-curing lamp with a collimating lens (Exfo, Ontario, Canada; 365 nm, 55 mW/cm2) for 10-30 min.

### Lyophilization and Dry-phase Storage:

Lyophilization proceeds by freeze-drying the acryl-modified PEM dispersions (e.g. PEO-b-PCL acryl PEM) after UV light exposure by placement in liquid nitrogen until bubbling ceases. The frozen PEM dispersions are then placed on a benchtop lyophilizer (FreeZone 4.5 L Benchtop Freeze Dry System, Labconco, Kansas City, MO; Model 77500) for 24 h until samples are dry. The dry, collapsed PEM dispersions are then stored in a dessicator under argon gas and placed at 4 °C.

### Point-of-Care Hydration:

The dried acry-modified PEM dispersions are taken out of the dessicator and placed in a vial. The same original volume of aqueous solution is added back to the samples to hydrate the vesicles. Polymersome rehydration is further augmented by gentle vortexing for 10 minutes to achieve full vesicle resuspension. Intact polymersomes are verified by DLS, which shows minimal vesicle aggregation and no destruction into micelles. Mb retention is verified by running the PEM dispersion over an aqueous size-exclusion column and taking aliquots of the running bands for UV-vis analysis. Only bands corresponding to polymersomes, as verified further by DLS of the elution aliquots, contain Mb as assessed by UV-vis spectroscopy. The stability of the retained Mb is further verified by the UV-vis spectra that show no bands corresponding to metMb generation or any further Mb breakdown products.

### Development of Molecularly-targeted PEO-b-PCL, PEO-b-PMCL, and PEO-b-PTMC-based PEM Dispersions:

Through well-established chemical conjugation methods, polymersome surfaces are modified with various biological ligands to impart specific multi-avidity biological adhesion. Similar methodology may are adopted to generate molecularly-and cellular-targeted polymersome-encapsulated PEM dispersions that are able to promote, amongst other things, wound healing and improved efficacy of radiation therapy to hypoxia tissues. Biological ligands are conjugated to these nanoparticles via a carbodiimide- poly-vinyl sulfone-mediated aqueous phase reactions. The degree of polymersome-surface coverage with ligand is systematically varied (from 1% to > 10% of the total surface area of the polymersomes) by using ligands of different concentrations and PEM dispersions that are synthesized from mixtures containing different ratios of functionalized to unfunctionalized polymers. After verifying peptide conjugation to polymersome surfaces, the kinetic binding of the resultant PEM formulations to recombinant molecular targets/receptors are characterized via surface plasmon resonance (Biacore SPR) measurements; dose-dependent curves are analyzed in a manner similar to that described for the free biological ligand. These studies reveal kinetic parameters of the interaction between PEM dispersions and molecular targets (on-rate, *kon* and off-rate, *koff*) and the change in affinity of ligands (dissociation constant, *K*) as affected by their conjugation to polymersomes.

### Experimental:

Established chemical modification procedures are used to functionalize the PEO terminus of biodegradable polymers (e.g. PEO-b-PCL diblock copolymers) with carboxyl groups and to verify the reactions by 1H NMR spectroscopy. PEM dispersions are created and purified from various combinations of functionalized and unfunctionalized copolymers using standard separation methods to yield mono-dispersed suspensions of unilamellar vesicles that are stable for several months. PEM size distributions are determined by dynamic light scattering (DLS). Ligand identity and purity are confirmed by reverse phase high performance liquid chromatography and MALDI mass spectrometry. Ligand conjugation to carboxyl-terminated PEO groups on the polymersome surface is carried in an aqueous reaction mediated by 1-ethyl-3-(3-dimethylaminopropyl) carbodiimide (EDC) and Nhydroxysuccinimide (NHS). The extent of ligand conjugation is determined using a micro-BCA assay. The resultant targeted PEM dispersions are extensively imaged by cryogenic transmission electron microscopy (cryo-TEM) to verify their stability after ligand conjugation. Their size distributions are again measured by DLS. The degree of ligand conjugation is verified using flow cytometry. SPR measurements are carried out on biosensor instruments Biacore X and Biacore 2000 (Biacore AG, Uppsala, Sweden) at 25°C. Recombinant purified recombinant ligand targets (e.g. protein receptors) are purchased commercially and immobilized by attachment to the dextran hydrogel on the sensor surface. Targeted PEM constructs are injected in various concentrations and their binding is monitored in real time. The kinetic rate constants (kₒₙ and k_{off}) and the equilibrium binding constant (KD) for receptor/PEM binding are estimated from kinetic analysis of the sensorgrams. PEMs without targeting ligands or irrelevant ligand-conjugated PEMs are used as controls.

Alternative ligand conjugation chemistries can also be employed. For example, organic phase reactions where the diblock polymer is chemically functionalized and conjugated with select ligands (small molecules, peptides that have organic-phase solubility) prior to forming PEM dispersions are possible; this organic coupling methods ensures that the PEO terminus is conjugated with ligand before it is exposed to aqueous solution where it might lose many of its modified surface reactive groups via competing hydrolysis. Also, as an alternative method to vary the degree of ligand surface conjugation, PEM dispersions composed of PEO-b-PCL copolymers that vary with respect to PEO and PCL block sizes are created. This approach controls the kinetics of ligand conjugation to polymersome surfaces as well as the degree of ligand surface coverage for a given PEM formulation. It is possible for targeted PEM formulations to bind to the sensor surface in a non-specific manner during SPR measurements, thereby affecting its regeneration and signal-to-background ratio. If a reliable measurement cannot be performed, ligand-conjugated PEM binding characteristics are also studied using ELISA or isothermal titration calorimetry, which are other established techniques for studying nanoparticle binding.

### In Vivo Tumor Oxygenation Modulation by PEM:

PEM dispersions formed from PEO-b-PCL, PEO-b-PMCL, PEO-b-PTMC and/or acryl-modified versions of these polymers are tested for their abilities to alter *in vivo* tumor oxygenation upon tail-vein injection into xenotransplanted-tumor bearing mice. The co-dependent effects of particle size, deformability and concentration on effective Mb delivery, and resultant tumor oxygenation, are also deconstructed. A hyperspectral optical imaging system that can spatially deconstruct real-time kinetic O₂ transport is used to assess the efficacy of a given PEM construct to alter mean and minimum tumor oxygen tensions (pO₂).^{151, 154, 156, 158} While mean pO₂s have been previously studied and are readily measured by other techniques, the spatially-distributed minimum tumor pO₂s are perhaps the most responsible for driving tumorigenesis and providing the cancer stem cell niche that helps tumors evade effective treatment.^{151, 154, 155, 189} A hyperspectral optical imaging system enables the spatial mapping of kinetic pO₂s, in real-time, and is used to visualize and quantify the degree of PEM-modulation of low tumor pO₂ areas. In addition, mild localized tumor heating increases vessel pore sizes in solid tumors for up to several hours, aiding in nanoparticle extravasation.^{190, 191} As such, localized tumor hyperthermia is further capable of increasing O₂ delivery by PEMs. Finally, PEM-related myoglobinuria and its effects on creatinine clearance (CCr) is monitored to assess acute post-treatment nephrotoxicity.

### Animal Storage, Handling and PEM Injections:

Mice are purchased and housed in appropriate animal facilities. Dorsal skin fold window chambers are surgically implanted on each animal approximately 1 week prior to treatment. During the surgical procedure, 10,000 4T1 mammary carcinoma cells are injected onto the mouse dorsum or flank. These cells are engineered to constitutively express RFP, with GFP expression induced in response to HIF-1 activity.¹⁹² The tumors are allowed to grow for approximately 1 week, at which point they are large enough to be hypoxic and have HIF-1 activity.¹⁵⁸ 100 µL of PEM suspensions are prepared as described above and injected via the tail vein at *t* = 0 and *t* = 24 h. These experimental parameters enable evaluation of the effects of PEMs that have accumulated in the perivascular space, which is expected to peak at approximately 24 h. For hyperthermia studies, a special housing unit is used to vary the window chamber/tumor temperature.^{190, 191}

### Visualization and Quantification of Kinetic Tumor Oxygen Modulation by PEM:

At the time points specified above, hyperspectral imaging is used to evaluate the effects of the various PEM constructs on modifying tumor pO₂. Temperatures are adjusted between 34 - 42 °C.^{190, 191} Hyperspectral imaging of Mb absorption is used to quantify Mb O2 saturation,¹⁵⁸ while ratiometric evaluation of boron nanoparticle fluorescence and phosphorescence is used to quantify absolute tumor pO₂.¹⁵³ HIF-1 activity is also evaluated by measuring GFP emission. This enables quantification of both vascular and tissue oxygenation, as well as the presence of the tumor hypoxic phenotype, independently and concurrently.

## Claims

1. A high-oxygen affinity agent for use in a method of increasing efficacy of radiation or chemotherapy applied to a tumor in a subject, said method comprising:
delivering said high-oxygen affinity agent to the tumor by administering to the subject an oxygen carrier that incorporates the high-oxygen affinity agent, wherein:
the high-oxygen affinity agent comprises a myoglobin composition; and
oxygen partial pressure gradient characteristics of the high-oxygen affinity agent are such that oxygen is released from the high-oxygen affinity agent at oxygen tensions of less than 10 mmHg, wherein the released oxygen diffuses out of the oxygen carrier while the high-oxygen affinity agent remains within the oxygen carrier.

2. The high-oxygen affinity agent for use according to claim 1, wherein said high-oxygen affinity agent has a P50 for oxygen of around 2-3 mmHg.

3. The high-oxygen affinity agent for use according to claim 2, wherein said high-oxygen affinity agent comprises one or more of unmodified human myoglobin, unmodified myoglobin from another biological species and chemically or genetically modified myoglobin from humans or from another biological species.

4. The high-oxygen affinity agent for use according to claim 2, wherein said high-oxygen affinity agent is a cooperative oxygen binder or a linear oxygen binder.

5. The high-oxygen affinity agent for use according to claim 2, wherein oxygen partial pressure gradient characteristics of said high-oxygen affinity agent are such that oxygen is tightly bound by the high-oxygen affinity agent while circulating in a bloodstream.

6. The high-oxygen affinity agent for use according to claim 1, wherein said high-oxygen affinity agent is PEGylated or polymerized.

7. The high-oxygen affinity agent for use according to claim 1, wherein said high-oxygen affinity agent is encapsulated in the oxygen carrier, and wherein the oxygen carrier comprises a
carrier vehicle that protects the high-oxygen affinity agent from being released into a bloodstream.

8. The high-oxygen affinity agent for use according to claim 1, wherein said high-oxygen affinity agent is co-encapsulated in a carrier vehicle with at least one other radiation-sensitizing or chemotherapeutic agent.

9. The high-oxygen affinity agent for use according to claim 7, wherein the carrier vehicle is selected from one of a nanoparticle-based vehicle, a lipid vesicle, a synthetic polymer vesicle, or a uni- or
multi-lamellar polymersome.

10. The high-oxygen affinity agent for use according to claim 7, wherein the carrier vehicle is a vesicle, and wherein the high-oxygen affinity agent is within an aqueous core or a membranous portion of the vesicle.

11. The high-oxygen affinity agent for use according to claim 7, wherein the carrier vehicle comprises a plurality of biodegradable polymers.

12. A kit, comprising:
a first container; and
a second container;
wherein the first container comprises the high-oxygen affinity agent of claim 1 and wherein the second container comprises a rehydration mixture.

13. The high-oxygen affinity agent for use according to claim 1, wherein administering to the subject the oxygen carrier that incorporates the high-oxygen affinity agent comprises administering the oxygen carrier intravenously, via inhalation, topically, per rectum, per the vagina, transdermally, subcutaneously, intraperitoneally, intrathecally, intramuscularly, or orally.

## Patentansprüche

1. Mittel mit hoher Sauerstoffaffinität für die Verwendung in einem Verfahren zur Erhöhung der Wirksamkeit von Bestrahlung oder Chemotherapie, die auf einen Tumor bei einem Subjekt angewendet wird, wobei das Verfahren Folgendes umfasst:
Abgeben des Mittels mit hoher Sauerstoffaffinität an den Tumor durch Verabreichung eines Sauerstoffträgers, der das Mittel mit hoher Sauerstoffaffinität enthält, an das Subjekt, wobei:
das Mittel mit hoher Sauerstoffaffinität eine Myoglobinzusammensetzung umfasst; und
Eigenschaften des Sauerstoffpartialdruckgefälles des Mittels mit hoher Sauerstoffaffinität derart sind, dass Sauerstoff aus dem Mittel mit hoher Sauerstoffaffinität bei Sauerstoffspannungen von kleiner als 10 mmHg freigesetzt wird, wobei der freigesetzte Sauerstoff aus dem Sauerstoffträger diffundiert, während das Mittel mit hoher Sauerstoffaffinität innerhalb des Sauerstoffträgers verbleibt.

2. Mittel mit hoher Sauerstoffaffinität für die Verwendung nach Anspruch 1, wobei das Mittel mit hoher Sauerstoffaffinität einen P50 für Sauerstoff von etwa 2-3 mmHg aufweist.

3. Mittel mit hoher Sauerstoffaffinität für die Verwendung nach Anspruch 2, wobei das Mittel mit hoher Sauerstoffaffinität ein oder mehrere von nicht modifiziertem menschlichem Myoglobin, nicht modifiziertem Myoglobin von einer anderen biologischen Spezies und chemisch oder genetisch modifiziertem Myoglobin von Menschen oder von einer anderen biologischen Spezies umfasst.

4. Mittel mit hoher Sauerstoffaffinität für die Verwendung nach Anspruch 2, wobei das Mittel mit hoher Sauerstoffaffinität ein kooperatives Sauerstoffbindemittel oder ein lineares Sauerstoffbindemittel ist.

5. Mittel mit hoher Sauerstoffaffinität für die Verwendung nach Anspruch 2, wobei die Eigenschaften des Sauerstoffpartialdruckgefälles des Mittels mit hoher Sauerstoffaffinität derart sind, dass Sauerstoff von dem Mittel mit hoher Sauerstoffaffinität während der Zirkulation in einem Blutstrom fest gebunden wird.

6. Mittel mit hoher Sauerstoffaffinität für die Verwendung nach Anspruch 1, wobei das Mittel mit hoher Sauerstoffaffinität PEGyliert oder polymerisiert ist.

7. Mittel mit hoher Sauerstoffaffinität für die Verwendung nach Anspruch 1, wobei das Mittel mit hoher Sauerstoffaffinität in dem Sauerstoffträger eingekapselt ist und wobei der Sauerstoffträger ein Trägervehikel umfasst, das das Mittel mit hoher Sauerstoffaffinität vor der Freisetzung in einen Blutstrom schützt.

8. Mittel mit hoher Sauerstoffaffinität für die Verwendung nach Anspruch 1, wobei das Mittel mit hoher Sauerstoffaffinität in einem Trägervehikel mit mindestens einem anderen Strahlungssensibilisator oder Chemotherapeutikum gleichzeitig eingekapselt ist.

9. Mittel mit hoher Sauerstoffaffinität für die Verwendung nach Anspruch 7, wobei das Trägervehikel aus einem von einem Nanopartikel-basierten Vehikel, einem Lipidvesikel, einem synthetischen Polymervesikel oder einem uni- oder multilamellaren Polymersom ausgewählt ist.

10. Mittel mit hoher Sauerstoffaffinität für die Verwendung nach Anspruch 7, wobei das Trägervehikel ein Vesikel ist und wobei das Mittel mit hoher Sauerstoffaffinität innerhalb eines wässrigen Kerns oder eines Membranteils des Vesikels vorliegt.

11. Mittel mit hoher Sauerstoffaffinität für die Verwendung nach Anspruch 7, wobei das Trägervehikel eine Vielzahl von biologisch abbaubaren Polymeren umfasst.

12. Kit, der Folgendes umfasst:
einen ersten Behälter; und
einen zweiten Behälter;
wobei der erste Behälter das Mittel mit hoher Sauerstoffaffinität nach Anspruch 1 umfasst und wobei der zweite Behälter eine Rehydratationsmischung umfasst.

13. Mittel mit hoher Sauerstoffaffinität für die Verwendung nach Anspruch 1, wobei die Verabreichung des Sauerstoffträgers, der das Mittel mit hoher Sauerstoffaffinität enthält, an das Subjekt die Verabreichung des Sauerstoffträgers intravenös, über Inhalation, topisch, über das Rektum, über die Vagina, transdermal, subkutan, intraperitoneal, intrathekal, intramuskulär oder oral umfasst.

## Revendications

1. Agent à haute affinité pour l'oxygène destiné à être utilisé dans une méthode d'augmentation de l'efficacité d'un rayonnement ou d'une chimiothérapie appliqués à une tumeur chez un sujet, ladite méthode comprenant :
l'acheminement dudit agent à haute affinité pour l'oxygène vers la tumeur par l'administration au sujet d'un transporteur d'oxygène qui incorpore l'agent à haute affinité pour l'oxygène :
l'agent à haute affinité pour l'oxygène comprenant une composition de myoglobine ; et
les caractéristiques de gradient de pression partielle d'oxygène de l'agent à haute affinité pour l'oxygène étant telles que l'oxygène est libéré de l'agent à haute affinité pour l'oxygène à des tensions d'oxygène inférieures à 10 mm de Hg, l'oxygène libéré diffusant hors du transporteur d'oxygène alors que l'agent à haute affinité pour l'oxygène reste au sein du transporteur d'oxygène.

2. Agent à haute affinité pour l'oxygène destiné à être utilisé selon la revendication 1, ledit agent à haute affinité pour l'oxygène ayant une P50 pour l'oxygène autour de 2-3 mm de Hg.

3. Agent à haute affinité pour l'oxygène destiné à être utilisé selon la revendication 2, ledit agent à haute affinité pour l'oxygène comprenant une ou plusieurs de la myoglobine humaine non modifiée, la myoglobine non modifiée provenant d'une autre espèce biologique et la myoglobine chimiquement ou génétiquement modifiée provenant de l'homme ou provenant d'une autre espèce biologique.

4. Agent à haute affinité pour l'oxygène destiné à être utilisé selon la revendication 2, ledit agent à haute affinité pour l'oxygène étant un agent de liaison à l'oxygène coopératif ou un agent de liaison à l'oxygène linéaire.

5. Agent à haute affinité pour l'oxygène destiné à être utilisé selon la revendication 2, les caractéristiques de gradient de pression partielle d'oxygène dudit agent à haute affinité pour l'oxygène étant telles que l'oxygène est fermement lié par l'agent à haute affinité pour l'oxygène alors qu'il est en circulation dans une circulation sanguine.

6. Agent à haute affinité pour l'oxygène destiné à être utilisé selon la revendication 1, ledit agent à haute affinité pour l'oxygène étant pégylé ou polymérisé.

7. Agent à haute affinité pour l'oxygène destiné à être utilisé selon la revendication 1, ledit agent à haute affinité pour l'oxygène étant encapsulé dans le transporteur d'oxygène et le transporteur d'oxygène comprenant un véhicule transporteur qui empêche l'agent à haute affinité pour l'oxygène d'être libéré dans une circulation sanguine.

8. Agent à haute affinité pour l'oxygène destiné à être utilisé selon la revendication 1, ledit agent à haute affinité pour l'oxygène étant co-encapsulé dans un véhicule transporteur avec au moins un autre agent de sensibilisation aux rayonnements ou chimiothérapeutique.

9. Agent à haute affinité pour l'oxygène destiné à être utilisé selon la revendication 7, le véhicule transporteur étant choisi parmi l'un d'un véhicule à base de nanoparticules, d'une vésicule lipidique, d'une vésicule en polymère synthétique ou d'un polymersome unilamellaire ou multilamellaire.

10. Agent à haute affinité pour l'oxygène destiné à être utilisé selon la revendication 7, ledit véhicule transporteur étant une vésicule et l'agent à haute affinité pour l'oxygène étant à l'intérieur d'un coeur aqueux ou d'une partie membraneuse de la vésicule.

11. Agent à haute affinité pour l'oxygène destiné à être utilisé selon la revendication 7, ledit véhicule transporteur comprenant une pluralité de polymères biodégradables.

12. Kit, comprenant :
un premier récipient ; et
un second récipient ;
le premier récipient comprenant l'agent à haute affinité pour l'oxygène selon la revendication 1 et le second récipient comprenant un mélange de réhydratation.

13. Agent à haute affinité pour l'oxygène destiné à être utilisé selon la revendication 1, ladite administration au sujet du transporteur d'oxygène qui incorpore l'agent à haute affinité pour l'oxygène comprenant l'administration du transporteur d'oxygène par voie intraveineuse, par inhalation, par voie topique, par le rectum, par le vagin, par voie transdermique, par voie sous-cutanée, par voie intrapéritonéale, par voie intrathécale, par voie intramusculaire ou par voie orale.
